# EUROPEAN PATENT APPLICATION

(11) **EP 4 242 316 A1**
(43) Date of publication of application: **13.09.2023**
(21) Application number: 21889200.8
(22) Date of filing: 02.11.2021
(51) Int. Cl.: C12N 15/86, C12N 1/21, C12N 7/01, C12N 15/34, C12N 15/35, C12N 15/75

(54) **INTEGRATIVE PLASMID**

(30) Priority: 04.11.2020 JP 2020184491
(71) Applicant: Synplogen Co., Ltd., Nada-ku Kobe-shi Hyogo 657-0013 (JP)
(72) Inventor: SAITO, Shunsuke, Kobe-shi, Hyogo 657-0013 (JP); TSUGE, Kenji, Kobe-shi, Hyogo 657-0013 (JP)
(74) Representative: Appleyard Lees IP LLP
(86) International application number: PCT/JP2021/040411
(87) International publication number: WO 2022/097647

(57) **Abstract**

The present disclosure pertains to production of a virus vector plasmid. According to one aspect, the present disclosure provides a method for producing a virus vector plasmid having a sequence to be replicated in *Bacillus subtilis.* The method includes a step for forming a plasmid in a host cell by introducing, into the host cell, a nucleic acid that has a sequence to be replicated in *Bacillus subtilis* and that includes a nucleic acid sequence for producing a virus vector. In one embodiment, *Bacillus subtilis* could have the ability to form a plasmid from a nucleic acid acquired from outside, and therefore, in this method, the nucleic acid introduced does not have to be a plasmid.

## Description

### [Technical Field]

The present disclosure provides a plasmid for producing a virus vector. The present disclosure also provides a virus vector produced from a plasmid and a composition comprising the same.

### [Background Art]

Various virus vectors such as adenovirus vectors (Patent Literature 1) have been developed for gene therapy, vaccine therapy, and the like.

Preparation of a virus vector generally requires introduction of a virus vector plasmid into a producer cell. Thus, synthesis/construction of a virus vector plasmid is required. Designed virus vector plasmids are often synthesized/constructed in *Escherichia coli* or the like and have never been synthesized/constructed in hay bacillus.

### [Citation List]

### [Patent Literature]

[PL1] International Publication No. WO 2001/090392

### [Summary of Invention]

### [Solution to Problem]

The present inventors found that a virus vector plasmid can be produced in hay bacillus. Based on this finding, the present disclosure provides a virus vector plasmid which can be replicated in hay bacillus. The present disclosure also provides hay bacillus comprising a virus vector plasmid, and a virus vector produced from a virus vector plasmid.

Thus, the present invention provides the following.

### (Item A1)

A plasmid comprising:
a nucleic acid sequence which promotes plasmid replication in hay bacillus; and
at least one of nucleic acid sequences required for constituting a virus vector.

### (Item A2)

The plasmid of any of the preceding items, wherein the nucleic acid sequences required for constituting a virus vector comprise:
a nucleic acid sequence encoding a capsid protein of the virus;
a nucleic acid sequence encoding a protein which packages, transcribes, and replicates a genome of the virus;
two terminal repeat sequences of the virus; and
a nucleic acid sequence encoding a helper gene.

### (Item A3)

The plasmid of any of the preceding items, comprising at least two of:
a nucleic acid sequence encoding a capsid protein of the virus;
a nucleic acid sequence encoding a protein which packages, transcribes, and replicates a genome of the virus;
two terminal repeat sequences of the virus; and
a nucleic acid sequence encoding a helper gene.

### (Item A4)

The plasmid of any of the preceding items, comprising at least three of:
a nucleic acid sequence encoding a capsid protein of the virus;
a nucleic acid sequence encoding a protein which packages, transcribes, and replicates a genome of the virus;
two terminal repeat sequences of the virus; and
a nucleic acid sequence encoding a helper gene.

### (Item A5)

The plasmid of any of the preceding items, comprising:
a nucleic acid sequence encoding a capsid protein of the virus;
a nucleic acid sequence encoding a protein which packages, transcribes, and replicates a genome of the virus;
two terminal repeat sequences of the virus; and
a nucleic acid sequence encoding a helper gene.

### (Item A6)

The plasmid of any of the preceding items, wherein the nucleic acid sequences required for constituting a virus vector are about 10 kb or greater.

### (Item A7)

The plasmid of any of the preceding items, wherein at least one of the nucleic acid sequence encoding a capsid protein, the nucleic acid sequence encoding a protein which packages, transcribes, and replicates a genome, and the helper gene is outside a region sandwiched by the two terminal repeat sequences.

### (Item A8)

The plasmid of any of the preceding items, wherein the nucleic acid sequence which promotes plasmid replication in hay bacillus comprises a replication origin point which operates in hay bacillus.

### (Item A9)

The plasmid of any of the preceding items, further comprising a nucleic acid sequence which amplifies a plasmid in *Escherichia coli.*

### (Item A10)

The plasmid of any of the preceding items, wherein the nucleic acid sequence encoding a capsid protein of the virus comprises at least one of L1, L2, L3, L4, L5, cap, and gag.

### (Item A11)

The plasmid of any of the preceding items, wherein the nucleic acid sequence encoding a protein which packages, transcribes, and replicates a genome of the virus comprises at least one of E1A, E1B, E2A, E2B, E4, rep, ψ, APP, MAAP, pol, and rev.

### (Item A12)

The plasmid of any of the preceding items, wherein the terminal repeat sequences are inverted terminal repeats (ITRs) or long terminal repeats (LTRs).

### (Item A13)

The plasmid of any of the preceding items, wherein the helper gene comprises at least one of E1A, E1B, E2A, E4, VA, env, tat, RPE, PPT, PRE, and pro.

### (Item A14)

The plasmid of any of the preceding items, wherein at least one of the nucleic acid sequence encoding a capsid protein of the virus and the nucleic acid sequence encoding a protein which transcribes and replicates a genome of the virus is derived from any of serotypes 1 to 52 of an adenovirus or serotypes 1 to 12 of an adeno-associated virus and a variant thereof.

### (Item A15)

The plasmid of any of the preceding items, wherein the plasmid does not comprise a sequence of a gene of at least a part of a whole genome of the virus.

### (Item A16)

The plasmid of any one of items A1 to 15, wherein the virus is an adenovirus, an adeno-associated virus, a lentivirus, a retrovirus, or a Sendai virus.

### (Item A17)

The plasmid of any of the preceding items, wherein the virus is an adeno-associated virus or a lentivirus.

### (Item A18)

The plasmid of any of the preceding items, wherein the virus is an adeno-associated virus.

### (Item A19)

The plasmid of any of the preceding items, wherein the terminal repeat sequences are inverted terminal repeats (ITRs) derived from any of serotypes 1 to 12 of an adeno-associated virus and a variant thereof.

### (Item A20)

The plasmid of any of the preceding items, comprising a promotor, a gene of interest, and a terminator from upstream between 5'ITR and 3'ITR.

### (Item A21)

The plasmid of any of the preceding items, wherein the helper gene comprises at least one of E1A, E1B, E2A, E4, and VA.

### (Item A22)

The plasmid of any of the preceding items, wherein the helper gene comprises E2A, E4, and VA.

### (Item A23)

The plasmid of any of the preceding items, wherein the helper gene is each derived from any of serotypes 1 to 52 of an adenovirus and a variant thereof.

### (Item A24)

The plasmid of any of the preceding items, wherein the nucleic acid sequence encoding a protein which packages, transcribes, and replicates a genome of the virus comprises a rep.

### (Item A25)

The plasmid of any of the preceding items, wherein the rep is derived from any of serotypes 1 to 12 of an adeno-associated virus and a variant thereof.

### (Item A26)

The plasmid of any of the preceding items, wherein the nucleic acid sequence encoding a capsid protein of the virus comprises a cap.

### (Item A27)

The plasmid of any of the preceding items, wherein the cap is derived from any of serotypes 1 to 12 of an adeno-associated virus and a variant thereof.

### (Item A28)

The plasmid of any of the preceding items, comprising a gene of interest between the two terminal repeat sequences of the virus.

### (Item A29)

The plasmid of any of the preceding items, wherein the gene of interest is 2 to 100 genes.

### (Item A30)

The plasmid of any of the preceding items, wherein the gene of interest is 10 to 100 genes.

### (Item A31)

The plasmid of any of the preceding items, wherein the gene of interest comprises a promoter sequence.

### (Item A32)

The plasmid of any of the preceding items, wherein the plasmid and a producer cell comprise a nucleic acid sequence required for constituting the virus vector together.

### (Item A33)

The plasmid of any of the preceding items, wherein virus vector particles which do not comprise a nucleic acid in all virus vector particles which are produced from a producer cell introduced with the plasmid are 65% or less.

### (Item A34)

The plasmid of any of the preceding items, wherein, when the plasmid is introduced into a producer cell, virus vector particles comprising a nucleic acid comprising all genes of interest in all virus vector particles which are produced are 90% or greater.

### (Item A35)

The plasmid of any of the preceding items, wherein, when the plasmid is introduced into a producer cell, virus vector particles comprising a nucleic acid derived from the plasmid other than a desired nucleic acid in all virus vector particles which are produced are 2% or less.

### (Item A36)

The plasmid of any of the preceding items, wherein a CCC (covalently closed circular) purity is 80% or greater.

### (Item A37)

A composition for use in expressing a gene of interest, the composition comprising the plasmid of any of the preceding items, wherein the gene of interest is placed between the two terminal repeat sequences of the virus on the plasmid.

### (Item A38)

A method for producing the plasmid of any of the preceding items, the method comprising operably linking the nucleic acid sequence of any of the preceding items.

### (Item A39)

A composition comprising a plasmid produced by the method of item A38.

### (Item A40)

The composition comprising a plasmid of any of the preceding items, wherein a CCC (covalently closed circular) purity of a plasmid is 80% or greater.

### (Item A41)

A kit for use in creating the plasmid of any of the preceding items, the kit comprising at least one nucleic acid comprising:
a nucleic acid sequence which promotes plasmid replication in hay bacillus; and
a nucleic acid sequence required for constituting a virus.

### (Item A42)

A composition for use in preparing a virus vector, the composition comprising the plasmid of any of the preceding items.

### (Item A43)

A method for preparing a virus vector, the method comprising producing a virus vector by using the plasmid of any of the preceding items.

### (Item A44)

The method of any of the preceding items, wherein at least a part of a nucleic acid contained in the plasmid is incorporated into a chromosome of a producer cell introduced with the plasmid.

### (Item A45)

A virus vector produced by using the plasmid of any one of items A1 to 36 or produced by using the method of item A43 or 44.

### (Item A46)

A composition comprising a virus vector produced by using the plasmid of any of the preceding items or produced by using the method of any of the preceding items.

### (Item A47)

The composition of any of the preceding items, wherein virus vector particles which do not comprise a nucleic acid in all virus vector particles are 65% or less.

### (Item A48)

The composition of any of the preceding items, wherein virus vector particles comprising a nucleic acid comprising all genes of interest in all virus vector particles are 90% or greater.

### (Item A49)

The composition of any of the preceding items, wherein virus vector particles comprising a nucleic acid derived from the plasmid other than a desired nucleic acid in all virus vector particles are 2% or less.

### (Item A50)

Hay bacillus or *Escherichia coli* comprising the plasmid of any of the preceding items.

### (Item A51)

A producer cell introduced with the plasmid of any of the preceding items, wherein at least a part of a nucleic acid contained in the plasmid is incorporated into a chromosome of the producer cell.

### (Item 1)

A plasmid comprising:
a nucleic acid sequence which promotes plasmid amplification in hay bacillus; and
at least one of nucleic acid sequences required for constituting a virus vector.

### (Item 2)

The plasmid of any of the preceding items, wherein the nucleic acid sequences required for constituting a virus vector comprise:
a nucleic acid sequence encoding a capsid protein of the virus;
a nucleic acid sequence encoding a protein which packages, transcribes, and replicates a genome of the virus;
two terminal repeat sequences of the virus; and
a helper gene.

### (Item 3)

The plasmid of any of the preceding items, wherein the nucleic acid sequence which promotes plasmid amplification in hay bacillus comprises a replication origin point which operates in hay bacillus.

### (Item 4)

The plasmid of any of the preceding items, wherein the nucleic acid sequence encoding a capsid protein of the virus comprises at least one of L2, L3, cap, and gag.

### (Item 5)

The plasmid of any of the preceding items, wherein the nucleic acid sequence encoding a protein which packages, transcribes, and replicates a genome of the virus comprises at least one of E1A, E1B, E2A, E2B, E4, rep, ψ, APP, MAAP, pol, and rev.

### (Item 6)

The plasmid of any of the preceding items, wherein the terminal repeat sequences are inverted terminal repeats (ITRs) or long terminal repeats (LTRs).

### (Item 7)

The plasmid of any of the preceding items, wherein the helper gene comprises at least one of E1A, E1B, E2A, E4, VA, env, tat, RPE, PPT, PRE, and pro.

### (Item 8)

The plasmid of any of the preceding items, wherein at least one of the nucleic acid sequence encoding a capsid protein of the virus and the nucleic acid sequence encoding a protein which transcribes and replicates a genome of the virus is derived from any of serotypes 1 to 51 of an adenovirus or serotypes 1 to 11 of an adeno-associated virus.

### (Item 9)

The plasmid of any of the preceding items, wherein the virus is an adenovirus, an adeno-associated virus, a lentivirus, a retrovirus, or a Sendai virus.

### (Item 10)

The plasmid of any of the preceding items, comprising a gene of interest between the two terminal repeat sequences of the virus.

### (Item 11)

The plasmid of any of the preceding items, wherein the gene of interest is 2 to 100 genes.

### (Item 12)

The plasmid of any of the preceding items, wherein the gene of interest comprises a promoter sequence.

### (Item 13)

The plasmid of any of the preceding items, wherein the plasmid is configured to function in a producer cell wherein the plasmid and the producer cell comprise a nucleic acid sequence required for constituting the virus vector.

### (Item 14)

The plasmid of any of the preceding items, wherein virus vector particles which do not comprise a nucleic acid in all virus vector particles which are produced from a producer cell introduced with the plasmid are 65% or less.

### (Item 15)

The plasmid of any of the preceding items, wherein, when the plasmid is introduced into a producer cell, virus vector particles comprising a nucleic acid comprising all genes of interest in all virus vector particles which are produced are 90% or greater.

### (Item 16)

The plasmid of any of the preceding items, wherein, when the plasmid is introduced into a producer cell, virus vector particles comprising a nucleic acid derived from the plasmid other than a desired nucleic acid in all virus vector particles which are produced are 2% or less.

### (Item 17)

A method for producing the plasmid of any of the preceding items, the method comprising operably linking the nucleic acid sequence of any of the preceding items.

### (Item 18)

A composition comprising a plasmid produced by the method of any of the preceding items.

### (Item 19)

The composition comprising a plasmid of any of the preceding items, wherein a CCC (covalently closed circular) purity of a plasmid is 80% or greater.

### (Item 20)

A kit for use in creating the plasmid of any of the preceding items, the kit comprising at least one nucleic acid comprising:
a nucleic acid sequence which promotes plasmid amplification in hay bacillus; and
a nucleic acid sequence required for constituting a virus.

### (Item 21)

A composition for use in preparing a virus vector, the composition comprising the plasmid of any of the preceding items.

### (Item 22)

A method for preparing a virus vector, the method comprising producing a virus vector by using the plasmid of any of the preceding items.

### (Item 23)

A virus vector produced by using the plasmid of any of the preceding items or produced by using the method of any of the preceding items.

### (Item 24)

A composition comprising a virus vector produced by using the plasmid of any of the preceding items or produced by using the method of any of the preceding items.

### (Item 25)

The composition of any of the preceding items, wherein virus vector particles which do not comprise a nucleic acid in all virus vector particles are 65% or less.

### (Item 26)

The composition of any of the preceding items, wherein virus vector particles comprising a nucleic acid comprising all genes of interest in all virus vector particles are 90% or greater.

### (Item 27)

The composition of any of the preceding items, wherein virus vector particles comprising a nucleic acid derived from the plasmid other than a desired nucleic acid in all virus vector particles are 2% or less.

### (Item 28)

Hay bacillus comprising the plasmid of any of the preceding items.

The present invention is intended so that one or more of the above features can be provided not only as the explicitly disclosed combinations, but also as other combinations thereof. Additional embodiments and advantages of the present invention are recognized by those skilled in the art by reading and understanding the following detailed explanation, as needed.

### [Advantageous Effects of Invention]

The present disclosure provides a virus vector plasmid which can be replicated in hay bacillus. It is possible to produce a virus vector in a novel and improved manner based on the virus vector plasmid.

### [Brief Description of Drawings]

[Figure 1] Figure **1** shows the structure of pAAV-CMV plasmid.
[Figure 2] Figure **2** shows the structure of pRC2-mi342 plasmid.
[Figure 3] Figure **3** shows the structure of pHelper plasmid.
[Figure 4] Figure **4** shows the structure of pGETS103-AV plasmid.
[Figure 5] Figure **5** shows the structure of pGETS103-RC2 plasmid.
[Figure 6] Figure **6** shows the structure of pGETS103-AAV-RC2 plasmid.
[Figure 7] Figure **7** shows the structure of pGETS103-AAV-Helper-RC2 plasmid (all-in-one structure).
[Figure 8] Figure **8** shows the structure of pGETS103-AAV plasmid.
[Figure 9] Figure **9** shows the structure of pGETS103-Helper plasmid.
[Figure 10] Figure **10** shows the outline of construction of pGETS103-AAV-Helper-RC2 plasmid from 22 fragments by OGAB method. The dotted line box indicates the moiety which is removed by AarI cleavage (the capital letters indicate a linker-derived sequence while small letters indicate a vector-derived sequence). The 1st, 3rd, and 16th fragments were obtained by chemical synthesis and MAP method. The rest of the fragments were obtained by PCR.
[Figure 11] Figure **11** shows the result of electrophoresis of pGETS103-AAV-Helper-RC2 plasmid constructed by OGAB method.
[Figure 12] Figure **12** shows the structure of an exemplary lentivirus vector plasmid constructed based on pGETS103ΔAarI.
[Figure 13] Figure **13** shows the structure of an exemplary retrovirus vector plasmid constructed based on pGETS103ΔAarI.
[Figure 14] Figure **14** shows the structure of an exemplary Sendai virus vector plasmid constructed based on pGETS103ΔAarI.
[Figure 15] Figure **15** shows the structure of an exemplary adenovirus vector plasmid constructed based on pGETS103ΔAarI.
[Figure 16] Figure **16** shows the structure of plasmid pGETS118-AarI and the site into which a nucleic acid is introduced. The dotted line box indicates the moiety which is removed by AarI cleavage.
[Figure 17] Figure **17** shows additional 7 types of all-in-one structures for producing an AAV vector introduced into pGETS118-AarI. The white squares shown below each all-in-one structure indicate the number of unit DNAs that were used for construction and the approximate corresponding regions.
[Figure 18] Figure **18** shows that a virus vector is produced in the same manner as pGETS103-AAV-Helper-RC2 plasmid (1) even when the additional vector plasmids (2 to 8) are used.
[Figure 19] Figure **19** shows an all-in-one structure for producing an adenovirus vector. The white squares shown at the bottom indicate the number of unit DNAs that were used for construction and the approximate corresponding regions. GOI indicates the gene of interest.
[Figure 20] Figure **20** shows the structure of plasmid pBET131-AarI and the sites into which a nucleic acid is introduced. The dotted line box indicates the moiety which is removed by AarI cleavage.
[Figure 21] Figure **21** shows the structure of an AAV virus all-in-one vector plasmid based on pGETS103-ΔAarI using Rep of AAV1 and Cap of AAV6. The white squares shown below the all-in-one structure indicate the number of unit DNAs that are used for construction and the approximate corresponding regions.
[Figure 22] Figure **22** shows the structure of an AAV virus all-in-one vector plasmid based on pGETS103-ΔAarI using CAG promoter, Rep of AAV5, and Cap of AAV1. The white squares shown below the all-in-one structure indicate the number of unit DNAs that are used for construction and the approximate corresponding regions.
[Figure 23] Figure **23** shows the structure of an AAV virus all-in-one vector plasmid based on pGETS103-ΔAarI using EF1α promoter, Rep of AAV8, and Cap of AAV9. The white squares shown below the all-in-one structure indicate the number of unit DNAs that are used for construction and the approximate corresponding regions.
[Figure 24] Figure **24** shows the structure of an AAV virus all-in-one vector plasmid based on pGETS103-ΔAarI, wherein Rep, Cap, and Helper are placed in a different order. This is also an example using SV40 promoter. The white squares shown below the all-in-one structure indicate the number of unit DNAs that are used for construction and the approximate corresponding regions.
[Figure 25] Figure **25** shows the structure of an AAV virus all-in-one vector plasmid based on pBETS131-AarI, wherein Rep, Cap, and Helper are placed in a different order. The white squares shown below the all-in-one structure indicate the number of unit DNAs that are used for construction and the approximate corresponding regions.
[Figure 26] Figure **26** shows the structure of an AAV virus all-in-one vector plasmid based on pBETS103-ΔAarI, in which the element of Helper gene has been changed. The white squares shown below the all-in-one structure indicate the number of unit DNAs that are used for construction and the approximate corresponding regions.
[Figure 27] Figure **27** shows the structure of a coronavirus all-in-one vector plasmid based on pGETS103-ΔAarI using Rep of AAV1 and Cap of AAV6. This is an example where a gene of interest (GOI) is positioned together with a promoter in the structural protein region.

### [Description of Embodiments]

The present disclosure is described hereinafter while showing the best modes thereof. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

The definitions of the terms and/or basic technical concepts that are particularly used herein are described hereinafter when appropriate.

As used herein, "hay bacillus" is an aerobic, Gram-positive, and catalase-positive bacterium which is generally present in soil and plants and present in the gastrointestinal tract of ruminants or humans. "Hay bacillus" is 0.7 to 0.8 × 2 to 3 µm in size, is a mesophile, has optimal growth temperature of 25 to 40°C, is said to form a spore, and refers to any non-pathogenic bacterium with a natural transformation ability having scientific name *Bacillus subtilis* or including closely related bacteria that belong to the genus *Bacillus* such as *Bacillus amyloliquefaciens, Bacillus licheniformis,* or *Bacillus pumilus.* In a preferred embodiment, hay bacillus is Marburg 168 strain with a high natural transformation ability among *Bacillus subtilis,* or its derivative strain, RM125 strain. The 168 strain is a Gram-positive bacterium that has been genetically studied the most, which can be obtained from ATCC (American Type Culture Collection) or the like along with the RM125 strain. It has been found that the 168 strain itself is harmless to humans and animals and OGAB method is applicable to the strain. Thus, the strain can be suitably used in the present disclosure.

As used herein, a "plasmid" refers to a cyclic DNA which is present in a cell separately from a chromosome, or present separately from a chromosome when being introduced into a cell.

As used herein, a "nucleic acid sequence which promotes plasmid replication" is used to have the same meaning as a "nucleic acid sequence which promotes plasmid amplification", and refers to any nucleic acid sequence which promotes replication (same as amplification in this context) of a plasmid present in a host cell when the nucleic acid sequence is introduced into the host cell (e.g., hay bacillus). Preferably, this nucleic acid sequence which promotes plasmid replication is operably linked to a nucleic acid sequence encoding a target plasmid. However, said nucleic acid sequence is not limited thereto. Details of a nucleic acid sequence which promotes plasmid replication, a target plasmid, the relationship thereof and the like are described elsewhere herein. Examples of a nucleic acid sequence which promotes plasmid replication can include a nucleic acid sequence comprising a replication origin point which operates in a target host cell (e.g., hay bacillus) and the like. For example, while a nucleic acid sequence which promotes plasmid replication in hay bacillus has an ability to enable promotion of plasmid replication in hay bacillus, the nucleic acid sequence may further have an ability to enable promotion of plasmid replication in a microorganism other than hay bacillus (e.g., *Escherichia coli*)*.* A nucleic acid sequence which promotes plasmid replication in hay bacillus and other organisms (e.g., *Escherichia coli*) may be a nucleic acid sequence wherein the same region is utilized for promotion of plasmid replication in both hay bacillus and other organisms (e.g., *Escherichia coli*)*,* may be a contiguous sequence on the vector plasmid of the present disclosure, or may be a sequence positioned away. For example, it has been demonstrated that plasmid pSTK1 (Issay Narumt et al., BIOTECHNOLOGY LETTERS, Volume 17 No.5 (May 1995) pp.475-480) and plasmid pBS195 (Molekuliarnaia Genetika et al., 01 May 1991, (5) : 26-29, PMID:1896058) can be replicated in the same replication initiating region in both hay bacillus and *Escherichia coli.* Such nucleic acid sequences can be used as a nucleic acid sequence which promotes plasmid replication in both hay bacillus and *Escherichia coli.* Further, a shuttle plasmid (P Trieu-Cuot et al., EMBO J. 1985 Dec 16;4(13A): 3583-3587.) created by linking BR322 (*Escherichia coli* plasmid) and pC194 (hay bacillus plasmid), and a plasmid of *B. subtilis* Secretory Protein Expression System comprising pUBori and ColE1 ori provided by Takara Bio (Shiga prefecture) can be replicated in both *Escherichia coli* and hay bacillus. Such nucleic acid sequences which are positioned away can also be used as a nucleic acid sequence which promotes plasmid replication in both hay bacillus and *Escherichia coli.* Examples of a replication mechanism for a nucleic acid (plasmid) in hay bacillus include rolling-circle type, theta-type, oriC, a mechanism using a phage, and the like. Any sequence utilizing any mechanism can be used as a sequence which is replicated in hay bacillus. The rolling-circle-type replication mechanism is a mechanism which replicates a single strand on one side of a double-stranded DNA and then replicates a strand on the other side, wherein a plasmid tends to be destabilized due to a long period of time during which a nucleic acid exists as a single-stranded DNA. The theta-type replication mechanism is a mechanism in which replication of a double-stranded DNA (plasmid) is simultaneously initiated in two directions from a replication origin point in the same manner as replication of a bacterial chromosome. The theta-type replication mechanism can be preferably used for replication of a long DNA (e.g., 10 kb or greater) as in the present disclosure (Janniere, L., A. Gruss, and S. D. Ehrlich. 1993. Plasmids, p. 625-644. InA. L. Sonenshein, J. A. Hoch, and R. Losick (ed.), Bacillus subtilis and other gram-positive bacteria: biochemistry, physiology and molecular genetics. American Society for Microbiology, Washington, D.C.). The oriC replication mechanism operates in the same manner as the replication of a chromosome of a host bacterium (e.g., hay bacillus) . Examples of a nucleic acid sequence which promotes plasmid replication in hay bacillus include a plasmid or a portion or replication origin point thereof or a variant thereof and the like, which are known to operate the rolling-circle-type replication mechanism, the theta-type replication mechanism, the oriC replication mechanism, or the replication mechanism using a phage or the like. Examples that are known as a rolling-circle-type plasmid include pUB110, pC194, pE194, pT181, and the like. Examples that are known as a theta-type plasmid include pAMβ1, pTB19, pLS32, pLS20, and the like. A nucleic acid sequence which promotes plasmid replication in hay bacillus can have a sequence identical or similar to a known replication origin (e.g., sequence identity of 90% or greater, 95% or greater, 97% or greater, 98% or greater, 99% or greater, or 99.5% or greater). For example, when a DNA fragment in which a candidate DNA fragment and a selection marker gene effective in hay bacillus such as drug resistant genes are linked is introduced into hay bacillus and then cultured (by adding an agent or the like), after which the DNA fragment is replicated outside a chromosome, it can be determined that the candidate DNA fragment has a nucleic acid sequence which promotes plasmid replication in hay bacillus. A nucleic acid sequence which promotes plasmid amplification refers to a DNA fragment having a replication origin, which can be replicated independently of a chromosomal DNA. Whether these DNA fragments can be replicated can be confirmed by an approach such as linking a selection marker gene such as drug resistant genes to these DNA fragments and culturing under a selection condition such as using an agent, followed by purifying a plasmid DNA and observing the band of the DNA by electrophoresis. In addition to a replication origin, a plasmid may comprise a gene of a rep protein which recruits a DNA replication enzyme of a host to the replication origin, a segregation mechanism gene for ensuring segregation of a plasmid to a daughter cell, a selection marker gene or the like. The replication origin may be fused inside the gene of the rep protein.

As used herein, a "packaging cell" refers to a cell for producing a vector plasmid.

As used herein, a "vector plasmid" or a "virus vector plasmid" refers to a plasmid for producing a virus vector in a producer cell, the plasmid comprising a gene to be loaded on the virus vector. A vector plasmid comprises a sequence of a gene (gene of interest) that is expressed in a target cell of a virus vector between two terminal repeat sequences, additionally comprises a promoter, and may further comprise other elements (e.g., an enhancer, a terminator, or the like).

As used herein, a "producer cell" refers to a cell capable of producing a desired virus vector, which can be a cell in which a gene required for production of the virus vector is expressed from a chromosome and an introduced plasmid. A desired virus vector can be produced by introducing the vector plasmid of the present disclosure into a producer cell. For example, although E1A and E1B are required for production of an AAV virus vector, since HEK293 has E1A and E1B, they can be removed from a helper plasmid. Other cells also function as a producer cell if they are modified to have such a helper factor.

As used herein, a "virus vector" refers to a construct which at least partially has a structure derived from a virus and can introduce a nucleic acid into a target cell. Typically, a virus vector is in the form of a viral particle comprising a capsid of a virus and a nucleic acid comprising a heterologous gene. As used herein, an "origin virus" refers to a virus which naturally has a virus-derived structure of the virus vector. In a virus vector, a nucleic acid contained in a capsid (loaded nucleic acid) comprises a gene (gene of interest) sequence that is expressed in a target cell of the virus vector between two terminal repeat sequences, and may additionally comprise a promoter, an enhancer, a terminator or the like, and may comprise a gene derived from the origin virus.

As used herein, a "nucleic acid sequence required for constituting a virus vector" refers to a nucleic acid sequence (e.g., a combination of nucleic acid sequences) wherein a producer cell comprising the nucleic acid sequence can produce a virus vector. In one embodiment, a nucleic acid sequence required for constituting a virus vector comprises: a nucleic acid sequence encoding a capsid protein of a virus; a nucleic acid sequence encoding a protein which packages, transcribes, and replicates a genome of the virus; two terminal repeat sequences of the virus; and a nucleic acid sequence of a helper gene. A "nucleic acid sequence of a helper gene", for example, can comprise or essentially consists of any nucleic acid sequence required for constituting a virus vector other than a nucleic acid sequence encoding a capsid protein of a virus, a nucleic acid sequence encoding a protein which packages, transcribes, and replicates a genome of the virus, and two terminal repeat sequences of the virus. Each of these nucleic acid sequences can vary depending on the virus vector. Details thereof are described elsewhere herein. In a preferred embodiment, an advantageous sequence that an AAV and an adenovirus have in common, more preferably, an advantageous sequence to an AAV may be utilized for a nucleic acid sequence required for constituting a virus vector. Such an advantageous sequence that an AAV and an adenovirus have in common is characterized in that the advantageous sequence may comprise a gene of interest between inverted terminal repeats (ITRs) and may comprise a helper gene, a rep, a cap, or L1, L2, L3, L4, or L5. An advantageous sequence to an AAV comprises a gene of interest between ITRs and may comprise a helper gene, a rep, or a cap. Said sequence is characterized in that the helper gene, the rep, or the cap may be placed outside the two ITRs. Examples thereof include, but are not limited to, the sequence of specific helper genes, E1A, E1B, E2A, E2B, E3, and E4.

As used herein, a "protein which packages, transcribes, and replicates a genome of a virus" includes a protein which causes a capsid to enclose the genome of the virus, a protein which performs transcription, a protein which replicates the genome of the virus, and a protein having both of those functions. Examples thereof can include E1A, E1B, E2A, E2B, E4, rep, pol, ϕ, APP, MAAP, rev, and the like. A protein that can be utilized as a protein which packages, transcribes, and replicates a genome of a virus is, but is not intended to be limited to, a protein derived from any of serotypes 1 to 52 of an adenovirus or serotypes 1 to 12 of an adeno-associated virus, or a variant thereof (rh10, DJ, DJ/8, PHP.eB, PHP.S, AAV2-retro, AAV2-QuadYF, AAV2.7m8, AAV6.2, rh.74, AAV2.5, AAV-TT, Anc80, or the like). This protein may be a naturally-occurring protein or a protein artificially introduced with a mutation. A protein "artificially" introduced with "a mutation" can be created by, for example, introducing an appropriate mutation into a sequence of a naturally-occurring protein described in a known document.

As used herein, a "capsid" refers to a protein produced from a gene of a virus which is present on the surface of the virus or a virus vector (enclosed in an envelope as required). A capsid is also referred to as "capsid protein". A capsid can be responsible for infectivity to a cell. Examples of a capsid protein can include, but are not limited to, L2, L3, cap, and gag. A nucleic acid sequence that can be utilized as a nucleic acid sequence encoding a capsid protein of a virus is, but is not intended to be limited to, derived from any of serotypes 1 to 52 of an adenovirus or serotypes 1 to 12 of an adeno-associated virus, or a variant thereof (rh10, DJ, DJ/8, PHP.eB, PHP.S, AAV2-retro, AAV2-QuadYF, AAV2.7m8, AAV6.2, rh.74, AAV2.5, AAV-TT, Anc80, or the like). A capsid may be a naturally-occurring capsid or a capsid artificially introduced with a mutation. A capsid "artificially" introduced with "a mutation" can be created by, for example, introducing an appropriate mutation into a sequence of a naturally-occurring capsid described in a known document.

As used herein, a "repeat sequence" or "tandem repeat" (nucleic acid sequence) is a general term for sequences in which the same sequence is observed repeatedly (particularly for a few times or more) in a nucleic acid sequence of a biological genome. Any repeat sequence which is used in the art can be used in the present disclosure. Typically, a promoter sequence repeatedly appears.

As used herein, a "terminal repeat sequence" is a general term for sequences present at the terminals among sequences in which the same sequence is observed repeatedly (particularly for a few times or more) in a nucleic acid sequence of a biological genome. Examples of a terminal repeat sequence can include, but are not limited to, an inverted terminal repeat (ITR), a long terminal repeat (LTR), and the like. A sequence derived from any of serotypes 1 to 52 of an adenovirus or serotypes 1 to 12 of an adeno-associated virus, or a variant thereof (rh10, DJ, DJ/8, PHP.eB, PHP.S, AAV2-retro, AAV2-QuadYF, AAV2.7m8, AAV6.2, rh.74, AAV2.5, AAV-TT, Anc80, or the like) can be utilized as a terminal repeat sequence. A terminal repeat sequence may be a naturally-occurring sequence or a sequence artificially introduced with a mutation. A sequence "artificially" introduced with "a mutation" can be created by, for example, introducing an appropriate mutation into a naturally-occurring sequence described in a known document.

As used herein, a "helper gene" refers to a gene that assists amplification of a virus, which cannot proliferate on its own. In the present disclosure, examples of a helper gene can include, but are not limited to, any nucleic acid sequence required for constituting, proliferating, improving the activity of, or decreasing the toxicity of a virus vector other than a nucleic acid sequence encoding the capsid protein of the virus, a nucleic acid sequence encoding a protein which packages, transcribes, and/or replicates the genome of the virus, and the two terminal repeat sequences of the virus. Examples of a helper gene that can be used can include, but are not limited to, E1A, E1B, E2A, E2B, E4, RPE, WRPE, PPT, oPRE, enhancer, insulator, silencer sequence, and the like.

As used herein, a "loaded nucleic acid" refers to a nucleic acid which is carried by a virus vector. Whether or not a certain nucleic acid is a loaded nucleic acid can be confirmed by processing a virus vector preparation with DNase and cleaving a nucleic acid outside the capsid, followed by deactivating the DNase and then confirming a nucleic acid extracted from the capsid. Whether or not a certain nucleic acid is a loaded nucleic acid can also be confirmed by studying the sequence of the obtained nucleic acid product (preferably having a full length or a length close to the full length).

As used herein, a "host cell" refers to a cell (including a descendant of such a cell) into which an exogenous nucleic acid or protein or virus or virus vector has been introduced.

As used herein, a "protein", a "polypeptide", and a "peptide" are used to have the same meaning and refer to a polymer of amino acids with any length. The polymer may be linear, branched, or cyclic. An amino acid may be a naturally-occurring, non-naturally-occurring, or modified amino acid. The terms also encompass naturally-occurring or artificially modified polymers. Examples of such an modification include disulfide bond formation, glycosylation, lipidation, acetylation, phosphorylation, and any other manipulation or modification (e.g., conjugation with a labeling component).

As used herein, a "polynucleotide" and a "nucleic acid" are used in the same meaning, referring to a polymer of nucleotides of any length. Examples of a nucleic acid include DNA, RNA, cDNA, mRNA, rRNA, tRNA, microRNA (miRNA), and lncRNA. The terms also encompass "polynucleotide derivative". A "polynucleotide derivative" refers to a polynucleotide comprising a nucleotide derivative or having a bond between nucleotides that is different from ordinary bonds. A "nucleotide derivative" refers to a nucleotide having a structure different from ordinary nucleotides used in a naturally-occurring DNA or RNA. Examples thereof include locked nucleic acids (LNAs), ethylene nucleic acids such as 2'-O,4'-C-ethylene bridged nucleic acids (ENAs), other bridged nucleic acids (BNAs), hexitol nucleic acids (HNAs), Amido-bridged nucleic acids (AmNAs), morpholino nucleic acids, tricyclo-DNA (tcDNA), polyether nucleic acids (e.g., see US Patent No. 5,908,845), cyclohexene nucleic acids (CeNAs), and the like. Examples of a bond between nucleotides that is different from ordinary bonds include a bond between oligonucleotides with a phosphodiester bond converted into phosphorothioate bond, a bond between oligonucleotides with a phosphodiester bond converted into an N3'-P5' phosphoramidate bond, a bond between oligonucleotides with a ribose and a phosphodiester bond converted into a peptide nucleic acid bond, and the like.

Unless noted otherwise, specific nucleic acid sequences are intended to encompass sequences that are explicitly set forth, as well as their conservatively modified variants (e.g., degenerate codon substitutes) and complementary sequences. Specifically, a degenerate codon substitute can be achieved by making a sequence in which the third position of one or more selected (or all) codons is substituted with a mixed base and/or deoxyinosine residue. For example, a variant based on a specific wild-type sequence such as serotypes 1 to 52 of an adenovirus and serotypes 1 to 12 of an adeno-associated virus includes not only known variants (e.g., rh10, DJ, DJ/8, PHP.eB, PHP.S, AAV2-retro, AAV2-QuadYF, AAV2.7m8, AAV6.2, rh.74, AAV2.5, AAV-TT, Anc80 and the like) but also nucleic acids comprising a sequence with at least 70%, at least 80%, at least 85%, at least 90%, at least 95%, at least 96%, at least 97%, at least 98%, at least 99%, or at least 99.5% sequence identity to the original sequence.

As used herein, a "gene" refers to a nucleic acid moiety which plays a certain biological function. Examples of the biological function include encoding a polypeptide or a protein, encoding a protein non-coding functional RNA (such as rRNA, tRNA, microRNA (miRNA), or lncRNA), controlling production of a polypeptide, a protein, or a protein non-coding functional RNA, being specifically bound to a specific protein, and controlling cleavage or replication of a nucleic acid. Thus, a gene herein includes not only a nucleic acid moiety encoding a protein or a protein non-coding functional RNA but also a transcription and translation regulating sequence such as promoters, terminators, enhancers, insulators, silencers, replication origin points, or internal ribosome entry sites, and a nucleic acid moiety required for packaging into a viral particle. As used herein, a "gene product" can refer to a polypeptide, protein, or protein non-encoding functional RNA that is encoded by a gene.

As used herein, two genes being cis means that those genes are present on the same nucleic acid molecule or a nucleic acid molecule of a complementary strand (in the case of a double-stranded nucleic acid) . As used herein, two genes being trans means that these genes are not present on the same nucleic acid molecule or a nucleic acid molecule of a complementary strand (in the case of a double-stranded nucleic acid) in a certain cell (in a certain organism). For example, a gene present on a genome and a gene on a nucleic acid introduced by a virus vector can be trans. Optionally, whether two genes are trans can be determined based on the state when the two genes become present in a cell at the same time (e.g., at a time of introduction of a nucleic acid into a cell).

When referring to the number of genes herein, one gene refers to a gene having a contiguous sequence in the form of being present normally (with the highest frequency or with a probability of 50% or greater) on a genome of a certain organism. For example, two exons encoding a certain protein can be two genes. For example, when a promoter sequence and a sequence encoding a protein form a contiguous sequence, a nucleic acid moiety comprising the promoter sequence and the sequence encoding the protein can be one gene. For example, when a protein which becomes functional by being cleaved is encoded by a contiguous sequence on a genome, the protein can be encoded by one gene. When referring to a gene from a functional aspect, a nucleic acid sequence does not need to be a contiguous sequence. For example, a plurality of exons encoding a certain protein are collectively referred to as a gene for the protein.

As used herein, "deficient" in a gene means that a nucleic acid does not comprise the gene or a nucleic acid comprises a gene modified not to exhibit a normal function of the gene (e.g., function of producing a functional protein).

As used herein, "operably linked" means that expression (operation) of a desired sequence is placed under the control of a certain transcription and translation regulating sequence (e.g., a promoter, an enhancer, or the like) or translation regulating sequence. In order for a promoter to be operably linked to a gene, the promoter is generally placed immediately upstream of the gene, but the promoter does not necessarily need to be adjacently placed.

As used herein, a "transcription and translation regulating sequence" is a general term for promoter sequences, polyadenylation signals, transcription termination sequences, upstream regulatory domains, replication origin points, enhancers, IRESs and the like, which cooperatively enable replication, transcription, and translation of a coding sequence in a recipient cell. Not all of these transcription and translation regulating sequences always need to be present as long as a selected coding sequence can be replicated, transcribed, and translated in a suitable host cell. Those skilled in the art can easily identify a regulatory nucleic acid sequence from published information. Furthermore, those skilled in the art can identify a transcription and translation regulating sequence applicable to the purpose of use, for example, in vivo, ex vivo, or in vitro.

As used herein, a "promoter" refers to a segment of a nucleic acid sequence which controls transcription of an operably linked nucleic acid sequence. A promoter comprises a specific sequence sufficient for recognition, binding, and initiation of transcription by an RNA polymerase. A promoter may comprise a sequence which regulates recognition, binding, or initiation of transcription of an RNA polymerase.

As used herein, an "enhancer" refers to a segment of a nucleic acid sequence which has a function of enhancing the efficiency of expression of a gene of interest.

As used herein, a "silencer" refers to a segment of a nucleic acid sequence which has a function of decreasing the efficiency of expression of a gene of interest, contrary to an enhancer.

As used herein, an "insulator" refers to a segment of a nucleic acid sequence which has a function of cis-regulation for regulating expression of a gene positioned away on a sequence of a DNA.

As used herein, a "terminator" refers to a segment of a nucleic acid sequence which is positioned downstream of a region encoding a protein and involved in termination of transcription when a nucleic acid is transcribed onto an mRNA.

As used herein, a "replication origin point" refers to a segment of a nucleic acid sequence at which a DNA double helix is partially unwound by binding of a protein (e.g., initiator DnaA protein or the like) recognizing the nucleic acid sequence or by synthesis of an RNA, and from which replication is initiated.

As used herein, an internal ribosome entry site ("IRES") refers to a nucleic acid segment which promotes entry or retention of a ribosome when a nucleic acid sequence downstream of the internal ribosome entry site is translated.

As used herein, "homology" of a nucleic acid means the degree of identity of two or more nucleic acid sequences to each other, and generally, having "homology" means a high degree of identity or similarity. Thus, as the homology of two certain nucleic acids increases, the identity or similarity of the sequences thereof increases. "Similarity" is a value calculated by taking into consideration similar bases in addition to identity. In this regard, a similar base refers to instances with a partial match in a mixed base (e.g., R = A + G, M=A+C, W=A+T, S = C + G, Y = C + T, K = G + T, H = A + T + C, B = G + T + C, D = G + A + T, V = A + C + G, N = A + C + G + T). Whether two types of nucleic acids have homology can be found by direct comparison of sequences or by a hybridization method under stringent conditions. When two nucleic acid sequences are directly compared, the genes are homologous typically if the nucleic acid sequences are at least 50% identical, preferably at least 70% identical, and more preferably at least 80%, 90%, 95%, 96%, 97%, 98%, or 99% identical between the nucleic acid sequences.

Amino acids may be mentioned herein by either their commonly known three letter symbols or their one character symbols recommended by the IUPAC-IUB Biochemical Nomenclature Commission. Similarly, nucleotides may be mentioned by their commonly recognized one character codes. Comparison of similarity, identity, and homology of an amino acid sequence and a base sequence is calculated herein by using a sequence analysis tool BLAST with default parameters. For example, identity can be searched using BLAST 2.10.1+ (published on June 18, 2020) of the NCBI. Herein, values for identity generally refer to a value obtained when aligned under the default conditions using BLAST described above. However, when a higher value is obtained by changing a parameter, the highest value is considered the value of identity. When identity is evaluated in a plurality of regions, the highest value thereamong is considered the value of identity. Similarity is a value calculated by taking into consideration a similar amino acid in addition to identity.

As used herein, unless specifically noted otherwise, it is understood that a reference to a certain biological substance (e.g., protein, nucleic acid, or gene) is also a reference to a variant (e.g., a variant having a modification in the amino acid sequence) of the biological substance which exhibits the same function (not necessarily to the same degree) as the biological function of the biological substance. Such a variant can include a fragment of the original molecule and a molecule that is at least 30%, 40%, 50%, 60%, 70%, 80%, 90%, 95%, 98%, or 99% identical throughout the amino acid sequence or nucleic acid sequence of the original biological substance of the same size or in comparison to the sequence of the original molecule that is aligned by performing alignment by a computer homology program known in the art. A variant can include a molecule having a modified amino acid (e.g., modification by disulfide bond formation, glycosylation, lipidation, acetylation, or phosphorylation) or a modified nucleotide (e.g., modification by methylation).

As used herein, a "stringent condition" refers to a well-known condition that is conventionally used in the art. For a stringent condition, the following are examples of conditions that can be used. (1) Low ionic strength and a high temperature are used for washing (e.g., 0.015 M sodium chloride/0.0015 M sodium citrate/0.1% sodium dodecyl sulfate at 50°C), (2) a denaturing agent such as formamide is used in hybridization (e.g., 50% (v/v) formamide, 0.1% bovine serum albumin/0.1% ficoll/0.1% polyvinyl pyrrolidone/50 mM sodium phosphate buffer with a pH of 6.5, 750 mM sodium chloride, and 75 mM sodium citrate at 42°C), or (3) a solution comprising 20% formamide, 5 × SSC, 50 mM sodium phosphate (pH of 7.6), 5 × Denhardt's solution, 10% dextran sulfate, and 20 mg/ml denatured sheared salmon sperm DNA is incubated overnight at 37°C and then a filter is washed with 1 × SSC at about 37 to 50°C. The formamide concentration may be 50% or greater. Washing time can be 5, 15, 30, 60, or 120 minutes or longer. A plurality of elements such as temperature and salt concentration are conceivable as elements affecting the stringency of hybridization reactions. Ausubel et al., Current Protocols in Molecular Biology, Wiley Interscience Publishers, (1995) can be referred for details. "Highly stringent condition" is, for example, 0.0015 M sodium chloride, 0.0015 M sodium citrate, and 65 to 68° C or 0.015 M sodium chloride, 0.0015 M sodium citrate, 50% formamide, and 42°C. Hybridization can be performed in accordance with the method described in experimental publications such as Molecular Cloning 2nd ed., Current Protocols in Molecular Biology, Supplement 1-38, DNA Cloning 1: Core Techniques, A Practical Approach, Second Edition, Oxford University Press (1995). In this regard, a sequence comprising only an A sequence or only a T sequence is preferably excluded from a sequence that hybridizes under a stringent condition. A moderately stringent condition can be easily determined by those skilled in the art based on, for example, the length of a DNA, and is shown in Sambrook et al., Molecular Cloning: A Laboratory Manual, Third Ed., Vol. 1, 7.42-7.45 Cold Spring Harbor Laboratory Press, 2001, including, for a nitrocellulose filter, use of hybridization conditions of a pre-wash solution of 1.0 mM EDTA (pH of 8.0), 0.5% SDS, and 5 × SSC, and about 50% formamide and 2 × SSC to 6 × SSC at about 40 to 50° C (or other similar hybridization solutions such as a Stark's solution in about 50% formamide at about 42°C) and washing conditions of 0.5 × SSC, 0.1% SDS at about 60°C. Thus, the polypeptides used in the present disclosure also encompass polypeptides encoded by a nucleic acid molecule that hybridizes under highly or moderately stringent conditions to a nucleic acid molecule encoding a polypeptide described in the present disclosure in particular.

As used herein, a "corresponding" amino acid or nucleic acid refers to: an amino acid or nucleotide that has or is expected to have a similar action as a predetermined amino acid or nucleotide in the polypeptide or polynucleotide to be used as a reference for comparison, in a certain polypeptide molecule or polynucleotide molecule; and for enzyme molecules in particular, an amino acid that is present at similar positions in the active site and makes a similar contribution to catalytic activity. For example, as for an antisense molecule, it can be a similar part in the ortholog corresponding to a particular part of the antisense molecule. The corresponding amino acid can be, for example, a specific amino acid that is cysteinylated, glutathioneized, S-S bond formed, oxidized (e.g., methionine side chain oxidation), formylated, acetylated, phosphorylated, glycosylated, myristilized, and the like. Alternatively, the corresponding amino acid can be the amino acid responsible for dimerization. Such a "corresponding" amino acid or nucleic acid may be a region or domain over a certain range. Therefore, in such cases, they are referred to herein as a "corresponding" region or domain.

As used herein, a "corresponding" gene (e.g., polynucleotide sequence or molecule) refers to a gene (e.g., polynucleotide sequence or molecule) of a certain species which has or is expected to have similar action as a predetermined gene in a benchmark species for comparison. When there is a plurality of genes having such action, the corresponding gene refers to a gene having the same evolutionary origin. Hence, a gene corresponding to a certain gene may be an ortholog of such a gene. For example, the cap of an AAV of serotype 1 can correspond to the cap of an AAV of serotype 2. For example, a corresponding gene in a certain virus can be found by searching a sequence database of the virus from using the genetic sequence of a virus that is a benchmark gene of the corresponding gene as a query sequence.

According to the present invention, the term "activity" used herein refers to a function of a molecule in the broadest sense. Activity, although not intended to be limiting, generally includes a biological function, biochemical function, physical function, or chemical function of a molecule. Examples of activity include enzymatic activity, an ability to interact with another molecule, an ability to activate, promote, stabilize, inhibit, suppress, or destabilize a function of another molecule, stability, and an ability to localize at a specific position in a cell. When applicable, the term also relates to a function of a protein complex in the broadest sense.

As used herein, a "biological function", when referring to a certain gene or a nucleic acid molecule or a polypeptide related thereto, refers to a specific function that the gene, the nucleic acid molecule or the polypeptide may have in a living body. Examples of such a function can include, but are not limited to, specific cell surface structure recognizing ability, enzyme activity, binding ability to a particular protein, and the like. In the present disclosure, examples of such a function can include, but are not limited to, a function of recognizing a certain promoter in a particular host cell, and the like. As used herein, a biological function can be exerted by "biological activity". As used herein, "biological activity" refers to the activity possibly possessed by a certain agent (e.g., polynucleotide, protein or the like) in a living body. Biological activity encompasses an activity of exerting a variety of functions (e.g., transcription promoting activity), and also encompasses, for example, an activity of activating or inactivating another molecule by an interaction with a certain molecule. For example, when a certain agent is an enzyme, the biological activity thereof encompasses enzyme activity thereof. In another example, when a certain agent is a ligand, binding to a receptor corresponding to the ligand is encompassed. Such biological activity can be measured by a technique that is well known in the art. Thus, "activity" refers to various measurable indicators, which indicate or reveal a bond (either direct or indirect) or affect a response (i.e., having a measurable effect in response to some exposures of stimuli). Examples thereof include the amount of proteins upstream or downstream in a host cell, or the level of other similar functions.

As used herein, "infectivity" of a virus or a virus vector refers to the ability to introduce a nucleic acid within the virus or the virus vector into a cell by adhesion or membrane fusion of the virus or the virus vector to the cell. A Sendai virus vector may have the same replication ability as that of a wild-type vector, or may be weaker due to a gene mutation. "Replication ability" of a virus or a virus vector refers to the ability to produce an infectious viral particle or virus vector particle in an infected cell.

As used herein, the terms "transformation", "transduction", and "transfection" are interchangeably used with each other, unless specifically noted otherwise, and refer to introduction of a nucleic acid into a host cell (via a virus or a virus vector as required). Any transformation method can be used as long as it is a method for introducing a nucleic acid into a host cell. Examples thereof include various well-known techniques such as use of a cell that has been made competent, electroporation method, method using a particle gun (gene gun), or calcium phosphate method.

As used herein, a "purified" substance or biological agent (e.g., nucleic acid, protein or the like) refers to a substance or a biological agent from which at least a part of an agent naturally accompanying the biological agent has been removed. Thus, the purity of a biological agent in a purified biological agent is generally higher than the purity in the normal state of the biological agent (i.e., concentrated). The term "purified" as used herein refers to the presence of preferably at least 75% by weight, more preferably at least 85% by weight, still more preferably at least 95% by weight, and most preferably at least 98% by weight of a biological agent of the same type. The substance used in the present invention is preferably a "purified" substance.

As used herein, a "pharmaceutical component" refers to any component that can constitute a medicament, and can exemplify, for example, an active ingredient (which itself exhibits a drug efficacy), an additive component (a component which itself is not expected to have a drug efficacy but is expected to play a certain role (e.g., an excipient, a lubricant, a surfactant, or the like) when contained as a medicament), or the like. A pharmaceutical component may be a single substance or may be a combination of multiple substances or agents. Any combination such as a combination of an active ingredient and an additive component or a combination of an adjuvant and an active ingredient can also be included.

As used herein, an "active ingredient" refers to a component which exhibits an intended drug efficacy. A single component or multiple components can fall under the active ingredient.

As used herein, an "additive component" refers to any component which is not expected to have a drug efficacy but plays a certain role when contained as a medicament. Examples thereof can include a pharmaceutically acceptable carrier, a stabilizer, an adjuvant, a solubility improving agent, a solubilizing agent, a diluent, an excipient, a buffer, a binding agent, a diluent, a flavoring agent, and a lubricant.

As used herein, "agent" is used broadly and may be any substance or other elements (e.g., energy such as light, radiation, heat, or electricity) as long as the intended objective can be achieved. Examples of such a substance include, but are not limited to, protein, polypeptide, oligopeptide, peptide, polynucleotide, oligonucleotide, nucleotide, nucleic acid (including for example DNAs such as cDNA and genomic DNA, RNAs such as mRNA), polysaccharide, oligosaccharide, lipid, organic small molecule (e.g., hormone, ligand, information transmitting substance, organic small molecule, molecule synthesized by combinatorial chemistry, small molecule that can be used as pharmaceutical products (e.g., small molecule ligand and the like) and the like), a composite molecule thereof, and a mixture thereof.

As used herein, a "complex" or "composite molecule" refers to any structure comprising two or more parts. For example, when one part is a polypeptide, the other part may be a polypeptide or other substance (e.g., substrate, sugar, lipid, nucleic acid, other carbohydrate or the like). Two or more constituent parts of the complex herein may be covalently bonded or bonded by other bonds (e.g., hydrogen bond, ionic bond, hydrophobic interaction, van der Waals force or the like).

As used herein, a "label" refers to an entity (e.g., substance, energy, electromagnetic wave or the like) for distinguishing a molecule or substance of interest from others. Such a method of labeling can include RI (radioisotope) method, fluorescence method, biotin method, chemiluminescent method and the like. When a plurality of target proteins or agents or means for capturing the same are labeled by a fluorescence method, labeling is performed with fluorescent substances having different fluorescent emission maximum wavelengths. It is preferable that the difference in fluorescent emission maximum wavelengths is 10 nm or greater. Any label that does not affect the function can be used. Examples of a fluorescent substance include Alexa^{™} Fluor. Alexa^{™} Fluor is a water-soluble fluorescent dye obtained by modifying coumarin, rhodamine, fluorescein, cyanine or the like. This is a series compatible with a wide range of fluorescence wavelengths. Relative to other fluorescent dyes for the corresponding wavelength, Alexa^{™} Fluor is very stable, bright and has a low level of pH sensitivity. Examples of a combination of fluorescent dyes with fluorescence maximum wavelength of 10 nm or greater can include a combination of Alexa^{™} 555 and Alexa^{™} 633, a combination of Alexa^{™} 488 and Alexa^{™} 555, and the like. Examples of other fluorescent labels include a cyanine dye (e.g., Cy3, Cy5 or the like of the CyDye^{™} series), rhodamine 6G reagent, N-acetoxy-N2-acetylaminofluorene (AAF), AAIF (iodine derivative of AAF), and the like. The present disclosure can utilize such a label to modify a subject of interest to be detectable by the detecting means to be used. Such modification is known in the art. Those skilled in the art can appropriately carry out such a method in accordance with the label and subject of interest.

As used herein, a "kit" refers to a unit generally providing portions to be provided (e.g., virus vector, manual, and the like) into two or more separate sections. This form of a kit is preferred when a composition that should not be provided in a mixed state and is preferably mixed immediately before use for stability or the like is intended to be provided. Preferably, such a kit advantageously comprises an instruction or manual describing how the provided portions should be used or how a reagent should be handled. When the kit is used herein as a reagent kit, the kit generally comprises an instruction or the like describing how to use a virus vector or the like.

As used herein, an "instruction" is a document with an explanation of the method of use of the present disclosure for a physician or other users. The instruction has a description instructing administration of a medicament or the like of the present disclosure. Further, an instruction may have a description instructing the administration form. The instruction is prepared in accordance with a format defined by a regulatory authority of the country in which the present disclosure is practiced (e.g., Health, Labor and Welfare Ministry in Japan or Food and Drug Administration (FDA) in the US or the like), with an explicit description showing approval by the regulatory authority. The instruction is a so-called package insert and is typically provided in, but not limited to, paper media. The instructions may also be provided in a form such as electronic media (e.g., web sites provided on the Internet or emails).

The term "about" refers to the indicated value plus or minus 10%. When "about" is used for temperature, "about" refers to the indicated temperature plus or minus 5°C. When "about" is used for pH, "about" refers to the indicated pH plus minus 0.5.

### (Preferred embodiments)

Preferred embodiments of the present disclosure are described below. Embodiments provided below are provided to facilitate the understanding of the present disclosure. It is understood that the scope of the present disclosure should not be limited to the following descriptions. Thus, it is apparent that those skilled in the art can make appropriate modifications within the scope of the present disclosure by referring to the descriptions herein. It is understood that the following embodiments can be used alone or in combination.

### (Vector plasmid)

In one aspect, the present disclosure provides a virus vector plasmid which can be replicated in hay bacillus. In one embodiment, the present disclosure provides a virus vector plasmid which can be replicated in *Bacillus subtilis.* Any means for achieving the above objective is intended as the scope of the present disclosure. For example, even without an explicit description, a description of a method using a certain component is also simultaneously intended as an embodiment reflecting other means such as a composition comprising the component, use of the component, and the component for use in the method.

In one aspect, the present disclosure provides a plasmid comprising: a nucleic acid sequence which promotes plasmid replication in hay bacillus; and a nucleic acid sequence required for constituting a virus vector. In one embodiment, a nucleic acid sequence which promotes plasmid replication in hay bacillus can comprise a replication origin point operating in hay bacillus, for example, oriC and a replication origin point contained in a plasmid such as pTB19 (Imanaka, T., et al. J. Gen. Microbiol. 130, 1399-1408. (1984)), pLS32 (Tanaka, T and Ogra, M. FEBS Lett. 422, 243-246. (1998)), or pAMβ1 (Swinfield, T. J., et al. Gene 87, 79-90. (1990)). Examples of a nucleic acid sequence which promotes plasmid replication in hay bacillus include a plasmid or a portion or replication origin point thereof or a variant thereof and the like, which are known to operate the rolling-circle-type replication mechanism, the theta-type replication mechanism, the oriC replication mechanism, or the replication mechanism using a phage or the like. In one embodiment, a nucleic acid sequence which promotes plasmid replication in hay bacillus can have a sequence identical or similar to a known replication origin (e.g., sequence identity of 90% or greater, 95% or greater, 97% or greater, 98% or greater, 99% or greater, or 99.5% or greater).

In one embodiment, the vector plasmid of the present disclosure can have a promoter and/or an enhancer operating in hay bacillus. Examples of a promoter of hay bacillus include Pspac (Yansura, D. and Henner, D. J. Pro. Natl. Acad. Sci, USA 81, 439-443.(1984.)) which is capable of controlling expression in IPTG (isopropyl s-D-thiogalactopyranoside) or Pr promoter (Itaya, M. Biosci. Biotechnol. Biochem. 63, 602-604. (1999)) and the like. A nucleic acid element operating in hay bacillus does not need to be derived from hay bacillus, and a nucleic acid element which highly efficiently operates or the like can be selected. In one embodiment, a replication origin point, a promoter, and/or an enhancer operating in hay bacillus in a vector plasmid are positioned outside a region encoding a genome (which may comprise a modification) of the origin virus of a virus vector or a portion thereof.

In one embodiment, since the vector plasmid of the present disclosure can be created or used in a cell of organisms other than hay bacillus, the vector plasmid can comprise a transcription and translation regulating sequence such as replication origin points, promoters, or transcription termination sequences operating in those organisms. The transcription and translation regulating sequence for each organism is known, and those skilled in the art can appropriately select the sequence. In one embodiment, since the vector plasmid of the present disclosure can be created or replicated in *Escherichia coli,* yeast or the like, the vector plasmid can comprise a transcription and translation regulating sequence operating in these microorganisms. In one embodiment, since the vector plasmid of the present disclosure can be introduced into a producer cell, the vector plasmid can comprise a transcription and translation regulating sequence operating in the producer cell. In one embodiment, the vector plasmid of the present disclosure does not comprise a sequence of a gene of at least a part of a whole genome of the virus.

In one embodiment, nucleic acid sequences required for constituting a virus are collectively placed in a region on a vector plasmid. A vector plasmid with such a structure can be formed by incorporating a nucleic acid fragment comprising nucleic acid sequences required for constituting a virus into the original plasmid. In one embodiment, nucleic acid sequences required for constituting the virus are present in a contiguous region with a base length of about 50% or less, about 40% or less, about 30% or less, about 25% or less, about 20% or less, about 15% or less, about 10% or less, or about 5% or less of the full base length of a vector plasmid. In one embodiment, a sequence which is replicated in hay bacillus may be placed in a contiguous region where a nucleic acid sequence required for constituting a virus is present, or may be placed outside the contiguous region. In one embodiment, each of the elements (e.g., one or more of a nucleic acid sequence encoding a capsid protein of the virus, a nucleic acid sequence encoding a protein which packages, transcribes, and replicates a genome of the virus, and a helper gene) other than the terminal repeat sequences of a nucleic acid sequence required for constituting a virus may be placed between or outside the two terminal repeat sequences. In the vector plasmid of the present disclosure, each element of a nucleic acid sequence required for constituting a virus may be placed in any order and position, and it is understood that various arrangements other than the arrangements specifically shown in the drawings and the like can be used. When a nucleic acid sequence described by a specific function such as a helper gene comprises a plurality of elements, each element in a vector plasmid can be in any order and position, unless specifically noted otherwise. Any elements (e.g., VA, E2A, and E4) may be placed contiguously (without sandwiching another gene therebetween).

In one embodiment, a nucleic acid sequence required for constituting a virus comprises two terminal repeat sequences of the virus, wherein the base length (excluding the terminal repeat sequences themselves) of a region sandwiched by the two terminal repeat sequences can be about 5 kb or greater, about 10 kb or greater, about 20 kb or greater, about 30 kb or greater, about 40 kb or greater, about 50 kb or greater, about 70 kb or greater, or about 100 kb or greater. In one embodiment, a nucleic acid sequence required for constituting a virus comprises two terminal repeat sequences of the virus and the other moiety, wherein the other moiety is positioned outside a region sandwiched by the two terminal repeat sequences. In one embodiment, a nucleic acid sequence required for constituting a virus comprises: a nucleic acid sequence encoding a capsid protein of the virus (e.g., cap, which can be derived from any of serotypes 1 to 12 of an adeno-associated virus or a variant thereof); a nucleic acid sequence encoding a protein which packages, transcribes, and replicates a genome of the virus (e.g., rep, which can be derived from any of serotypes 1 to 12 of an adeno-associated virus or a variant 8 thereof); two terminal repeat sequences of the virus (for example, which are derived from any of serotypes 1 to 12 or a variant thereof); and a helper gene (e.g., at least one of E1A, E1B, E2A, E4, and VA, which can be derived from any of serotypes 1 to 52 of an adenovirus or a variant thereof). In one embodiment, a promotor, a gene of interest, and a terminator are comprised from upstream between 5'ITR and 3'ITR. In particular, it is intended that a nucleic acid sequence encoding a capsid protein of the virus may be a variant of a wild-type sequence (e.g., serotypes 1 to 12 of an adeno-associated virus).

In one embodiment, the vector plasmid of the present disclosure comprises a gene of interest. In one embodiment, a gene of interest can be positioned between two terminal repeat sequences. In one embodiment, a gene of interest in the vector plasmid of the present disclosure can be ultimately contained in a loaded nucleic acid in a virus vector. Such a gene of interest may encode a therapeutic protein, may encode a gene for gene therapy, may encode a gene for gene cell therapy such as CART therapy, may encode a protein non-coding functional RNA (such as rRNA, tRNA, microRNA (miRNA), or IncRNA), or may comprise a transcription and translation regulating sequence such as promoters, terminators, insulators, enhancers, silencers, or replication origin points in combination with or independently of the foregoing. In one embodiment, a gene of interest comprises a virus promoter such as cytomegalovirus promoters, CAG promoters, SV40 promoters, or RSV promoters (upstream of the protein encoding gene as required). In one embodiment, a gene of interest comprises an IRES (upstream of the protein encoding gene as required) . In one embodiment, a gene of interest can be incorporated into a chromosome of a subject to which a virus vector is administered. In this embodiment, the gene of interest may have a function of controlling the expression of a subject's inherent gene, or may result in long-term protein expression. For example, a virus vector based on an adeno-associated virus, a retrovirus or the like can be incorporated into a chromosome of a subject. In one embodiment, a gene of interest can be incorporated into a therapeutic cell (e.g., chromosome) (e.g., via processing of a cell with a virus vector outside of the body). In one embodiment, the vector plasmid of the present disclosure may be configured not to comprise all genes required for the origin virus of a virus vector to proliferate so that the virus vector that is produced would not have a proliferation ability.

In one embodiment, the vector plasmid of the present disclosure may be such that virus vector particles not comprising a nucleic acid in all virus vector particles produced from a producer cell (e.g., HEK293 cell) introduced with the plasmid are about 90% or less, about 80% or less, about 75% or less, about 70% or less, about 65% or less, about 60% or less, about 55% or less, or about 50% or less. In one embodiment, the proportion of the virus vector particles not comprising a nucleic acid is achieved when freeze-thawing the producer cell for three times, followed by processing the cell with Benzonaze^{®} at 37°C for 1 hour and performing centrifugation at 12500 rpm for 30 minutes, followed by obtaining the supernatant, subjecting the supernatant to cesium chloride density-gradient ultracentrifugation at 28000 rpm for 18 hours, and obtaining a virus vector fraction. In one embodiment, when the vector plasmid of the present disclosure is introduced into a producer cell (e.g., HEK293 cell), virus vector particles comprising a nucleic acid comprising all genes of interest in all virus vector particles produced can be about 75% or greater, about 80% or greater, about 85% or greater, about 90% or greater, about 92% or greater, about 95% or greater, about 97% or greater, or about 99% or greater. In one embodiment, when the vector plasmid of the present disclosure is introduced into a producer cell (e.g., HEK293 cell), virus vector particles comprising a nucleic acid derived from the plasmid of the present disclosure other than a desired nucleic acid in all virus vector particles produced can be about 10% or less, about 7% or less, about 5% or less, about 4% or less, about 3% or less, about 2% or less, about 1.5% or less, about 1% or less, about 0.7% or less, about 0.5% or less, about 0.4% or less, about 0.3% or less, about 0.2% or less, or about 0.1% or less.

In one embodiment, a gene of interest in the vector plasmid of the present disclosure can comprise a plurality of genes. In one embodiment, a gene of interest can comprise 2 to 100 genes, for example, 2 or greater, 3 or greater, 4 or greater, 5 or greater, 7 or greater, 10 or greater, 12 or greater, 15 or greater, 20 or greater, 25 or greater, 30 or greater, 40 or greater or 50 or greater, and 100 or less, 90 or less, 80 or less, 70 or less, 60 or less, 50 or less, 40 or less, 35 or less, 30 or greater, 25 or less, 20 or less, 17 or less, 15 or less, 12 or less or 10 or less genes. In one embodiment, a gene of interest in the vector plasmid of the present disclosure can comprise a base length of about 0.1 to 1000 kbp, for example, about 0.1 kbp or greater, about 0.3 kbp or greater, about 1 kbp or greater, about 2 kbp or greater, about 5 kbp or greater, about 7 kbp or greater, about 10 kbp or greater, about 20 kbp or greater, about 50 kbp or greater or about 100 kbp or greater, and about 1000 kbp or less, about 700 kbp or less, about 500 kbp or less, about 200 kbp or less, about 100 kbp or less, about 70 kbp or less, about 50 kbp or less, about 20 kbp or less or about 10 kbp or less. Since the vector plasmid of the present disclosure can be constructed in a complex structure comprising a plurality of genes by OGAB method, a gene of interest can also be constructed in a complex structure. Since the size of the loaded nucleic acid can be restricted by the type of the virus vector, the type of the virus vector may be selected depending on the size of the gene of interest. In one embodiment, a gene of interest comprises a plurality of genes, whereby a nucleic acid with a high functionality can be delivered to the subject, such as a combination of a promoter specific to a tissue (e.g., cancer tissue) of a subject or specific to timing and a therapeutic gene (such as therapeutic protein encoding sequence, gene for gene therapy, or gene for gene cell therapy) operably linked thereto or a sequence encoding a series of enzymes that enable coordinate expression of a series of enzymes controlling a metabolic cascade.

In one embodiment, examples of a protein encoded by a gene of interest in the vector plasmid of the present disclosure include a therapeutic polypeptide (e.g., replacement therapy), an immunogenic polypeptide (e.g., a pathogen polypeptide or a cancer antigen polypeptide), and the like. Examples of a therapeutic polypeptide include cystic fibrosis transmembrane regulator protein (CFTR), dystrophin (mini-dystrophin and micro-dystrophin), myostatinpropeptide, follistatin, soluble activin type II receptor, IGF-1, anti-inflammatory polypeptide, sarcospan, utrophin, mini-utrophin, coagulation factor (e.g., Factor VIII, Factor IX, Factor X, or the like), erythropoietin, angiostatin, endostatin, catalase, tyrosine hydroxylase, superoxide dismutase, leptin, LDL receptor, lipoprotein lipase, ornithine transcarbamylase, β-globin, α-globin, spectrin, α1-antitrypsin, adenosine deaminase, hypoxanthine-guanine phosphoribosyltransferase, β-glucocerebrosidase, sphingomyelinase, lysosomal hexosaminidase A, branched chain ketoacid dehydrogenase, RP65 protein, cytokine (e.g., α-interferon, β-interferon, interferon-γ, interleukin-2, interleukin-4, granulocyte-macrophage colony-stimulating factor, lymphotoxin, or the like), peptide growth factor, neurotrophic factor and hormone (e.g., somatotropin, insulin, insulin-like growth factors 1 and 2, platelet-derived growth factor, epidermal growth factor, fibroblast growth factor, nerve growth factor, neurotrophic factor-3 and -4, brain-derived neurotrophic factor, bone morphogenetic protein, glial-derived growth factor, transforming growth factor-α and -β, or the like), lysosomal acid α-glucosidase, α-galactosidase A, soluble tumor necrosis growth factor α receptor, S100A1, parvalbumin, adenylyl cyclase type 6, anti-inflammatory factor, anti-myostatin protein, aspartoacylase, suicide gene product (e.g., thymidine kinase, cytosine deaminase, diphtheria toxin, or tumor necrosis factor), tumor suppressor gene product (e.g., p53, Rb, or Wt-1), TRAIL, FAS-ligand, and the like.

Examples of a pathogen polypeptide include a cell surface protein of a pathogenic organism such as bacteria, fungi, or parasites, and a protein (e.g., spike protein, envelope protein, capsid protein, and the like) expressed on the surface of a virus. Specific examples of a pathogen polypeptide include orthomyxovirus immunogen (e.g., influenza virus hemagglutinin (HA) or nucleoprotein), lentivirus immunogen (e.g., HIV or SIV envelope GP160 protein, matrix/capsid protein, gag, pol, or env gene product), arenavirus immunogen (e.g., Lassa fever virus nucleocapsid protein or envelope glycoprotein), poxvirus immunogen (e.g., vaccinia L1 or L8 gene product), flavivirus immunogen (e.g., yellow fever virus or Japanese encephalitis virus immunogen), filovirus immunogen (e.g., Ebola virus or Marburg virus immunogen such as NP and GP gene products), bunyavirus immunogen (e.g., RVFV, CCHF, or SFS virus immunogen), coronavirus immunogen (e.g., human coronavirus immunogen such as human coronavirus envelope glycoprotein), polio immunogen, herpes virus immunogen (e.g., CMV, EBV, or HSV immunogen), mumps virus immunogen, measles virus immunogen, rubella virus immunogen, diphtheria toxin or other diphtheria immunogens, pertussis antigen, and hepatitis (e.g., hepatitis A, hepatitis B, hepatitis C, or the like) immunogen, and the like.

Examples of a cancer antigen polypeptide include BRCA1 gene product, BRCA2 gene product, gp100, tyrosinase, GAGE-1/2, BAGE, RAGE, LAGE, NY-ESO-1, CDK-4, β-catenin, MUM-1, caspase-8, KIAA0205, HPVE, SART-1, PRAME, p15, melanoma tumor antigen, MART-1, gp100 MAGE-1, MAGE-2, MAGE-3, CEA, TRP-1, TRP-2, P-15, tyrosinase, HER-2/neu gene product, CA125, LK26, FB5 (endosialin), TAG72, AFP, CA19-9, NSE, DU-PAN-2, CA50, SPan-1, CA72-4, HCG, STN (sialyl Tn antigen), c-erbB-2 protein, PSA, L-CanAg, estrogen receptor, milk fat globulin, p53 tumor suppressor protein, mucin antigen, telomerase, nuclear matrix protein, prostatic acid phosphatase, papilloma virus antigen, and the like.

In one embodiment, a gene of interest can be a gene for treating a specific disease (e.g., gene therapeutic gene). Those skilled in the art can appropriately select a gene that should be selected for a specific disease. Examples of such a disease include infections due to various types of pathogens, cystic fibrosis, hemophilia A, hemophilia B, thalassemia, anemia, Alzheimer's disease, multiple sclerosis, Parkinson's disease, Huntington's disease, amyotrophic lateral sclerosis, spinal muscular atrophy, epilepsy, cancer (such as melanoma, adenocarcinoma, thymoma, lymphoma, sarcoma, lung cancer, liver cancer, colon cancer, leukemia, uterine cancer, breast cancer, prostate cancer, ovarian cancer, cervical cancer, bladder cancer, kidney cancer, pancreatic cancer, or brain cancer), diabetes, muscular dystrophy, Gaucher disease, Hurler disease, adenosine deaminase deficiency, glycogen storage disease, congenital emphysema, Lesch-Nyhan syndrome, Niemann-Pick disease, Tay-Sachs disease, Angelman syndrome, maple syrup urine disease, age-related macular degeneration, amaurosis, diabetic retinopathy, retinal degenerative disease, astrocytomas, glioblastoma, heart failure, peripheral artery disease, arthritis, joint disorder, intimal hyperplasia, AIDS, muscle wasting, kidney deficiency, hepatitis, LDL receptor deficiency, hyperammonemia, Krabbe's disease, Batten disease, spinal cerebral ataxias, phenylketonuria, autoimmune disease, amino acid metabolic disorder, organic acid metabolic disorder, fatty acid metabolic disorder, mitochondrial disease, sugar metabolic disorder, lysosomal disease, peroxisomal disorder, metal metabolic disorder, purine pyrimidine metabolic disorder, vitamin metabolic disorder, neurotransmitter disorder, lipid metabolic disorder, connective tissue disorder, congenital porphyria, α1-antitrypsin deficiency, lysosomal storage disease, mucopolysaccharidosis disorder, Fabry disease, Canavan disease, Leigh disease, Refsum's disease, Tourette's Syndrome, primary lateral sclerosis, progressive muscular atrophy, Pick disease, muscular dystrophy, myasthenia gravis, Binswanger disease, cerebral infarction, mood disorder, depression, bipolar affective disorder, persistent affective disorder, secondary mood disorder, schizophrenia, drug dependence, anxiety, obsessional disorder, somatoform disorder, dissociative disorder, grief, post-partum depression, hallucination, delusion, dementia, paranoia, autism spectrum disorder, attention-deficit hyperactivity disorder, psychosexual disorder, sleeping disorder, pain disorder, eating disorder, weight disorder, obesity, cachexia, anorexia nervosa, bulimia, and the like. In one embodiment, a gene of interest can be incorporated into a therapeutic cell (e.g., chromosome) which is used for CART therapy or the like (e.g., via processing of a cell with a virus vector outside of the body).

### (Virus vector)

The vector plasmid of the present disclosure is used for producing a virus vector. In one embodiment, the present disclosure provides a method for creating a virus vector by using the vector plasmid of the present disclosure. In one embodiment, the present disclosure provides a composition comprising a virus vector or a virus vector created in this manner. Examples of a virus vector include a virus vector based on a retrovirus, a lentivirus, an adeno-associated virus, an adenovirus, or a Sendai virus.

In one embodiment, the vector plasmid of the present disclosure is introduced into a producer cell to produce a virus vector. Examples of a producer cell include, but are not limited to, 911 cells, PER.C6 cells, E1-transformed amniocytes, E1-transformed A549 cells, GH329: HeLa cells, HEK293 cells, IT293SF cells, HEK293T, HEK293F, Vero cells, CHO cells, Sf9 cells, Freestyle^{™} 293-F, Expi293-F^{™}, Expi293 inducible, Expi293 NGT-Viral Production Cells 1.0, Viral Production Cells 2.0, AAVpro^{®} 293T Cell Line, Lenti-X^{™} 293T Cell Line, Freestyle^{™} CHO-S cells, ExpiCHO-S^{™} , and the like. Any known suitable producer cell can be selected depending on the type of the virus vector that is produced. In one embodiment, the vector plasmid of the present disclosure may be deficient in a few (e.g., one, two, three, four, or five) of the genes of the origin virus of a virus vector and comprise all of the rest of the genes. In one embodiment, the vector plasmid of the present disclosure is deficient in a part of a gene set required for producing a virus vector, wherein the gene in the gene set which is deficient in the vector plasmid is supplied in a producer cell in trans with respect to the vector plasmid. In one embodiment, the introduction of the vector plasmid of the present disclosure alone into a producer cell without being combined with another plasmid can enable virus vector production. In one embodiment, the vector plasmid of the present disclosure can be introduced into a producer cell that expresses all of the genes in the gene set required for producing a virus vector which are deficient in the vector plasmid. For example, the vector plasmid of the present disclosure comprising a cap and a rep of an AAV and E2A, E4, and VA of an adenovirus alone can produce a virus vector by being introduced into an HEK293 cell expressing E1A and E1B of an adenovirus. In one embodiment, the vector plasmid of the present disclosure can be introduced into a producer cell together with another nucleic acid comprising a gene in a gene set required for producing a virus vector which is deficient in the vector plasmid or a product of the gene (e.g., viral particle).

In one embodiment, a virus vector can be created by introducing a vector plasmid into a producer cell infected with the origin virus of the virus vector and causing homologous recombination in the cell. The virus vector plasmid which is used in this embodiment comprises a gene of interest and a nucleic acid sequence having homology to any region (e.g., a region between genes) of the genome of the origin virus. In one embodiment, a vector plasmid can have a structure comprising a gene of interest between any genes of the genome of the origin virus of a virus vector to be produced. In one embodiment, at least a part of a nucleic acid contained in a vector plasmid is incorporated into a chromosome of a producer cell.

In one embodiment, the vector plasmid of the present disclosure comprises segments (such as LTR in a retrovirus or ITR in an AAV virus) for cutting out a loaded nucleic acid of the origin virus of a virus vector to be produced. In one embodiment, a gene of interest is contained between these segments. In one embodiment, the vector plasmid of the present disclosure comprises a gene of interest and a promoter and/or a terminator (e.g., those operable in a subject targeted by a virus vector) operably linked thereto between these segments. In one embodiment, the vector plasmid of the present disclosure does not comprise a gene required for replication of the origin virus of a virus vector between these segments. In one embodiment, the vector plasmid of the present disclosure comprises a packaging signal of the origin virus of a virus vector to be produced.

A virus vector to be produced may have a capsid, wherein the capsid can be involved in binding with a tissue or cell. Thus, targeting of a virus vector to a tissue or cell can be adjusted by modifying a capsid to modify the binding property with the tissue or cell (or surface structure thereof). In one embodiment, the vector plasmid of the present disclosure may have a gene encoding a capsid, wherein the capsid may be modified. Examples of capsid modification for adjusting tissue or cell targeting include substitution or fusion with another protein (such as another virus capsid (e.g., a capsid of a virus of another serotype, VSV-G), an antigen-binding region of an antibody, or a ligand protein in a subject organism (ligand to a cancer antigen)). Further, a virus vector having an envelope can comprise a cell membrane component of a producer cell in the envelope. Thus, targeting of a virus vector having an envelope to a tissue or cell can be adjusted by modifying a cell membrane component of a producer cell. In one embodiment, a virus vector may be designed to target a nerve cell (such as cells of the peripheral nervous system or the central nervous system, or brain cells such as neurons and oligodendrocytes), a lung cell, a cell of an eye (such as retinal cells, retinal pigment epithelial cells, or corneal cells), an epithelial cell (e.g., epithelial cells of the intestines or respiratory organs), a muscle cell (e.g., skeletal muscle cells, myocardial cells, smooth muscle cells, or diaphragm muscle cells), a dendritic cell, a pancreatic cell (such as islet cells), a hepatocyte, a myocardial cell, a bone cell (e.g., bone marrow stem cells), a hematopoietic stem cell, a splenocyte, a keratinocyte, a fibroblast, an endothelial cell, a prostate cell, a germ cell, a cancer cell, or the like. The surface structure (such as receptors) specific to each cell is known, and those skilled in the art can appropriately select a protein which strongly or specifically binds to the surface structure.

In one embodiment, the vector plasmid of the present disclosure may comprise a gene encoding a protein resulting from attenuating a protein of the origin virus of a virus vector to be produced. The vector plasmid of the present disclosure can comprise a gene encoding any known attenuated virus protein.

In one embodiment, the present disclosure provides a composition comprising a plasmid comprising a population of the vector plasmids of the present disclosure. In one embodiment, a composition comprising a plasmid may have a CCC (covalently closed circular) purity of a plasmid that is about 60% or greater, about 70% or greater, about 75% or greater, about 80% or greater, about 85% or greater, about 90% or greater, or about 95% or greater.

### * Adenovirus vector

An adenovirus is a virus of the genus *Mastadenovirus* in the family *Adenoviridae.* A viral particle consists of a nucleocapsid and a double-stranded linear DNA genome and has an icosahedron structure of 90 to 100 nm. Infection with an adenovirus is initiated by adsorption of the virus capsid to a coxackie-adenovirus receptor (CAR) on the cell surface, and the virus can then enter the cell via an integrin on the cell surface. Subsequently, the virus genome that escaped from a lysosome reaches the inside of the nucleus and can result in replication of the virus genome. First, E1A protein is expressed to activate expression of other early proteins, E1B, E2, E3, and E4, thereby initiating virus replication. The replication of the virus genome is initiated after a terminal protein (TP) expressed from E2 and a deoxycytidine are covalently bound and a polymerase is further bound thereto to form a complex. The genome also comprises five late transcriptional units (L1, L2, L3, L4, and L5), which encode structural proteins including penton (L2), hexon (L3), scaffold protein (L4), and fiber protein (L5) and are under the control of a single promoter. Both ends of the genome comprise an inverted terminal repeat (ITR) required for replication of the virus. After the structural proteins of the virus are translated in the cytoplasm, the structural proteins migrate into the nucleus to constitute a viral particle, and recognize a packaging signal (ψ) of the virus genome to package the genome. Further, an adenovirus produces protein non-coding VA RNA, which is encoded by a VA gene. An adenoviral particle can have a capsid of L2 and L3.

To date, 52 human adenovirus antigen types have been identified, which are classified into 6 subgroups based on hemagglutination reaction properties and sequence homology: subgroup A (e.g., serotypes 12, 18, and 31); subgroup B (e.g., serotypes 3, 7, 11, 14, 16, 21, 34, 35, and 50); subgroup C (e.g., serotypes 1, 2, 5, and 6); subgroup D (e.g., serotypes 8, 9, 10, 13, 15, 17, 19, 20, 22 to 30, 32, 33, 36 to 39, and 42 to 48); subgroup E (e.g., serotype 4); subgroup F (e.g., serotypes 40 and 41); and unclassified serotype group (e.g., serotypes 49 and 51).

In one embodiment, an adenovirus vector plasmid comprises: at least one of nucleic acid sequences required for constituting an adenovirus vector; and a gene of interest. In one embodiment, an adenovirus vector plasmid comprises a gene of interest between 5'ITR and 3'ITR. In one embodiment, an adenovirus vector plasmid comprises a gene of interest, a promoter, and a terminator between 5'ITR and 3'ITR. In one embodiment, an adenovirus vector plasmid does not comprise one or more (e.g., all) of nucleic acid sequences required for constituting an adenovirus vector between 5'ITR and 3'ITR. In one embodiment, an adenovirus vector plasmid is configured to function in a producer cell wherein the vector plasmid and the producer cell comprise a nucleic acid sequence required for constituting an adenovirus vector. In one embodiment, one or more (e.g., all) of genes not contained in a vector plasmid among nucleic acid sequences required for constituting an adenovirus vector may be encoded by a chromosome of a producer cell.

In one embodiment, a nucleic acid sequence required for constituting an adenovirus vector can comprise a gene encoding E1A, E1B, E2A, E2B, E3, E4, L1, L2, L3, L4, L5, IX, and IVa2. In one embodiment, a nucleic acid sequence required for constituting an adenovirus vector can be all genes of an adenovirus other than E3. In one embodiment, a nucleic acid sequence required for constituting an adenovirus vector can comprise: a nucleic acid sequence encoding a capsid protein (L2 and L3); a nucleic acid sequence encoding a protein which packages, transcribes, and replicates a genome (E1A, E1B, E2A, E2B, and E4); two terminal repeat sequences (5'ITR and 3'ITR); and a nucleic acid sequence of a helper gene. In one embodiment, a nucleic acid sequence of a helper gene can be all adenovirus genes other than L2, L3, E1A, E1B, E2A, E3, E2B, E4, 5'ITR, and 3'ITR. In one embodiment, an adenovirus vector plasmid does not need to comprise one or more of VA, E1A, E1B, E2A, E2B, and E4, while in one embodiment, a nucleic acid sequence required for constituting an adenovirus vector comprises the same. In a specific embodiment, an adenovirus vector plasmid does not comprise E1A and E1B. In one embodiment, an adenovirus vector plasmid is deficient in E1A, wherein a gene of interest may be inserted into this deficiency site. It is known that VA RNA interacts with exportin, RISK, Dicer or the like in humans. Side effects on a cell infected with an adenovirus vector can be reduced by causing an adenovirus vector plasmid to be deficient in VA. Since E1A, E1B, E2A, E2B, and E4 can be genes required for replication of an adenovirus, the replication ability of an adenovirus vector plasmid deficient in these genes in an infected cell can be reduced.

In one embodiment, the adenovirus vector of the present disclosure can be derived from human subgroup C, e.g., serotype 2 or serotype 5. In one embodiment, the adenovirus vector of the present disclosure can be derived from serotype 12 (subgroup A), serotype 7 or serotype 35 (subgroup B), serotype 30 or serotype 36 (subgroup D), serotype 4 (subgroup E), or serotype 41 (subgroup F). Examples of a producer cell for producing an adenovirus vector include cells such as HEK293, HEK293T, HEK293F, Hela, or Sf9.

### * Adeno-associated virus vector

An adeno-associated virus (AAV) is a linear single-stranded DNA virus of the genus *Dependovirus* in the family *Parvoviridae.* A viral particle is 20 to 26 nm in diameter. An adenovirus element is required for proliferation of an AAV. A T-shaped hairpin structure called ITR (inverted terminal repeat) is present at both terminals of an AAV genome. The ITR moiety is a replication origin, which plays a role of a primer. Further, the ITR is also required for packaging into a viral particle and incorporation into a chromosomal DNA of a host cell. A rep gene encoding non-structural proteins, in other words, regulatory proteins (Rep78, Rep68, Rep52, and Rep40) responsible for replication and transcription, is present in the left half of the genome while a cap gene encoding three capsid proteins that are structural proteins (VP1, VP2, and VP3), is present in the right half of the genome.

A life cycle of an AAV is divided into latent infection and lytic infection. The former refers to infection with an AAV alone, characterized in that the virus is incorporated into AAVS1 region (19q13.3-qter) of the long arm of chromosome 19 of a host cell. This incorporation results from non-homologous recombination, in which Rep is involved. It is reported that Rep78/Rep68 bind to a base sequence (GAGC repeat sequence) that AAVS1 region and Rep binding region of ITR have in common. Thus, it is believed that, when a wild-type AAV infects a target cell, Rep binds to ITR and AAVS1 of the AAV, and site-specific incorporation of the AAV genome into chromosome 19 occurs via Rep. When a helper virus such as an adenovirus simultaneously infects, or when the helper virus further superinfects the cell that is latently infected with an AAV, AAV replication occurs and a large amount of viruses are released due to cell disruption (lytic infection) .

In one embodiment, an AAV vector plasmid comprises: at least one of nucleic acid sequences required for constituting an AAV vector; and a gene of interest. In one embodiment, an AAV vector plasmid comprises a gene of interest between 5'ITR and 3'ITR. In one embodiment, an AAV vector plasmid comprises a gene of interest, a promoter, and a terminator between 5'ITR and 3'ITR. In one embodiment, an AAV vector plasmid does not comprise one or more (e.g., all) of nucleic acid sequences required for constituting an AAV vector between 5'ITR and 3'ITR. In one embodiment, an AAV vector plasmid is configured to function in a producer cell wherein the vector plasmid and the producer cell comprise a nucleic acid sequence required for constituting an AAV vector. In one embodiment, one or more (e.g., all) of genes not contained in a vector plasmid among nucleic acid sequences required for constituting an AAV vector may be encoded by a chromosome of a producer cell.

In one embodiment, a nucleic acid sequence required for constituting an AAV vector can be 5'ITR, rep, cap, AAP (assembly activating protein), MAAP (membrane-associated accessory protein), and 3'ITR of an AAV, and E1A, E1B, E2A, VA, and E4 of an adenovirus. In one embodiment, a nucleic acid sequence required for constituting an AAV vector can comprise: a nucleic acid sequence encoding an AAV capsid protein (cap); a nucleic acid sequence encoding an AAV protein which packages, transcribes, and replicates a genome; two terminal repeat sequences (5'ITR and 3'ITR) of an AAV (rep); and a nucleic acid sequence of a helper gene (E1A, E1B, E2A, VA, and E4 of an adenovirus). In one embodiment, an AAV vector plasmid does not need to comprise one or more of rep, cap, VA, E1A, E1B, E2A, and E4, while in one embodiment, a nucleic acid sequence required for constituting an adenovirus vector comprises the same.

For an AAV, 1 to 12 serotypes based on capsids have been reported. Further, serotypes rh10, DJ, DJ/8, PHP.eB, PHP.S, AAV2-retro, AAV2-QuadYF, AAV2.7m8, AAV6.2, rh.74, AAV2.5, AAV-TT, and Anc80 have been also reported. The AAV vector of the present disclosure may be created based on an AAV of a suitable serotype depending on the target tissue. For example, the relationship of (serotype):(target tissue) can be selected as follows; (AAV1):(muscle, liver, respiratory tract, and nerve cell), (AAV2):(muscle, liver, and nerve cell), (AAV3):(muscle, liver, and nerve cell), (AAV4):(muscle and ventricular ependymal cell), (AAV5):(muscle, liver, nerve cell, glial cell, and respiratory tract), (AAV6):(muscle, liver, respiratory tract, and nerve cell), (AAV7):(muscle and liver), (AAV8):(muscle and liver), (AAV9) : (muscle, liver, and respiratory tract) . Examples of a producer cell for producing an AAV vector include cells such as HEK293, HEK293T, HEK293F, Hela, or Sf9. Apart from a wild type, examples of a capsid of an AAV include a capsid with a targeting mutation (such as AAV2i8, AAV2.5, AAV-TT, or AAV9.HR), a capsid with a random mutation (such as AAV-PHP.B), and a capsid designed in silico (such as Anc80). In one embodiment, the AAV vector of the present disclosure may comprise these modified capsids, and the AAV vector plasmid of the present disclosure may be constructed to encode these modified capsids. When it is described herein that a nucleic acid sequence is derived from a specific serotype, it is intended that the nucleic acid sequence may encode a wild-type capsid or may encode a capsid with the modification as described above based on a wild-type capsid.

### * Retrovirus vector

As used herein, a "retrovirus" generally refers to a virus in the family *Retroviridae.* A retrovirus is a double-stranded RNA envelope virus, mainly characterized by the ability to reverse-transcribe the genome from RNA to DNA. The length of a virion is about 100 to 120 nm in diameter. A retrovirus contains a dimeric genome of identical positive RNA strands forming a complex with a nucleocapsid protein. The genome is enclosed in a capsid containing enzymatic proteins required for viral infection, in other words, reverse transcriptase, integrase, and protease. A matrix protein forms a layer outside the capsid core that interacts with the envelope, a lipid bilayer derived from the host cell membrane, which surrounds the viral nucleus particle. A viral envelope glycoprotein which recognizes a specific receptor on the host cell and initiates the infection process is anchored on the bilayer. An envelop protein is formed of two subunits, the transmembrane (TM) which anchors the protein inside the lipid membrane and the surface (SU) which binds to the cellular receptor.

Examples of a retrovirus include, but are not limited to, murine leukemia virus (MLV), human immunodeficiency virus (HIV), equine infectious anemia virus (EIAV), mouse mammary tumor virus (MMTV), Rous sarcoma virus (RSV), Fujinami sarcoma virus (FuSV), Moloney-murine leukemia virus (Mo-MLV), FBR murine sarcoma virus (FBR MSV), Moloney-murine sarcoma virus (Mo-MSV), Abelson-murine leukemia virus (A-MLV), avian myelocytomatosis virus 29 (MC29), and avian encephalomyelitis virus (AEV), and lentivirus. Lentiviruses can be classified into "primate" and "non-primate". Examples of a primate lentivirus include human immunodeficiency virus (HIV), which is a substance causing human acquired immunodeficiency syndrome (AIDS), and simian immunodeficiency virus (SIV). The non-primate lentivirus group includes the prototype "slow virus" visna/maedi virus (VMV), and associated caprine arthritis encephalitis virus (CAEV), equine infectious anemia virus (EIAV), and feline immunodeficiency virus (FIV) that was described more recently, and bovine immunodeficiency virus (BIV).

During the infection process, a retrovirus first attaches to a specific cell surface receptor. After entering a susceptible host cell, the retrovirus RNA genome is copied to DNA by a reverse transcriptase. This DNA is transported to the host cell nucleus and then integrated into the host genome. This state is called provirus. The provirus is stable in the host chromosome during cell division, and is transcribed like other cell proteins. The provirus can encode a protein and packaging mechanism required for creating more viruses, bud, and leave the cell. When retroviruses bud from the host cell, they comprise a host cell lipid membrane. In this manner, the host cell-derived membrane protein becomes a part of the retroviral particles.

The genome of a retrovirus comprises four genes, gag (group-specific antigen), pro (protease), pol (polymerase), and env (envelope). The gag sequence encodes three primary structural proteins, matrix protein, nucleocapsid protein, and capsid protein. The pro sequence encodes protease which is responsible for cleaving Gag and Gag-Pol during particle assembly, budding, and maturation. The pol sequence encodes the enzymes of the reverse transcriptase and integrase, wherein the former catalyzes the reverse transcription of the virus genome from RNA to DNA during the infection process, and the latter is responsible for processing LTRs and integrating the provirus DNA into the host cell genome. The env sequence encodes both SU and TM subunits of the envelope glycoprotein. While the ability of a retrovirus to bind to a target host cell thereof using a specific cell surface receptor is given by the surface component (SU) of the Env protein, the ability of a retrovirus to enter the cell via membrane fusion can be imparted by the membrane-anchored trans-membrane component (TM). The retrovirus genome contains an element required for promoting gene expression, reverse transcription, and integration into the host cell chromosome. Examples of the element include non-coding cis-acting sequences such as two LTRs (long terminal repeats), a packaging signal (ψ) sequence required for specific packaging of the viral RNA into a newly formed virion, and a polypurine tract (PPT) which functions as a site for initiating positive strand DNA synthesis during the reverse transcription. A long terminal repeat (LTR) is about 600 nt in length, wherein the U3 region is 450 nt in length, the R sequence is 100 nt in length, and the U5 region is about 70 nt in length.

In addition to gag, pro, pol, and env, the genome of a complex retrovirus such as lentivirus can comprise an accessory gene which regulates virus gene expression and assembly of infectious particles and modulates virus replication in an infected cell. A typical lentivirus is HIV-1. A lentivirus can comprise two control genes, tat and rev. For example, HIV-1 further comprises vif, vpr, vpu, and nef. Other examples of an accessory gene include vpx and the like. These accessory genes are involved in synthesis and processing of a viral RNA and control of other replication functions. In particular, HIV also includes a structural landmark (TAR, RRE, PE, SLIP, CRS, or INS) or the like. A lentiviral particle can comprise a capsid protein of p24.

In one embodiment, a retrovirus vector plasmid comprises: at least one of nucleic acid sequences required for constituting a retrovirus vector; and a gene of interest. In one embodiment, a retrovirus vector plasmid comprises a gene of interest between 5'LTR and 3'LTR. In one embodiment, a retrovirus vector plasmid may comprise a primer binding site (PBS) and/or a polypurine tract (PPT) between 5'LTR and a gene of interest. In one embodiment, a retrovirus vector plasmid may comprise a woodchuck hepatitis virus posttranscriptional regulatory element (WPRE) between a gene of interest and 3'LTR. In one embodiment, a retrovirus vector plasmid is configured to function in a producer cell wherein the vector plasmid and the producer cell comprise a nucleic acid sequence required for constituting a retrovirus vector. In one embodiment, one or more (e.g., all) of genes not contained in a vector plasmid among nucleic acid sequences required for constituting a retrovirus vector may be encoded by a chromosome of a producer cell. In one embodiment, when a retrovirus vector is constructed, env may be replaced with a gene encoding VSV-G (glycoprotein G of a vesicular stomatitis virus (VSV)). In one embodiment, a retrovirus vector (including a lentivirus) plasmid may additionally comprise a VSV-G gene. In one embodiment, when a retrovirus vector (including a lentivirus) is constructed, a fusion glycoprotein (FuG-B) in which the glycoprotein gene (RV-G) of a rabies virus or the intracellular domain of RV-G has been substituted with that of the vesicular stomatitis virus glycoprotein (VSV-G) may be used in addition to or instead of env, and the retrovirus vector (including a lentivirus) plasmid may comprise these genes.

In one embodiment, a nucleic acid sequence required for constituting a retrovirus vector can comprise: a nucleic acid sequence encoding a capsid protein (gag); a nucleic acid sequence encoding a protein which packages, transcribes, and replicates a genome (pol); two terminal repeat sequences (5'LTR and 3'LTR); and a nucleic acid sequence of a helper gene. In one embodiment, a nucleic acid sequence of a helper gene can be packaging signal (ψ) sequence, pro, pol, and env. In one embodiment, a retrovirus vector plasmid does not need to comprise one or more of gag, pro, pol, and env, while in one embodiment, a nucleic acid sequence required for constituting a retrovirus vector comprises the same. Since pol can be a gene required for replication of a retrovirus, the replication ability of a retrovirus vector plasmid deficient in said gene in an infected cell can be reduced. In one embodiment, a retrovirus vector plasmid does not comprise one or more (e.g., all) of nucleic acid sequences required for constituting a retrovirus vector between 5'LTR and 3'LTR. In one embodiment, a retrovirus vector plasmid comprises a gene of interest between 5'LTR and 3'LTR. In one embodiment, a retrovirus vector plasmid comprises a gene of interest, a promoter, and a terminator between 5'LTR and 3'LTR. In one embodiment, a retrovirus vector plasmid may be modified to be deficient in the U3 region of an LTR. Examples of a producer cell for producing a retrovirus vector include cells such as HEK293T.

In one embodiment, a nucleic acid sequence required for constituting a lentivirus vector can comprise: a nucleic acid sequence encoding a capsid protein (gag); a nucleic acid sequence encoding a protein which packages, transcribes, and replicates a genome (at least one of pol, rev, packaging signal (ψ) sequence, APP, and MAAP); two terminal repeat sequences (5'LTR and 3'LTR); and a nucleic acid sequence of a helper gene. In one embodiment, a nucleic acid sequence of a helper gene can be pro, pol, and env. In one embodiment, a lentivirus vector plasmid does not need to comprise one or more of rev, gag, pro, pol, and env, while in one embodiment, a nucleic acid sequence required for constituting a lentivirus vector comprises the same. Since pol and rev can be genes required for replication of a lentivirus, the replication ability of a lentivirus vector plasmid deficient in these genes in an infected cell can be reduced. In one embodiment, a lentivirus vector plasmid may comprise one or more of tat, vif, vpr, vpu, nef, vpx, TAR, RRE, PE, SLIP, CRS, INS, APP, MAAP, RPE, PPT, PRE, WRPE, and oPRE. In one embodiment, a lentivirus vector plasmid does not comprise one or more (e.g., all) of nucleic acid sequences required for constituting a lentivirus vector between 5'LTR and 3'LTR. In one embodiment, a lentivirus vector plasmid comprises a gene of interest between 5'LTR and 3'LTR. In one embodiment, a lentivirus vector plasmid comprises a gene of interest, a promoter, a terminator, and a WPRE between 5'LTR and 3'LTR. In one embodiment, a lentivirus vector plasmid may be modified to be deficient in the U3 region of an LTR and TAT. In one embodiment, a retrovirus vector plasmid may be modified to be deficient in the U3 region of an LTR. Examples of a producer cell for producing a lentivirus vector include cells such as HEK293T, HEK293, or Hela.

### * Sendai virus vector

A Sendai virus (SeV) is a negative strand RNA virus that is classified into the genus *Respirovirus* in the family *Paramyxoviridae.* A Sendai virus can also infect a non-dividing cell including neurons. After infection with a Sendai virus, the virus genome stays in the cytoplasm as RNA without being incorporated into the chromosome of the host cell.

Examples of a gene encoding the protein of a Sendai virus include N, P, M, F, HN, and L genes. N, P, M, F, HN, and L genes encode nucleocapsid protein, phosphoprotein, matrix protein, fusion protein, hemagglutinin-neuraminidase protein, and large protein, respectively. In general, six genes encoding N (nucleocapsid protein), P (phosphoprotein), M (matrix protein), F (fusion protein), HN (hemagglutinin-neuraminidase), and L (large protein) are arranged, following a short 3' leader region (LE), on the genome of a wild-type Sendai virus. A short 5' trailer region (TR) is present on the other end. Accessory proteins called C and V are also translated from the region of the P gene.

A Sendai virus expresses the gene by both a tubulin in the cytoplasm of a host cell and an RNA polymerase (L protein) of the Sendai virus. A Sendai virus does not interact with the genome of a host cell and is not pathogenic to humans. These features of a Sendai virus suggest the safety of a Sendai virus vector to humans. M protein, F protein, and HN protein can be responsible for formation of a viral particle and viral infection of a Sendai virus. N protein, P protein, and L protein can be responsible for expression and replication of a virus genome.

In one embodiment, a nucleic acid transcribed from a Sendai virus vector plasmid in a producer cell becomes a loaded nucleic acid of a Sendai virus vector. Thus, the following features relating to a Sendai virus vector plasmid can also be features of a loaded nucleic acid of a Sendai virus vector.

In one embodiment, a Sendai virus vector plasmid comprises: at least one of nucleic acid sequences required for constituting a Sendai virus vector; and a gene of interest. In one embodiment, a gene of interest can be positioned upstream and/or downstream of any of the Sendai virus genes (N, P, M, F, HN, and L genes). In one embodiment, a Sendai virus vector plasmid can comprise an EIS sequence (transcription termination (E) sequence-intervening (I) sequence-transcription initiating (S) sequence) upstream or downstream of a gene of interest, whereby expression of a gene upstream or downstream of the gene of interest can be promoted. In one embodiment, a Sendai virus vector plasmid can be modified to insert a sequence having a base number that is a multiple of 6 (e.g., a sequence comprising a gene of interest). In one embodiment, a Sendai virus vector plasmid is configured to function in a producer cell wherein the vector plasmid and the producer cell comprise a nucleic acid sequence required for constituting a Sendai virus vector. In one embodiment, one or more (e.g., all) of genes not contained in a vector plasmid among nucleic acid sequences required for constituting a Sendai virus vector or a gene product thereof can be supplied in a producer cell in trans with respect to the vector plasmid.

In one embodiment, a nucleic acid sequence required for constituting a Sendai virus vector can comprise: a nucleic acid sequence encoding a capsid protein (N and HN); a nucleic acid sequence encoding a protein which packages, transcribes, and replicates a genome (P and L); two terminal sequences (LE and TR); and a nucleic acid sequence of a helper gene. In one embodiment, a nucleic acid sequence of a helper gene can be F, V, C, and M. In one embodiment, a nucleic acid sequence required for constituting a Sendai virus vector can be all genes of a Sendai virus. In one embodiment, a Sendai virus vector plasmid comprises a nucleic acid sequence required for constituting a Sendai virus between LE and TR. In one embodiment, a nucleic acid sequence required for constituting a Sendai virus vector can comprise N, P, V, C, M, F, HN, or L gene. In one embodiment, a nucleic acid sequence required for constituting a Sendai virus vector can be all genes of a Sendai virus other than one or more of M, F, and HN. When a Sendai virus vector plasmid which is deficient in one or more of M, F, and HN genes is used, a loaded nucleic acid can lose the transmitting ability in a host. In one embodiment, a Sendai virus vector plasmid does not need to comprise one or more of M, F, and HN, while in one embodiment, a nucleic acid sequence required for constituting a Sendai virus vector comprises the same. In one embodiment, a Sendai virus vector plasmid may comprise a gene encoding an RNA polymerase of bacteriophage T7. In one embodiment, a Sendai virus vector plasmid may comprise a self-cleaving ribozyme Rbz.

In one embodiment, a Sendai virus gene may be modified in order to decrease the antigenicity of a Sendai virus protein or to increase the transcription efficiency and replication efficiency of RNA. In one embodiment, one or more of N gene, P gene, and L gene, which are replication factors, may be modified in order to enhance the function of transcription or replication. In one embodiment, HN protein, which is a structural protein, may be modified, whereby hemagglutinin activity and/or neuraminidase activity can change, wherein weakening the hemagglutinin activity can improve the stability of a virus in blood while changing the neuraminidase activity can change the infectivity of a virus. In one embodiment, F protein, which is involved in membrane fusion, may be modified. In one embodiment, modification may be performed such that V gene, which is an accessory gene, is lacked. Examples of a producer cell for producing a Sendai virus vector include cells such as BHK/T7.

In one embodiment, the present disclosure provides a composition comprising a virus vector comprising a population of the virus vectors of the present disclosure. In one embodiment, in a composition comprising a virus vector, virus vector particles not comprising a nucleic acid in all virus vector particles can be about 90% or less, about 80% or less, about 75% or less, about 70% or less, about 65% or less, about 60% or less, about 55% or less, or about 50% or less. In one embodiment, in the composition comprising a virus vector of the present disclosure, virus vector particles comprising a nucleic acid comprising all genes of interest in all virus vector particles can be about 75% or greater, about 80% or greater, about 85% or greater, about 90% or greater, about 92% or greater, about 95% or greater, about 97% or greater, or about 99% or greater. In one embodiment, in the composition comprising a virus vector of the present disclosure, virus vector particles comprising a nucleic acid derived from the plasmid of the present disclosure other than a desired nucleic acid in all virus vector particles can be about 10% or less, about 7% or less, about 5% or less, about 4% or less, about 3% or less, about 2% or less, about 1.5% or less, about 1% or less, about 0.7% or less, about 0.5% or less, about 0.4% or less, about 0.3% or less, about 0.2% or less, or about 0.1% or less.

### (Creation of a virus vector plasmid)

In one embodiment, the present disclosure provides a method for creating the vector plasmid of the present disclosure, the method comprising operably linking a plurality of nucleic acid sequences. In one embodiment, the present disclosure provides a method for creating the vector plasmid of the present disclosure, the method comprising replicating the vector plasmid of the present disclosure in hay bacillus. In one embodiment, the present disclosure provides a method for creating the vector plasmid of the present disclosure, the method comprising introducing a nucleic acid comprising a nucleic acid sequence for producing a virus vector having a sequence which is replicated in hay bacillus into a host cell to form a plasmid in the host cell. In one embodiment herein, since hay bacillus can have an ability to form a plasmid from a nucleic acid that was taken in from outside, a nucleic acid to be introduced does not need to be a plasmid in this method. For example, when hay bacillus comes into contact with a nucleic acid (e.g., acyclic nucleic acid having a tandem repeat nucleic acid sequence), hay bacillus takes in the nucleic acid and can form a plasmid in hay bacillus (for example, see OGAB method described herein).

In one embodiment, the method for introducing a nucleic acid into hay bacillus can be any method, examples thereof include use of competent hay bacillus, electroporation method, method using a particle gun (gene gun), calcium phosphate method, and the like. "Competent" refers to a state in which a cell has become more permeable to an exogenous substance (e.g., nucleic acid). Although any known method can be used in order to make hay bacillus competent, for example, the method described in Anagnostopoulou, C. and Spizizen, J. J. Bacteriol., 81, 741-746(1961) can be used. In one embodiment, the vector plasmid of the present disclosure is created in hay bacillus, and may be directly amplified in the same hay bacillus.

When a nucleic acid is introduced into hay bacillus to create a virus vector plasmid having a different structure from that of the nucleic acid, the nucleic acid to be introduced can comprise each element of a virus vector plasmid described in detail below.

### (OGAB method)

In one embodiment, the vector plasmid of the present disclosure can be created by OGAB method. OGAB (Ordered Gene Assembly in *Bacillus subtilis*) method is a method of causing a transformation organism to take in an assembled nucleic acid to generate a cyclic plasmid in the organism. OGAB method does not necessarily need to use hay bacillus (*Bacillus subtilis*). Any organism which is capable of taking in an acyclic long strand nucleic acid and generating a plasmid can be used. Such an organism is herein referred to as "transformation organism". OGAB method can easily prepare a plasmid comprising a nucleic acid with a large size. A nucleic acid to be taken in by a transformation organism is herein referred to as "assembled nucleic acid". Typically, an assembled nucleic acid has a tandem repeat-like structure in which one unit of plasmid and one set of unit nucleic acids repeatedly appear in the same direction. Generation of a plasmid in a transformation organism can be promoted by using an assembled nucleic acid having such a structure. As used herein, a "unit nucleic acid" refers to a nucleic acid molecule or a portion of a nucleic acid molecule having a partial sequence constituting the sequence of an assembled nucleic acid. As described in detail below, a plurality of types of unit nucleic acids are prepared as a unit vector and an assembled nucleic acid is then constructed.

The procedure for creating an assembled nucleic acid to be taken in by a transformation organism is described below.

### * Preparation of a unit nucleic acid

Unit nucleic acids to be incorporated into an assembled nucleic acid are prepared. Unit nucleic acids can be created by any known method. For example, unit nucleic acids can be created by polymerase chain reaction (PCR) or chemical synthesis. Unit nucleic acids can have any desired sequence such as a sequence encoding a desired protein (such as therapeutic proteins or proteins constituting a virus vector) or a portion thereof, a sequence controlling a gene (such as promoters or enhancers), or a sequence for manipulating a nucleic acid (such as restriction enzyme recognition sequences). The terminals of each unit nucleic acid can be configured to provide a specific overhang sequence so that a plurality of types of unit nucleic acids are arranged in a specific order and/or specific direction when the unit nucleic acids are incorporated into an assembled nucleic acid.

Since a large number of unit nucleic acids can be finally assembled on a plasmid, one or more unit nucleic acids may be designed to encode one or more genes with a great base length. Examples of a gene with a great base length can include a group of genes constituting a series of metabolic pathway.

### * Preparation of a unit vector

A unit vector can be prepared by linking a unit nucleic acid with an additional nucleic acid different from the unit nucleic acid. Use of a unit vector can enable a unit nucleic acid to be more easily handled.

An additional nucleic acid may be a linear nucleic acid or may be a cyclic plasmid. When a cyclic plasmid is used as an additional nucleic acid, a unit vector can also have a cyclic structure. Thus, the unit vector can be used, for example, for transformation of *Escherichia coli* or the like. In one embodiment, an additional nucleic acid can comprise a replication origin so that a unit vector is replicated in a host into which the unit vector has been introduced. In one embodiment, all unit nucleic acids for constructing a certain assembled nucleic acid may be linked to the same type of additional nucleic acids, whereby the size difference between unit vectors can be reduced and a plurality of types of unit vectors can be more easily handled. In one embodiment, unit nucleic acids for constructing a certain assembled nucleic acid may be linked to different types of additional nucleic acids. In one embodiment, for one or more of unit nucleic acids for constituting a certain assembled nucleic acid, the proportion of (base length of a unit nucleic acid)/(base length of a unit vector) or an average thereof can be 50% or less, 40% or less, 30% or less, 20% or less, 15% or less, 10% or less, 7% or less, 5% or less, 2% or less, 1.5% or less, 1% or less, or 0.5% or less. The more the size of an additional nucleic acid is greater than a unit nucleic acid, the more uniformly different types of unit vectors can be handled. Linking between a unit nucleic acid and an additional nucleic acid can be performed by any method such as, for example, ligation using a DNA ligase or TA cloning method. In one embodiment, for one or more of unit nucleic acids for constituting a certain assembled nucleic acid, the proportion of (base length of a unit nucleic acid)/(base length of a unit vector) or an average thereof can be 1% or greater, 0.3% or greater, 0.1% or greater, 0.03% or greater, 0.01% or greater, 0.003% or greater, or 0.001% or greater, wherein unit vectors can be more easily manipulated.

In one embodiment, the length of a unit nucleic acid can be 10 bp or greater, 20 bp or greater, 50 bp or greater, 70 bp or greater, 100 bp or greater, 200 bp or greater, 500 bp or greater, 700 bp or greater, 1000 bp or greater, or 1500 bp or greater, and 5000 bp or less, 5000 bp or less, 2000 bp or less, 1500 bp or less, 1200 bp or less, 1000 bp or less, 700 bp or less, or 500 bp or less.

In one embodiment, an assembled nucleic acid can be constructed from 2 or more types, 4 or more types, 6 or more types, 8 or more types, 10 or more types, 15 or more types, 20 or more types, 30 or more types, 40 or more types, 50 or more types, 60 or more types, 70 or more types, 80 or more types, 90 or more types, or 100 or more types, and 1000 or less types, 700 or less types, 500 or less types, 200 or less types, 120 or less types, 100 or less types, 80 or less types, 70 or less types, 60 or less types, 70 or less types, or 50 or less types of unit nucleic acids. A desired assembled nucleic acid having a tandem repeat-like structure can be efficiently created by adjusting the mole number of each unit nucleic acid (or unit vector) to be nearly the same.

In one embodiment, a unit nucleic acid may have a base length resulting from nearly equally dividing one set of repeat sequences in an assembled nucleic acid by the number of unit nucleic acids. Doing so can facilitate the manipulation of making the mole number of each unit nucleic acid (or unit vector) even. In one embodiment, a unit nucleic acid can have a base length that is increased or decreased from a base length resulting from nearly equally dividing one set of repeat sequences in an assembled nucleic acid by the number of unit nucleic acids by 30% or less, 25% or less, 20% or less, 15% or less, 10% or less, 7% or less, or 5% or less.

In one embodiment, a unit nucleic acid can be designed to have a non-palindromic sequence (a sequence which is not a palindromic sequence) at an end of the unit nucleic acid. A unit nucleic acid designed in this manner can easily give a structure in which unit nucleic acids are linked to each other while keeping the order in an assembled nucleic acid when the non-palindromic sequence is configured as an overhang sequence.

### * Creation of an assembled nucleic acid

An assembled nucleic acid can be constructed by linking unit nucleic acids to each other. In one embodiment, a unit nucleic acid can be prepared by being cut out from a unit vector by a restriction enzyme or the like. An assembled nucleic acid can comprise 1 or more, 2 or more, 3 or more, 4 or more, 5 or more, 6 or more, 7 or more, 8 or more, 9 or more, or 10 or more sets of repeat sequences. A repeat sequence in an assembled nucleic acid can comprise a sequence of a unit nucleic acid and, optionally, a sequence of a vector nucleic acid for assembling. An assembled nucleic acid can have a sequence that enables replication of a nucleic acid in a transformation organism. In one embodiment, a sequence that enables replication of a nucleic acid in a transformation organism can comprise a replication origin that is effective in the transformation organism (e.g., bacteria that belong to the genus *Bacillus* (hay bacillus)). Although a sequence of a replication origin that is effective in hay bacillus is not particularly limited, examples of a sequence having a θ-type replication mechanism include a sequence of a replication origin or the like comprised in a plasmid such as pTB19 (Imanaka, T., et al. J.Gen.Microbioi. 130, 1399-1408.(1984)), pLS32 (Tanaka, T and Ogra, M. FEBS Lett. 422, 243-246.(1998)), or pAMβ1 (Swinfield, T.J., et al. Gene 87, 79-90.(1990)).

An assembled nucleic acid may optionally comprise an additional base sequence in addition to a unit nucleic acid. In one embodiment, an assembled nucleic acid may comprise a base sequence which controls transcription and translation such as promoters, operators, activators, or terminators. Specific examples of a promoter of when hay bacillus is a host include Pspac (Yansura, D. and Henner, D.J. Pro. Natl. Acad. Sci, USA 81, 439-443.(1984.)) of which expression can be controlled by IPTG (isopropyl s-D-thiogalactopyranoside), or Pr promoter (Itaya, M. Biosci. Biotechnol. Biochem. 63, 602-604.(1999)) and the like.

Unit nucleic acids can form a repeat structure in an assembled nucleic acid in which they maintain a certain order and direction. In one embodiment, unit nucleic acids cut out from a unit vector are constructed such that the base sequences of the overhang ends of the unit nucleic acids are complementary to each other, whereby a repeat structure in which the unit nucleic acids maintain a certain order and direction can be formed in an assembled nucleic acid. In one embodiment, a repeat structure in which unit nucleic acids maintain a certain order and direction can be efficiently formed by making the structure of the overhang ends unique for each different unit nucleic acid. In one embodiment, an overhang end may have a non-palindromic sequence, and may be either 5' terminal overhang or 3' terminal overhang.

In one embodiment, a unit nucleic acid having an overhang end can be cut out from a unit vector using a restriction enzyme. In this embodiment, the unit vector can have one or more restriction enzyme recognition sequences. When the unit vector has a plurality of restriction enzyme recognition sequences, each of the restriction enzyme recognition sequences may be recognized by the same restriction enzyme, or may be recognized by different restriction enzymes. In one embodiment, a unit vector can comprise a pair of regions recognized by the same restriction enzyme such that a full unit nucleic acid region is comprised between these regions. Although the restriction enzyme that is used is not particularly limited, a type II restriction enzyme (e.g., type IIS restriction enzyme such as AarI, BbsI, BbvI, BcoDI, BfuAI, BsaI, BsaXI, BsmAI, BsmBI, BsmFI, BspMI, BspQI, BtgZI, FokI, or SfaNI) can be used. These restriction enzymes can be capable of creating an overhang end at a site which is outside the recognition sequence and away from the recognition sequence by a certain distance. When (for example, a single) type IIS restriction enzyme is used, the overhang end of each unit nucleic acid that was cut out can have different sequences such that said restriction enzyme can be advantageous in assembling a plurality of unit nucleic acids in a certain order and direction. In one embodiment using a type IIS restriction enzyme, a unit vector does not comprise a region recognized by the type IIS restriction enzyme in a unit nucleic acid region. In an embodiment using a restriction enzyme which cleaves a recognition region, a unit vector can comprise a region recognized by the restriction enzyme at a terminal of a unit nucleic acid region.

In one embodiment, when the same type of restriction enzyme is used to cut out unit nucleic acids from a plurality of unit vectors, restriction enzyme processing can be performed in a solution comprising the plurality of unit vectors, such that the efficiency of the operation can be improved. The number of types of restriction enzymes that are used to create a certain assembled nucleic acid can be, for example, 5 or less, 4 or less, 3 or less, 2 or less types, or 1. A variation in the mole number between unit nucleic acids can be reduced by using a less number of types of restriction enzymes. In one embodiment, a unit nucleic acid that was cut out from a unit vector can be easily purified by any known fractionating method such as agarose gel electrophoresis.

Unit nucleic acids and, optionally, vector nucleic acids for assembling can be linked (ligated) to each other by using a DNA ligase or the like, whereby an assembled nucleic acid can be created. For example, linking of unit nucleic acids and, optionally, vector nucleic acids for assembling can be performed in the presence of a component such as polyethylene glycol (e.g., PEG2000, PEG4000, PEG6000, PEG8000 or the like) and a salt (e.g., monovalent alkali metal, sodium chloride or the like). The concentration of each unit nucleic acid in a ligation reaction solution is not particularly limited and can be 1 fmol/µl or greater or the like. The reaction temperature and the time of ligation are not particularly limited and can be at 37°C for 30 minutes or longer, or the like. In one embodiment, before unit nucleic acids and, optionally, vector nucleic acids for assembling are linked, a composition comprising the unit nucleic acids and, optionally, vector nucleic acids for assembling may be subjected to any condition where a restriction enzyme is deactivated (e.g., phenol and chloroform processing).

Unit nucleic acids can be adjusted to have nearly the same mole number by using the method described in WO 2015/111248 or the like. A desired assembled nucleic acid having a tandem repeat-like structure can be efficiently created by adjusting unit nucleic acids to have nearly the same mole number. The mole number of unit nucleic acids can be adjusted by measuring the concentration of the unit vectors or unit nucleic acids.

### * Creation of a plasmid from an assembled nucleic acid

A plasmid can be formed in a transformation organism by bringing an assembled nucleic acid into contact with the transformation organism. In one embodiment, examples of a transformation organism include bacteria that belong to the genus *Bacillus,* bacteria that belong to the genus *Streptococcus,* bacteria that belong to the genus *Haemophilus,* the genus *Neisseria,* and the like. Bacteria that belong to the genus *Bacillus* include *B.subtilis, B.megaterium, B.stearothermophilus,* and the like. In a preferred embodiment, a transformation organism is hay bacillus. In one embodiment, a transformation organism for taking in an assembled nucleic acid is competent and can actively take in a nucleic acid. For example, competent hay bacillus cleaves a double-stranded nucleic acid as a substrate on a cell, degrades either one single strand of the two strands from this cleavage point, and takes in the other single strand into the microbial body. The single strand that was taken in can be repaired into a cyclic double-stranded nucleic acid in the microbial body. Any known method can be used to make a transformation organism competent. For example, hay bacillus can be made competent by using the method described in Anagnostopoulou, C. and Spizizen, J. J. Bacteriol., 81, 741-746(1961). A known method suitable for each transformation organism can be used as a method for transformation.

In one embodiment, a vector plasmid produced from a packaging cell can be purified using any known method, and the present disclosure also provides a vector plasmid purified in this manner. In one embodiment, it can be confirmed that a purified vector plasmid has a desired nucleic acid sequence by studying a size pattern of a fragment generated by restriction enzyme cleavage, or by PCR method, sequencing method, or the like. In one embodiment, a composition comprising a vector plasmid prepared by the vector plasmid creation method of the present disclosure may contain a small amount of endotoxin. In one embodiment, hay bacillus comprising the vector plasmid of the present disclosure is provided.

### (Composition)

In one embodiment, the present disclosure provides a composition comprising a vector plasmid or a virus vector described herein.

### (Dosage form or the like)

A composition described herein can be provided in various forms. The form of a composition may be, for example, injection, capsule, tablet, granule, inhalant or the like. An aqueous solution for injection may be preserved in, for example, a vial or a stainless container. Further, for example, saline, sugar (e.g., trehalose), NaCl, or NaOH or the like may be added to an aqueous solution for injection.

In one embodiment, the composition of the present disclosure comprises a pharmaceutically acceptable carrier or excipient. Such a carrier can be an aseptic liquid such as water or oil, including but not limited to liquids derived from petroleum, animal, plant or synthesis, as well as peanut oil, soybean oil, mineral oil, sesame oil and the like. When a medicament is orally administered, water is a preferred carrier. For intravenous administration of a pharmaceutical composition, saline and aqueous dextrose are preferred carriers. Preferably, aqueous saline solution and aqueous dextrose and glycerol solution are used as a liquid carrier of an injectable solution. Suitable excipients include light anhydrous silicic acid, crystalline cellulose, mannitol, starch, glucose, lactose, sucrose, gelatin, malt, rice, wheat flour, chalk, silica gel, sodium stearate, glyceryl monostearate, talc, sodium chloride, powdered skim milk, glycerol, propylene, glycol, water, ethanol, carmellose calcium, carmellose sodium, hydroxypropyl cellulose, hydroxypropyl methylcellulose, polyvinyl acetal diethylamino acetate, polyvinylpyrrolidone, gelatin, medium-chain fatty acid triglyceride, polyoxyethylene hydrogenated castor oil 60, poloxamer, saccharose, carboxymethylcellulose, corn starch, inorganic salt and the like. When desired, the composition can also contain a small amount of wetting agent or emulsifier or pH buffer. These compositions can be in a form of solution, suspension, emulsion, tablet, pill, capsule, powder, sustained release mixture or the like. It is also possible to use traditional binding agents and carriers, such as tryglyceride, to prepare a composition as a suppository. An oral preparation can also comprise a standard carrier such as medicine grade mannitol, lactose, starch, magnesium stearate, sodium saccharin, cellulose, or magnesium carbonate. Examples of a suitable carrier are described in E.W.Martin, Remington's Pharmaceutical Sciences (Mark Publishing Company, Easton, U.S.A). In addition, the composition may comprise, for example, a surfactant, an excipient, a coloring agent, a flavoring agent, a preservative, a stabilizer, a buffer, a suspension, an isotonizing agent, a binding agent, a disintegrant, a lubricant, a fluidity improving agent, a corrigent or the like. In one embodiment, the pH of any liquid composition of the present disclosure can be about 3, about 3.5, about 4, about 4.5, about 5, about 5.5, about 6, about 6.5, about 7, about 7.5, about 8, about 8.5, about 9, about 9.5, about 10, about 10.5, about 11, or within the range between any two values described above.

Any component of the composition of the present disclosure can be provided as a pharmaceutically acceptable salt, which can be, for example, salts formed with a free carboxyl group, derived from hydrochloric acid, phosphoric acid, acetic acid, oxalic acid, tartaric acid or the like, salts formed with a free amine group, derived from isopropylamine, trimethylamine, 2-ethylaminoethanol, histidine, procaine or the like, and salts formed with sodium, potassium, ammonium, calcium, and ferric hydroxide.

In a preferred embodiment, a composition can be prepared as a pharmaceutical composition adapted to administration to humans in accordance with a known method. Such a composition can be administered by an injection. A composition for injection administration is typically a solution in an aseptic isotonic aqueous buffer. A composition can also comprise a local anesthetic such as lidocaine which alleviates the pain at the site of injection and a solubilizing agent as needed. Generally, ingredients can be supplied separately or by mixing the ingredients together in a unit dosing form and supplied, for example, in a sealed container such as an ampoule or sachet showing the amount of active agent or as a lyophilized powder or water-free concentrate. When a composition is to be administered by infusion, the composition can be distributed by using an infusion bottle containing aseptic agent-grade water or saline. When a composition is to be administered by injection, an aseptic water or saline ampoule for injection can also be provided such that the ingredients can be mixed prior to administration.

### (Use/application)

A vector plasmid or a virus vector described herein or a composition comprising the same can be used in various applications such as gene therapy, functional genomics, cancer vaccination, and/or anti-virus vaccination.

When the virus vector or the composition comprising a virus vector of the present disclosure is applied to a subject, the subject is not particularly limited and can be a mammal (e.g., mouse, rat, hamster, rabbit, cat, dog, cow, sheep, pig, monkey, human or the like), a bird, a reptile, an amphibian, an arthropod, a fish or the like.

The amount of the virus vector or the composition comprising a virus vector of the present disclosure may vary depending on the property of the disorder or condition which is treated or prevented. However, such an amount can be determined by those skilled in the art by a standard clinical technique based on the descriptions herein. An in vitro assay can be used in some cases to assist the identification of the optimal dosing range. The precise dose to be used in a preparation may also vary depending on the administration pathway and the severity of the disease or disorder. Thus, the dose should be determined in accordance with the judgment of the attending physician or the condition of each patient. The dosage of the virus vector or the composition comprising a virus vector of the present disclosure is not particularly limited, but may be, for example, 1 × 10⁵, 1 × 10⁶, 1 × 10⁷, 1 × 10⁸, 1 × 10⁹, 1 × 10¹⁰, 1 × 10¹¹, 1 × 10¹², 1 × 10¹³, 1 × 10¹⁴, or 1 × 10¹⁵ virus vectors per dosing, or may be within the range between any two values described above. The dosing interval is not particularly limited, but may be, for example, 1 or 2 administration every 1, 7, 14, 21, or 28 days or 1 or 2 administrations in the range of period between any two values described above. The dosage, dosing interval, and dosing method may be appropriately selected depending on the age, weight, symptom, target organ or the like of the patient.

The administration pathway of a virus vector or a composition comprising a virus vector described herein may be, for example, intravenous, intracutaneous, subcutaneous, intramuscular, intraperitoneal, intrathecal, intracerebroventricular, intraparenchymal, pulmonary, intranasal, epidural, oral administration or the like. In one embodiment, the composition of the present disclosure and various delivery systems can be used together. Such a system, for example, can use encapsulation in liposomes, microparticles and microcapsules, endocytosis mediated by a receptor, or the like. A medicament can be administered by a suitable pathway, such as by infusion, bolus injection, or by absorption through epithelial or mucocutaneous lining (e.g., oral cavity, rectum, intestinal mucosa or the like). In addition, an inhaler or mistifier using an aerosolizing agent can be used as needed. Moreover, other agents can also be administered together. Administration can be systemic or local.

The composition of the present disclosure can be provided as a kit. In one embodiment, the present disclosure provides a kit for use in creating a vector plasmid, the kit comprising at least one nucleic acid comprising: a nucleic acid sequence which promotes plasmid replication in hay bacillus; and a nucleic acid sequence required for constituting a virus. In one embodiment, the present disclosure provides an agent pack or kit comprising one or more containers filled with one or more ingredients that can be added to the composition of the present disclosure. Optionally, information indicating approval for manufacture, use or sale for administration to a human by a government agency regulating the manufacture, use or sale of medicaments or biological products in a stipulated form can be appended to such a container.

The formulation procedure for the composition of the present disclosure as a medicament or the like is known in the art. The procedure is described, for example, in the Japanese Pharmacopoeia, the United States Pharmacopeia, pharmacopeia of other countries, or the like. Thus, those skilled in the art can determine the embodiment, such as the amount to be used, without undue experimentation from the descriptions herein.

As used herein, "or" is used when "at least one or more" of the matters listed in the sentence can be employed. When explicitly described herein as "within the range" of "two values", the range also includes the two values themselves.

Reference literatures such as scientific literatures, patents, and patent applications cited herein are incorporated herein by reference to the same extent that the entirety of each document is specifically described.

As described above, the present disclosure has been described while showing preferred embodiments to facilitate understanding. The present disclosure is described below based on Examples. The aforementioned description and the following Examples are not provided to limit the present disclosure, but for the sole purpose of exemplification. Thus, the scope of the present disclosure is not limited to the embodiments and Examples specifically described herein and is limited only by the scope of claims.

### [EXAMPLES]

For reagents, the specific products described in the Examples were used. However, the reagents can be substituted with an equivalent product from another manufacturer (Sigma-Aldrich, Wako Pure Chemical, Nacalai Tesque, R & D Systems, USCN Life Science INC, or the like).

### (Example 1: Amplification of an AAV vector plasmid)

An AAV vector plasmid was created and amplified in *Escherichia coli* and hay bacillus according to the following procedure.

### Materials

Plasmids of pAAV-CMV vector, pRC2-mi342 vector, and pHelper vector (AAVpro^{®} Helper Free System) were purchased from Takara Bio (Shiga prefecture). pGETS103ΔAarI, which is a hay bacillus-Escherichia *coli* shuttle plasmid vector, is a plasmid in which AarI recognition site has been eliminated by introducing a point mutation (5'-CACCAGC-3') into the sole restriction enzyme AarI recognition site (5'-CACCTGC-3') in plasmid pGETS103 (Tsuge, K., Itaya, M. (2001) Recombinational transfer of 100-kilobase genomic DNA to plasmid in Bacillus subtilis 168, Journal of Bacteriology, 183, 5453-5458.), which was transferred from Kobe University. Chemical competent cells of *Escherichia coli* JM109 strain were purchased from Takara Bio. Hay bacillus BUSY9797 strain (Tsuge, K., Sato, Y., Kobayashi, Y., Gondo, M., Hasebe, M., Togashi, T., Tomita, M., Itaya, M. (2015) Method of preparing an equimolar DNA mixture for one-step DNA assembly of over 50 fragments, Scientific Reports, 5, 10655.) was transferred from Kobe University. pMD19-Tv-vector, which is a vector for TA cloning, and DNA Ligation Kit <Mighty Mix> were purchased from Takara Bio. Restriction enzyme AarI was purchased from Thermo Fisher Scientific (US). The rest of the restriction enzymes were all purchased from New England Biolab (US). TE buffer (10 mM Tris-HCl, 1 mM EDTA, pH 8.0) was purchased from Nacalai Tesque (Kyoto). Carbenicillin and tetracycline were purchased from Sigma-Aldrich (US). 2-hydroxyethyl agarose (Sigma-Aldrich) was used as a low melting point agarose gel. UltraPure Agarose of Invitrogen (US) was used for other common agarose gels for electrophoresis. TE saturated phenol was purchased from Nacalai Tesque. QIA quick miniprep kit and PCR purification Kit of Qiagen (Germany) were used as a plasmid column purification kit. Bactotryptone, Yeast extract, and Bacto Agar were purchased from Becton, Dickinson (US). The rest of the reagents for media were purchased from Nacalai Tesque. Egg white lysozyme and ethidium bromide were purchased from Sigma-Aldrich. Ribonuclease A was purchased from Nacalai Tesque. P1, P2, and P3 for use in plasmid purification were purchased from Qiagen. Cesium chloride was purchased from Nacalai Tesque.

### Medium

An LB medium that was used was prepared by dissolving 10 g of Bactotryptone, 5 g of Yeast extract, and 5 g of sodium chloride into 1 L of water, and further adding 15 g of Bacto Agar when forming an agar plate, followed by subjecting the mixture thereof to an autoclave (at 121°C for 20 minutes). Optionally, carbenicillin (final concentration of 100 µg/ml) or tetracycline (final concentration of 10 µg/ml) was added. A TF-1 medium and a TF-II medium for hay bacillus transformation were prepared as follows. First, 10 × Spizizen (140 g of K₂HPO₄ (anhydrous), 60 g of KH₂PO₄ (anhydrous), 20 g of (NH₄)₂SO₄, and 10 g of Na₃ citrate·2H₂O per 1 L), 50% glucose, 2% MgSO₄·7H₂O, 2% casamino acid, and water were each subjected to an autoclave and individually prepared. An aqueous solution (5 mg/ml) of each of the amino acids, tryptophan, arginine, leucine, and threonine, was prepared by filter-sterilization. For preparation of 500 ml of TF-I medium, 50 ml of 10 × Spizizen, 50% glucose, 2% MgSO₄·7H₂O, 2% casamino acid, and 5 ml of each of the amino acids, 5 mg/ml of tryptophan, arginine, leucine, and threonine, were used, 415 ml of sterile water was finally mixed thereto, the mixture thereof was filtered with a filter, and the product thereof was preserved at 4°C until it was used. For preparation of 500 ml of TF-II medium, the same amount for the TF-I medium was added except for adding 2.5 ml of 2% casamino acid, 0.5 ml of each amino acid solution (5 mg/ml), and 435.5 ml of sterile water, the mixture thereof was filtered with a filter, and the product thereof was preserved at 4°C until it was used.

### In vitro gene manipulation

Other common DNA manipulations were performed according to a standard protocol (Sambrook, J., et al., Molecular Cloning: A Laboratory Manual. Cold Spring Harbor Laboratory Press, Cold Spring Harbor, New York (1989)).

### Cutting out a DNA fragment from an electrophoresis gel and purification thereof

An amplified DNA fragment was applied with a voltage of 100 V (about 8 V/cm) by a general-purpose agarose gel electrophoresis device (i-MyRun.N, electrophoresis system for nucleic acids, Cosmo Bio (Tokyo)) and subjected to electrophoresis for 1 hour in the presence of 1 × TAE buffer (Tris-Acetate-EDTA Buffer, Nacalai Tesque) in 0.7% low melting point agarose gel (2-Hydroxyethyl Agarose TypeVII, Sigma-Aldrich), whereby a vector plasmid and a unit DNA were separated. This electrophoresis gel was stained with 100 ml of 1 × TAE buffer comprising 1 pg/ml of ethidium bromide for 30 minutes and illuminated with an ultraviolet ray with a long wavelength (366 mn) to be visualized, whereby a PCR product having a size of interest was cut out with a razor and collected in a 1.5 ml tube. 1 × TAE buffer was added to the collected low melting point agarose gel (about 300 mg) so that the total volume was about 700 µl, which was then incubated at 65°C for 10 minutes to thereby dissolve the gel. An equal quantity of TE saturated phenol was then added and mixed well to deactivate the restriction enzyme. The mixture was separated into a phenol phase and an aqueous phase by centrifugation (20,000 × g, 10 minutes), and the aqueous phase (about 900 µl) was collected in a new 1.5 ml tube. 500 µl of 1-butanol was added thereto and mixed well, followed by separation by centrifugation (20,000 × g, 1 minute) and removal of a water-saturated 1-butanol phase. This operation was repeated until the volume of the aqueous phase was 450 µl or less, thereby decreasing the volume of the aqueous phase. 50 µl of 3M potassium acetate-acetic acid buffer (pH 5.2) and 900 µl of ethanol were added thereto and centrifugation (20,000 × g, 10 minutes) was performed to precipitate the DNA, which was then rinsed with 70% ethanol and dissolved into 20 µl of TE buffer. This collected DNA was preserved at -20°C until it was used.

### Construction of a recombinant plasmid

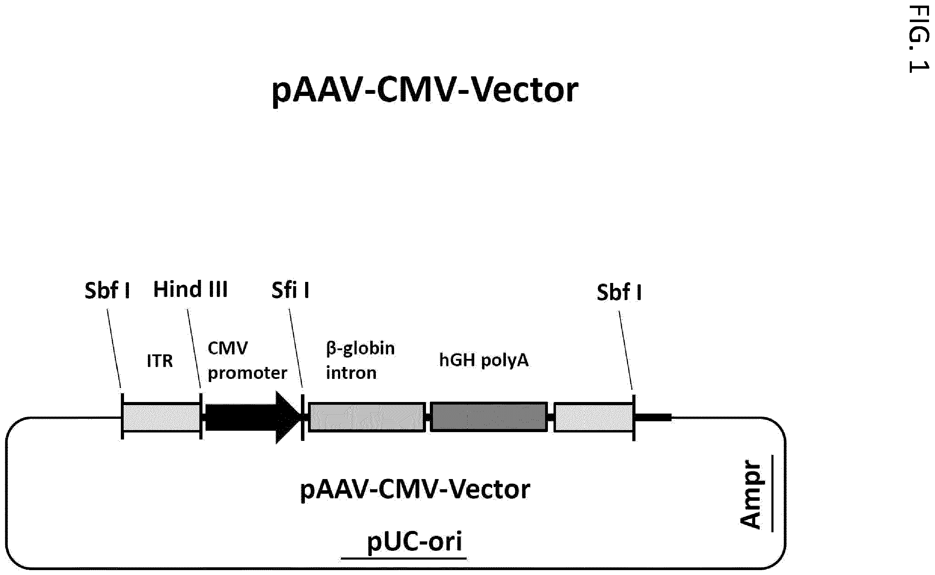

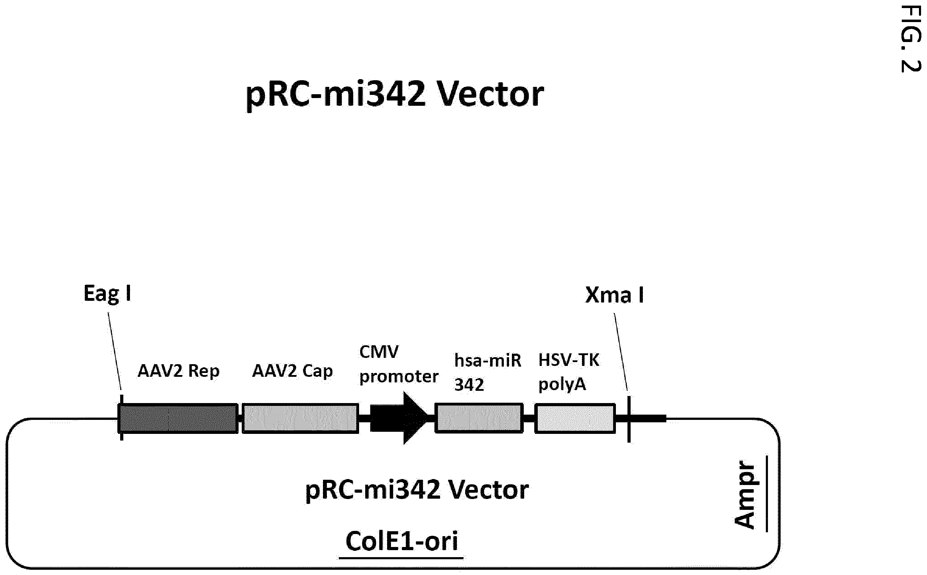

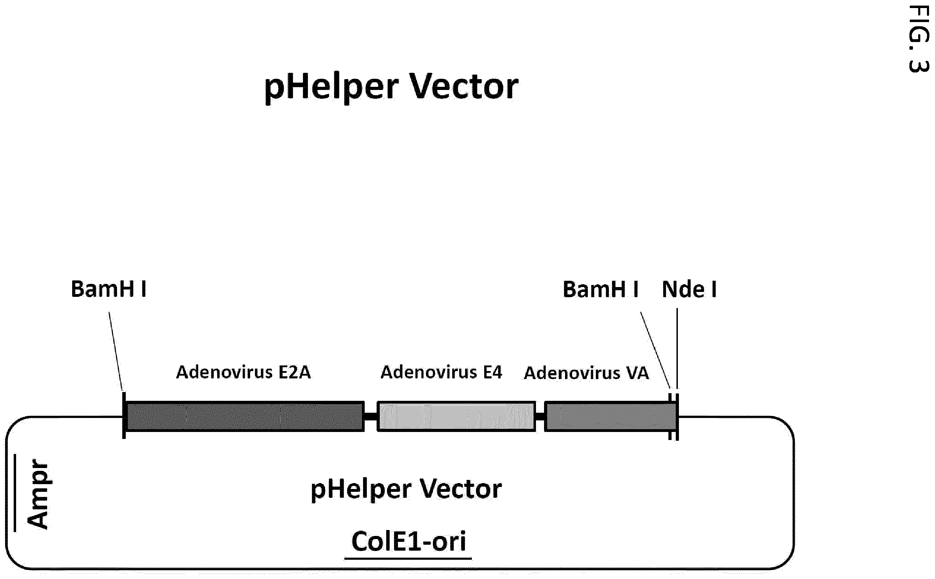

Necessary fragments obtained from pAAV-CMV plasmid (Figure **1**)**,** pRC2-mi342 plasmid (Figure **2**)**,** and pHelper plasmid (Figure **3**) by cutting out by a restriction enzyme or the like (1.8 kb SbfI fragment from pAAV-CMV, 5.2 kb EagI-XmaI fragment from pRC2-mi342, and 9.3 kb BamHI-BamHI-NdeI fragment from pHelper) were linked to a plasmid for assembling, thereby constructing a recombinant plasmid. Since a BamHI site is present in a VA region in pHelper, a 0.3 kb fragment from the BamHI site to a near NdeI site was obtained by PCR amplification. This fragment was amplified using a primer into which an SbfI site, an AarI site, and an EagI-XmaI site had been introduced so that the above-described three fragments could be introduced, thereby obtaining a PCR product having the sequence set forth in SEQ ID NO: 1.

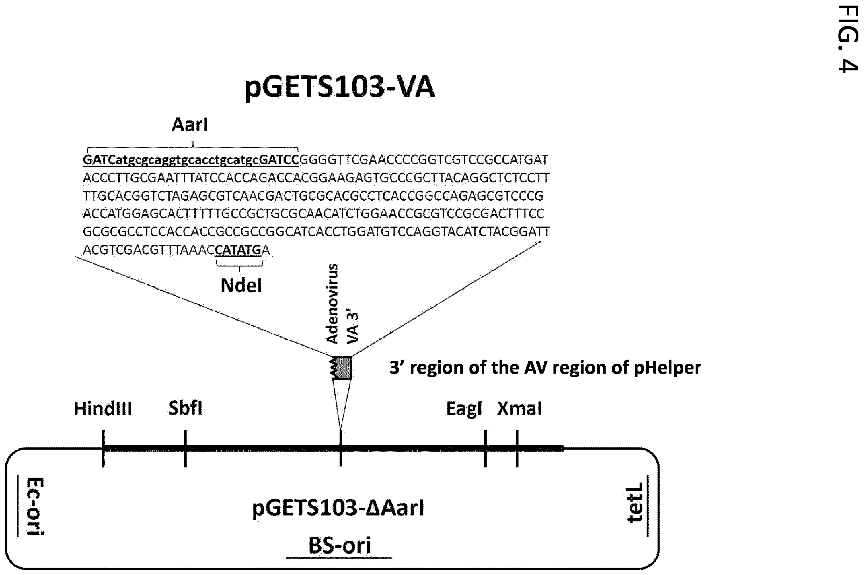

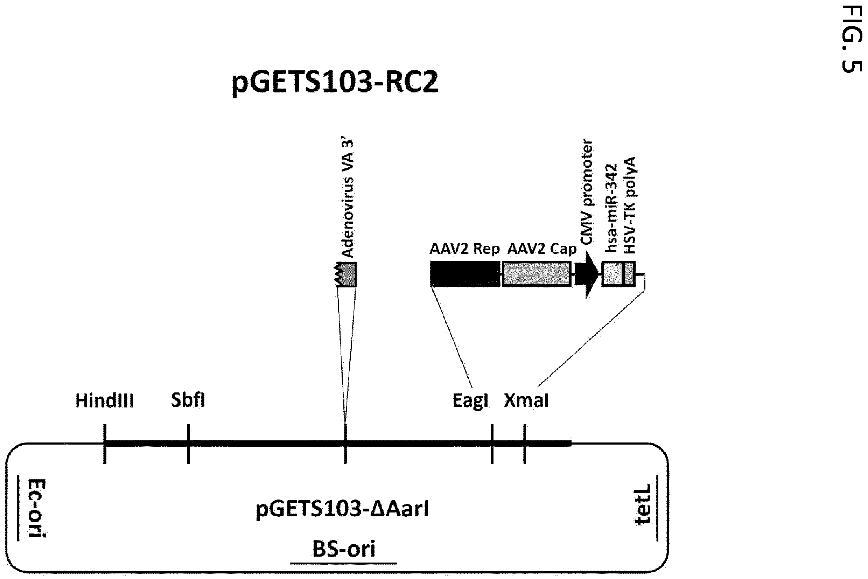

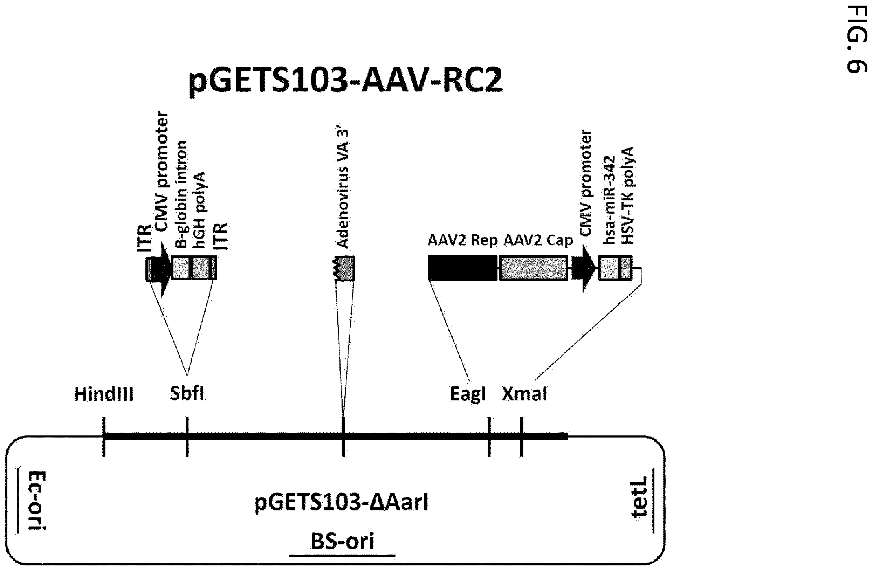

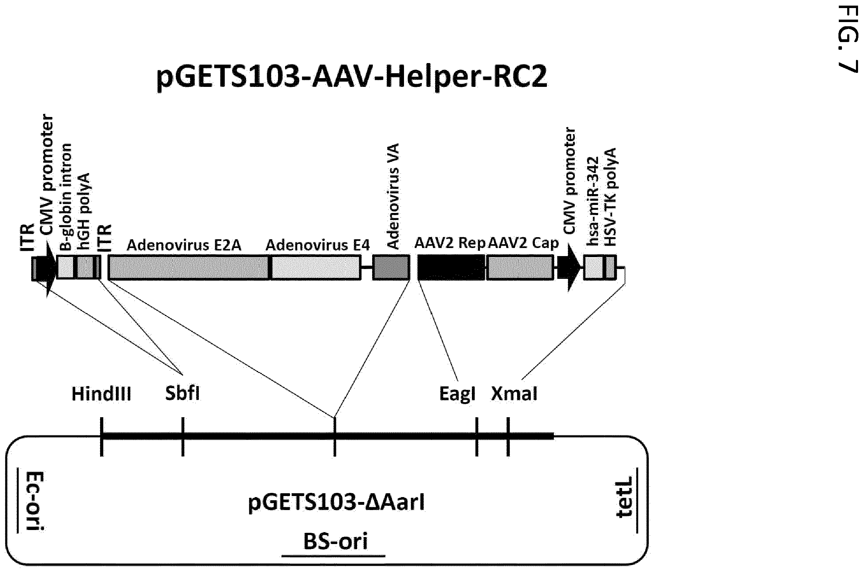

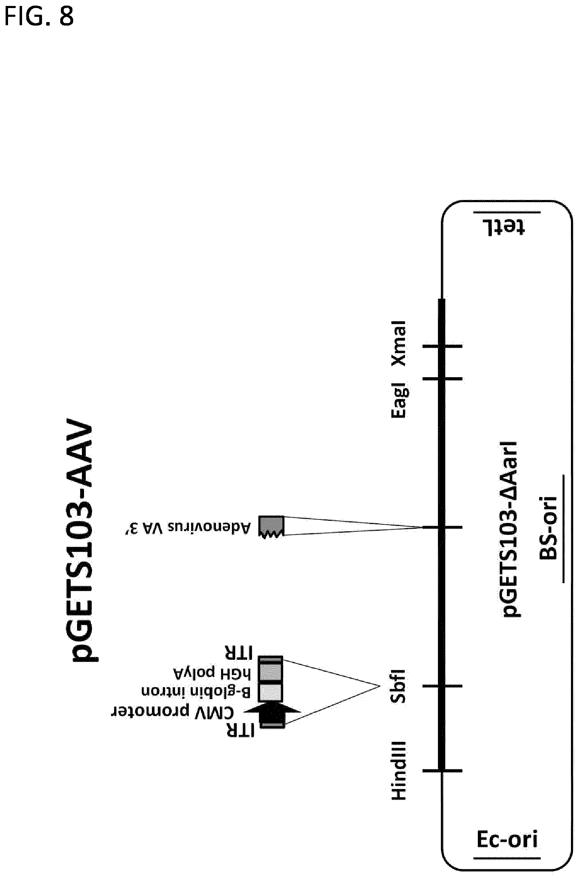

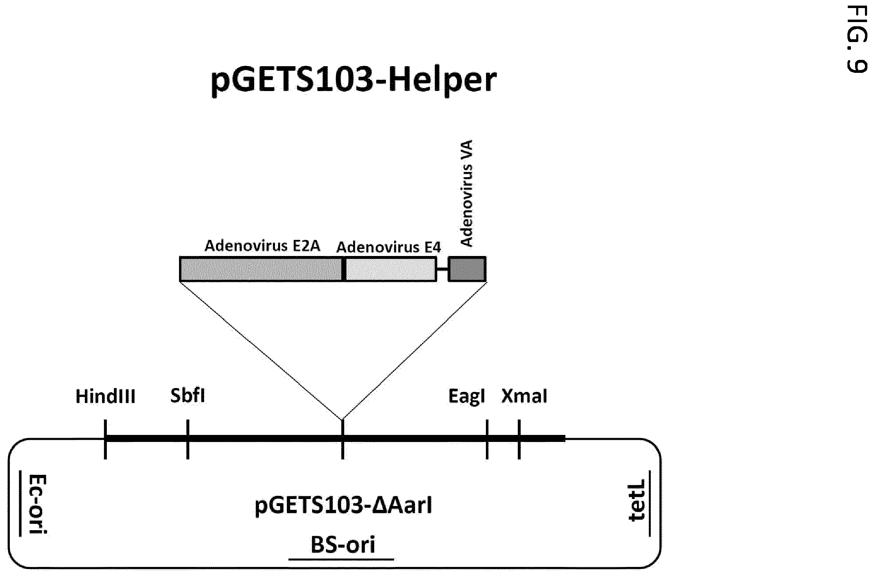

This PCR product was cloned into plasmid pMD19. After it was confirmed that the base sequence was correct, cleavage was performed at BsaI designed near a terminal of the above-described DNA to thereby obtain a fragment. This fragment was linked to a fragment which was obtained by cleaving pGETS103ΔAarI by HindIII and performing dephosphorylation processing. The obtained plasmid was named pGETS103-VA (Figure **4**)**.** After pGETS103-VA was cleaved by EagI and XmaI, a short fragment was removed by electrophoresis, plasmid pRC2-mi342 was cleaved by EagI and XmaI, large fragments of 5.2 kb were then cut out from the gel and purified, and a plasmid obtained by linking those fragments was named pGETS103-RC2 (Figure **5**). A 1.8 kb fragment obtained by cleaving plasmid pAAV-CMV by SbfI was linked to a fragment obtained by cleaving pGETS103-RC2 by SbfI and then performing phenol processing and ethanol precipitation for deactivation of the restriction enzyme and purification. The obtained plasmid was named pGETS103-AAV-RC2 (Figure **6**)**.** Said plasmid was further cleaved by AarI to remove a short DNA fragment, a 9.0 kb BamHI fragment of plasmid pHelper was linked thereto, and a plasmid introduced with the BamHI fragment in a direction in which the VA region regenerates in linkage with the pre-introduced BamHI-NdeI region was selected, thereby constructing pGETS103-AAV-Helper-RC2 with an all-in-one structure in which three plasmids were integrated into one plasmid (Figure **7****,** SEQ ID NO: 2). Furthermore, a plasmid independently having the fragment was constructed. Specifically, a plasmid obtained by linking a 1.8 kb fragment obtained by cleaving plasmid pAAV-CMV by SbfI to a fragment obtained by cleaving pGETS103-VA by SbfI was named pGETS103-AAV (Figure **8**)**.** Further, pGETS103-VA was cleaved by AarI to remove a short DNA fragment, a 9.0 kb BamHI fragment of plasmid pHelper was linked thereto, and a plasmid introduced with a BamHI fragment in a direction in which the VA region regenerates in linkage with the pre-introduced BamHI-NdeI region was selected, thereby constructing pGETS103-Helper (Figure **9**)**.**

### Escherichia coli transformation method

50 µl of dissolved *Escherichia coli* JM109 competent cells were transferred to a 1.5 ml centrifugation tube prepared on ice. Up to 5 µl of a recombinant plasmid was added thereto, and the mixture thereof was left standing on ice for 15 minutes and then incubated for 45 seconds in a 42°C warm bath. Subsequently, the product thereof was returned on ice, and after 2 minutes, 200 µl of SOC medium attached to the competent cells was added. The mixture thereof was placed in a rotary culture apparatus (RT-50 equipped with a culture holder for test tube SA-1811, Taitec (Osaka)) and cultured at 37°C for 1 hour at a rotation rate of 30 rpm. Subsequently, the product thereof was spread on an LB medium agar plate comprising carbenicillin and cultured overnight at 37°C.

### Hay bacillus transformation method

2 ml of LB medium was placed in a 14 ml test tube (2051) of Falcon^{®}, hay bacillus in a glycerol stock preserved at - 70°C was inoculated thereto, and the mixture thereof was cultured at 37°C for 17 hours while being rotated by a rotary culture apparatus. 900 µl of TF-I medium was dispensed into a new 14 ml test tube (Falcon 2051), 25 µl of 2% casamino acid was added thereto, 50 µl of the above-described culture broth was added thereto, and the mixture thereof was cultured at 37°C for 4 hours while being rotated by a rotary culture apparatus. Subsequently, 900 µl of TF-II medium was dispensed into a new 14 ml test tube, 100 µl of the TF-I culture broth was added thereto, and the mixture thereof was cultured at 37°C for 1.5 hours while being rotated by a rotary culture apparatus. 100 µl of the TF-II culture broth was placed in a 1.5 ml centrifugation tube, and 8 µl of a recombinant plasmid was added thereto. After the product thereof was cultured at 37°C for 30 minutes while being rotated by a rotary culture apparatus, 300 µl of LB medium was added thereto, and the mixture thereof was further cultured at 37°C for 1 hour while being rotated by a rotary culture apparatus. Subsequently, the culture was spread on an LB medium agar plate comprising 10 µg/ml of tetracycline and incubated overnight at 37°C to obtain a transformant.

### Preparation of a small amount of plasmid of Escherichia coli

Purification of a small amount for confirming the structure of a recombinant plasmid constructed in *Escherichia coli* was performed using Qiagen's QIAprep Spin Miniprep Kit and QIA cube, an automation apparatus, according to the manuals.

### Method for preparing a large amount of plasmid from hay bacillus and Escherichia coli

A plasmid DNA with high purity was supplied by a cesium chloride-ethidium bromide density-gradient ultracentrifugation method. Although the following method shows a purification method for 200 ml of LB medium, when the medium was over 200 ml, the following operation was repeated for every 200 ml. 200 ml of an LB medium supplemented with an antibiotic (tetracycline) was prepared, 100 ml of each thereof was placed in a 500 ml conical flask, an *Escherichia coli* or hay bacillus plasmid retaining strain was inoculated thereto, and cultured overnight at 37°C.

After culture was complete, 50 ml of each resulting product was dispensed into four 50 ml tubes (Falcon 2070) and centrifuged at 5,000 × g for 10 minutes. The supernatant was disposed of, and the bacterial pellet was completely loosened by vortexing. 10 mg/ml of lysozyme and 10 mg/ml of P1 solution supplemented with ribonuclease A were prepared, and 5 ml of each solution was added to the four tubes containing bacteria and mixed well, and incubated at room temperature for 5 minutes. 5 ml of each P2 was added to the four tubes, and the mixture thereof was slowly mixed and incubated at room temperature for 5 minutes. 5 ml of each P3 was further added and mixed with strong force to a certain extent so that the white turbid substance could be uniformly dispersed. Centrifugation was performed at 5,000 × g for 10 minutes, and the supernatant was aspirated with a pipette and transferred to four new 50 ml tubes (Falcon 2070) with a screw cap. 5 ml of TE saturated phenol was added to each tube and vigorously mixed. Centrifugation was performed at 5,000 × g for 10 minutes, and the supernatant was aspirated with a pipette and transferred to four new 50 ml tubes (Falcon 2070) with a screw cap. 20 ml of each 100% ethanol was added and mixed, followed by centrifugation at 5,000 × g for 10 minutes to remove the supernatant. 5.4 ml of TE was added to the precipitate and completely dissolved. Next, 6.40 g of cesium chloride was placed therein and completely dissolved. Furthermore, 2.6 ml of 1.1 g/ml of cesium chloride solution (solution made by mixing 1.1 g cesium chloride and 1 ml water without volumetric adjustment) was added. Finally, 600 µl of 10 mg/ml of ethidium bromide solution was added and mixed well. The above mixture was transferred to one ultracentrifugation tube (Beckman 362181). Water or 1.1 g/ml of cesium chloride solution (with a specific gravity of about 1.5 g/ml) was added to finely adjust the weight so that a difference in weight from the balance would be 20 mg or less. Centrifugation was performed with an ultracentrifugation instrument (Beckman Coulter) under the following condition. Centrifugation was performed for 15 hours or longer at a temperature of 18°C, a rate of 50,000 rpm, an acceleration of Max, and a deceleration of Max.

After centrifugation was complete, a 1 ml syringe set with a needle (21G × 5/8") was inserted into the ccc-form plasmid band to collect the plasmid solution and transfer it to a 15 ml tube under observation with an ultraviolet ray (365 nm). 500 µl of P3 was added thereto, followed by adding water so that the total volume was 3 ml. Furthermore, 9 ml of 100% ethanol was added. Centrifugation was performed at 5,000 × g for 10 minutes to remove the supernatant. 700 µl of TE was added to the obtained precipitate in the 15 ml tube and the DNA was dissolved. The resulting product was transferred to a 1.5 ml tube, 600 µl of 1-butanol was added thereto and mixed, the mixture thereof was centrifuged at 20,000 × g for about 10 seconds to separate the mixture into two layers, and the upper butanol layer was disposed of. 600 µl of 1-butanol was newly added and mixed, the mixture thereof was centrifuged at 20,000 × g for about 10 seconds to separate the mixture into two layers, and the upper butanol layer was disposed of. This operation was continued until the water layer was 200 µl or less. When the water layer was 200 µl or less, 1 ml of PB (Qiagen) was added and mixed well, and 600 µl of the PB mixture was applied to a spin column of QIAprep Spin Miniprep Kit and centrifuged at 20,000 × g for 1 minute. The flow-through was disposed of and the remaining 600 µl of the PB mixture was re-applied to the above-described column and centrifuged at 20,000 × g for 1 minute. The flow-through was disposed of, the column was placed in the collection tube again, and 700 µl of PE was applied and centrifuged at 20,000 × g for 1 minute (the first time). The flow-through was disposed of again, the column was placed in the collection tube again, and 700 µl of PE was applied and centrifuged at 20,000 × g for 1 minute (the second time). The flow-through was disposed of, the column was placed in the collection tube again, and 700 µl of PE (Qiagen) was applied and centrifuged at 20,000 × g for 1 minute (the third time). The flow-through was disposed of, the column was placed in the collection tube again, and the empty column was centrifuged at 20,000 × g for 1 minute to completely shake down the residue. 50 µl of TE was applied and incubated at room temperature for 1 minute, followed by centrifugation at 20,000 × g for 1 minute to elute a plasmid.

1 µl of the eluate was applied to NanoDrop to measure the concentration and purity. It was confirmed that the concentration was several dozen ng/µl, and the purity was 260/280 = 1.8 to 2.0, 260/230 = 2.0 to 3.0. 26.2 µl of the sample was dissolved into 158 µl of the sample and digested by the following restriction enzymes at 8 µl each to confirm the structure: BamHI-HF, BglII (NEB3.1), EcoRI-HF, EcoRV-HF, HindIII-HF, KpnI-HF, NotI-HF, PstI-HF, PvuII-HF, SacI-HF, SalI-HF, SfiI, SmaI, or XbaI (except for BglII, CutSmart buffer) without cleavage.

### (Example 2: Production of an AAV vector from an amplified vector plasmid)

A vector plasmid amplified in *Escherichia coli* and hay bacillus was introduced into a producer cell to cause the producer cell to produce an AAV vector. SignaGen Laboratories (US) was asked to conduct the experiment of this example.

pGETS103-AAV-Helper-RC2 (1.19 × 10¹³ copies) amplified in *Escherichia coli* or hay bacillus, pGETS103-AAV:pGETS103-Helper:pGETS103-RC2=1:1:1 (1.19 × 10¹³ copies in total) amplified in *Escherichia coli* or hay bacillus, or pAAV-CMV:pHelper:pRC2-mi342=1:1:1 (1.19 × 10¹³ copies in total) amplified in *Escherichia coli* was used for transfection. To these plasmid DNAs, PolyJet^{™} reagent at 2.7-fold amount (weight ratio) was mixed. The prepared DNA complex was transfected into 2 × 10⁸ cultured HEK293 cells (the number of passages: 12), and culture was further continued for 5 hours. After the transfected cells were collected and freeze-thawed three times, the cells were processed with Benzonaze^{®} at 37°C for 1 hour. Centrifugation was performed at 12500 rpm for 30 minutes, and the supernatant was collected. Next, after cesium chloride density-gradient ultracentrifugation was performed at 28000 rpm for 18 hours, rAAVs (recombinant adeno-associated virus vector particles) were collected and dispersed in PBS comprising 0.001% Pluronic^{®} F-68.

The genome copy titer of the created rAAVs was quantified using quantitative PCR. After each rAAV was processed with DNaseI, DNaseI was deactivated using proteinase K. Heat processing was performed at 98°C for 15 minutes, the capsid was denatured, and dilution was performed so that the rAAV would remain within the range of a calibration curve. Subsequently, measurement was performed by quantitative PCR. The target was ITR, and primers of the sequences of 5'-GGAACCCCTAGTGATGGAGTT-3' (SEQ ID NO: 67) and 5'-CGGCCTCAGTGAGCGA-3' (SEQ ID NO: 68) were used. Further, the amount of endotoxin comprised in the rAAV dispersed solution was quantified using Pierce LAL Endotoxin Quantitation Kit (Thermo Fisher Scientific, US).

**[Table 1]**

| Plasmids used for rAAV production | | | |
|---|---|---|---|
| rAAV # | Plasmid | Length [bp] | Microorganism in which plasmid was amplified |
| 1 | pGETS103-AAV-Helper-RC2 | 31884 | Hay bacillus |
| 2 | pGETS103-AAV | 17722 | Hay bacillus |
| | pGETS103-Helper | 24812 | |
| | pGETS103-RC2 | 20982 | |
| 3 | pGETS103-AAV-Helper-RC2 | 31884 | Escherichia coli |
| 4 | pGETS103-AAV | 17722 | Escherichia coli |
| | pGETS103-Helper | 24812 | |
| | pGETS103-RC2 | 20982 | |
| 5 | pAAV-CMV | 5031 | Escherichia coli |
| | pHelper | 11635 | |
| | pRC2-mi342 | 8189 | |

The amount of rAAV virus genome [VG/mL] produced under each plasmid condition and the amount of endotoxin [Unit/mL] were measured. Production of an rAAV virus was observed in a similar manner under all conditions. No endotoxin was detected under any condition.

### (Example 3: CCC purity of a constructed plasmid DNA)

The CCC (covalently closed circular) purity of the plasmid DNA that was used for rAAV creation was quantified using PlACE MDQ Plus (SCIEX) and dsDNA 1000 kit (AB SCIEX, Tokyo). 20 mL of ultrapure water was added to gel powder contained in the kit, and the mixture thereof was stirred all night. It was confirmed that the gel was completely dissolved, and the mixture was diluted 10-fold with 1 × TBE electrophoresis buffer (Thermofisher). SYBR Gold nucleic acid gel stain (Thermofisher) was added to the diluted gel to achieve 0.01 vol%, followed by filling the capillary with the gel. Each plasmid DNA was adjusted to 10 ng/µL with ultrapure water and measured using PlACE MDQ Plus.

The measurement samples were injected into the capillary at 0.2 psi for 2 seconds and subjected to electrophoresis at 9.0 kV for 20 minutes. Fluorescence (excitation wavelength: 488 nm, fluorescent wavelength: 520 nm, Dynamic range: 1000 RFU) was used for detection. The CCC purity of the plasmid DNA was calculated by dividing the peak area value of the CCC plasmid DNA by the sum of peak area values including other impurities.

The table below shows the results.

**[Table 2]**

| Type of plasmid | Presence or absence of BS-ori | Microorganism in which plasmid was amplified | CCC purity |
|---|---|---|---|
| pGETS103-AAV-Helper-RC2 | Present | Hay bacillus | 87% |
| pGETS103-AAV-Helper-RC2 | Present | Escherichia coli | 78% |
| pGETS103-RC2 | Present | Hay bacillus | 94% |
| pGETS103-RC2 | Present | Escherichia coli | 91% |
| pRC2-mi342 | Absent | Escherichia coli | 91% |

For both plasmids pGETS103-AAV-Helper-RC2 and pGETS103-RC2, the CCC purity was higher in hay bacillus than in *Escherichia coli.* The plasmid of the present disclosure can have a high CCC purity. The guidance of the FDA specifies that the CCC purity shall exceed 80%. Since a plasmid produced using hay bacillus was observed to have a high CCC purity, said plasmid can reduce the burden of the purification operation for improving the CCC purity and the manufacturing costs. It is believed that CCC purity is also associated with safety, functionality, and transfection efficiency of a plasmid DNA. A plasmid DNA with a high CCC purity is highly useful.

### (Example 4: The proportion of rAAVs comprising a complete genome)

Each rAAV that had been cryopreserved in SignaGen was thawed, and the rAAV genome was subjected to quantitative PCR using Step One plus (Thermofisher). DNaseI (Takara Bio) prepared to 250 U/mL and the created rAAV were mixed at an equal amount, followed by heating the mixture thereof at 37°C for 30 minutes using a thermal cycler for PCR (Thermofisher). Next, 0.04 M of EDTA buffer (pH 8.0, Takara Bio) was added, and the mixture thereof was diluted 2-fold and heated at 55°C for 30 minutes. Furthermore, the resulting product was diluted 2.5-fold with nuclease-free water (Promega) and heated at 95°C for 10 minutes. Finally, TE buffer (Promega) was added to dilute the product 10-fold, and the extracted rAAV genome was used as a template for quantitative PCR.

A reaction solution for quantitative PCR comprises 10 µL of QuantiTect SYBR Green PCR Master mix (QIAgen), 1 µL of 0.01 mM primer (forward), 1 µL of 0.01 mM primer (reverse), 6 µL of water, and 2 µL of template per well on a plate. After the plate was sealed with a seal, quantitative PCR was performed. As a reaction solution, the following two types of reaction solutions were prepared: a reaction solution comprising primers targeting ITR (forward: 5'-GGAACCCCTAGTGATGGAGTT-3' (SEQ ID NO: 67) and reverse: 5'-CGGCCTCAGTGAGCGA-3' (SEQ ID NO: 68)); and a reaction solution comprising primers targeting CMV (forward: 5'-CATCAATGGGCGTGGATAGC-3' (SEQ ID NO: 69) and reverse: 5'-GGAGTTGTTACGACATTTTGGAAA-3' (SEQ ID NO: 70)).

As a PCR condition, after heating at 95°C for 15 minutes, a cycle of (1) 15 seconds at 94°C, (2) 30 seconds at 60°C, and (3) 30 seconds at 72°C was repeated 40 times. A calibration curve was created using a DNA that was prepared by linearizing pAAV-CMV by a restriction enzyme. The rAAV genome copy concentration was calculated from each Ct value. The proportion of rAAVs comprising a complete genome was calculated by dividing the genome copy concentration calculated with CMV as a target by the genome copy number calculated with ITR as a target.

The table below shows the results.

**[Table 3]**

| rAAV # | Genome copy titer [10⁹ VG/mL] | | Proportion of rAAVs comprising a complete genome |
|---|---|---|---|
| | Target: ITR | Target: CMV | |
| 1 | 1.35 | 1.26 | 94% |
| 2 | 32.0 | 27.1 | 85% |
| 3 | 6.25 | 6.00 | 96% |
| 4 | 186 | 128 | 69% |
| 5 | 207 | 139 | 67% |

The proportion of complete genomes was particularly high in rAAV#1 (all-in-one, hay bacillus) and rAAV#3 (all-in-one, *Escherichia coli).* Since a genome comprises a nucleic acid in a viral particle, it is difficult to separate a viral particle comprising a complete genome from a viral particle comprising an incomplete genome after creating a virus vector, such that it is very preferable that a viral particle comprising a complete genome at a high proportion can be prepared when creating a virus vector. The plasmid of the present disclosure can provide a virus vector which highly efficiently comprises a complete genome.

### (Example 5: The proportion of rAAVs comprising a marker gene)

Each rAAV that had been cryopreserved in SignaGen was thawed, and Ampr gene comprised in a plasmid backbone was subjected to quantitative PCR using Step One plus. The rAAV genome extracted from a liquid prepared by adding TE buffer to the created rAAV to dilute it 3-fold and then heating the resulting product at 95°C for 10 minutes by a thermal cycler for PCR was used as a template for quantitative PCR.

A reaction solution for quantitative PCR comprises 10 µL of QuantiTect SYBR Green PCR Master mix, 1 µL of 0.01 mM primer (forward: 5'-TTGATCGTTGGGAACCGGAG-3' (SEQ ID NO: 71)), 1 µL of 0.01 mM primer (reverse: 5'-TTGTTGCCGGGAAGCTAGAG-3' (SEQ ID NO: 72)), 6 µL of water, and 2 µL of template per well on a plate. After the plate was sealed with a seal, PCR was performed.

As a PCR condition, after heating at 95°C for 15 minutes, a cycle of (1) 15 seconds at 94°C, (2) 30 seconds at 60°C, and (3) 30 seconds at 72°C was repeated 40 times. A calibration curve was created using a DNA that was prepared by linearizing pAAV-CMV by a restriction enzyme. The genome copy concentration was calculated from each Ct value. The proportion of rAAVs comprising a marker gene was calculated by dividing the genome copy concentration calculated with Ampr as a target by the rAAV genome copy number calculated with CMV as a target.

The table below shows the results.

**[Table 4]**

| rAAV # | Genome copy titer [VG/mL] | | Proportion of rAAVs comprising a marker gene |
|---|---|---|---|
| | Target: CMV | Target: Ampr | |
| 1 | 1.26E+09 | 2.72E+06 | 0.2% |
| 2 | 2.71E+10 | 8.76E+08 | 3.2% |
| 3 | 6.00E+09 | 1.42E+08 | 2.4% |
| 4 | 1.28E+11 | 2.45E+08 | 0.2% |
| 5 | 1.39E+11 | 2.16E+09 | 1.6% |

The proportion of impurities was particularly low in rAAV#1 (all-in-one, hay bacillus) and rAAV#4 (three types-mixed plasmid, *Escherichia coli*)*.* It was suggested that a combination of a plasmid with an all-in-one structure and hay bacillus is superior.

### (Example 6: The proportion of empty capsids)

Each rAAV that had been cryopreserved in SignaGen was thawed, and the capsid particle concentration was quantified by the ELISA method using ARVO X5 (PerkinElmer). AAV2 Titration ELISA 2.0R (PROGEN) was utilized as a quantification kit, and the test was conducted based on the protocol. 100 µL of each of rAAV and a standard product (empty capsid reagent contained in the kit) was added to an anti-AAV2 antibody-immobilized 96-well plate contained in the kit. The sample was left standing at 37°C for 1 hour, followed by disposing of the liquid and washing with 200 µL of assay buffer three times. 100 µL of biotin-conjugated anti-AAV2 antibody was added and the product thereof was left standing at 37°C for 1 hour, followed by disposing of the liquid and washing with 200 µL of assay buffer three times. Furthermore, 100 µL of HRP-labelled streptavidin was added and the product thereof was left standing at 37°C for 1 hour, followed by disposing of the liquid and washing with 200 µL of assay buffer three times. Finally, 100 µL of TMB was added and the product thereof was left standing at room temperature for 15 minutes, followed by adding a reaction stopping reagent and measuring an absorbance (450 nm, 650 nm) by ARVO X5.

The proportion of empty capsids was calculated by subtracting a value obtained by dividing the rAAV genome copy concentration (genome copy titer) calculated with CMV as a target by the capsid particle concentration (viral particle titer) quantified by the ELIZA method from 1.

The table below shows the results.

**[Table 5]**

| rAAV # | Genome copy titer | Viral particle titer | Proportion of empty capsids |
|---|---|---|---|
| | [10⁹VG/mL] | [10⁹VP/mL] | |
| 1 | 1.26 | 2.93 | 57% |
| 2 | 27.1 | 116 | 77% |
| 3 | 6.00 | 24.8 | 76% |
| 4 | 128 | 417 | 69% |
| 5 | 139 | 558 | 75% |

The proportion of empty capsids was particularly low in rAAV#1 (all-in-one, hay bacillus). Since empty capsids can be immunogenic, they are preferably decreased. As described in Example 2, cesium chloride density-gradient ultracentrifugation was performed when obtaining rAAVs, and there is a possibility that empty capsids have been partially removed through this operation. However, it is considered that empty capsids have not been essentially removed. It is generally difficult to efficiently remove empty capsids even by performing column chromatography. Thus, it is particularly preferable to decrease the generation of empty capsids in the upstream process. However, the proportion of empty capsids is particularly low in a virus vector created by the method of the present disclosure, and highly efficient production of a virus vector can be possible. Thus, burden of the operation to remove empty capsids, which can require labor such as separation methods based on a slight difference in density, can be reduced.

### (Example 7: Plasmid construction by OGAB method)

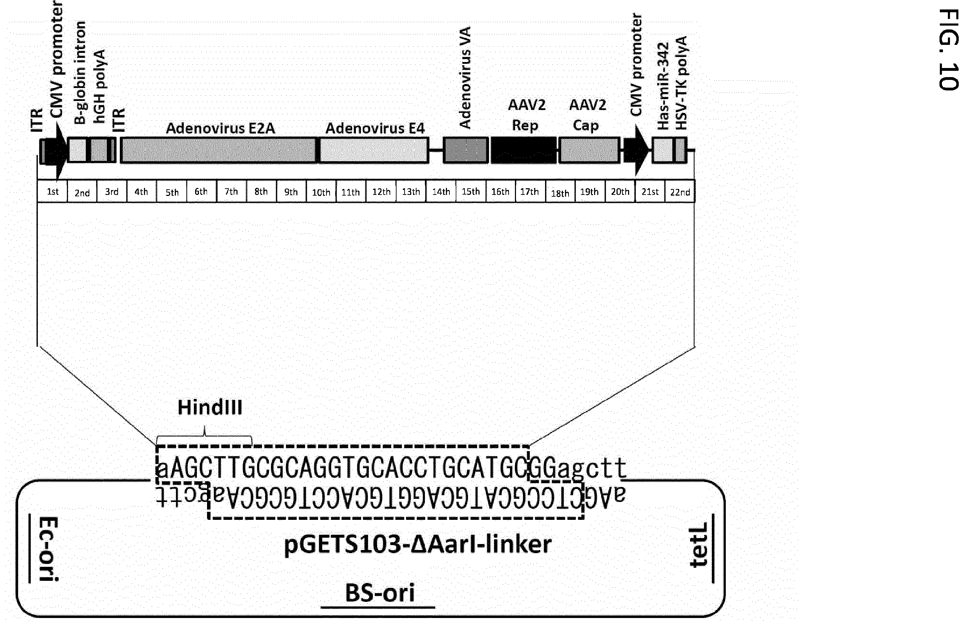

pGETS103-AAV-Helper-RC2 was constructed by OGAB method (Figure **10**).

### Construction of a vector DNA for OGAB assembling

Plasmid pGETS103ΔAarI was cleaved by restriction enzyme HindIII and purified through TE saturated phenol processing, butanol extraction, and ethanol precipitation. A linker DNA created by annealing a single-stranded DNA set forth in SEQ ID NOs: 3 and 4 was inserted thereto to create a vector for OGAB assembling, pGETS103-ΔAarI-Linker. This plasmid was cleaved by restriction enzyme AarI, size separation was performed by low melting point agarose gel electrophoresis, and large fragments of 15 kb were cut out from the gel and purified, thereby obtaining a vector fragment for OGAB assembling.

### Preparation of assembling fragments for OGAB

It was investigated whether it would be possible to reconstitute the region of AAV-Helper-RC2 in the base sequence of pGETS103-AAV-Helper-RC2 which is a region excluding the base sequence of pGETS103 that is the vector moiety (Figure **10**) by OGAB method. First, the above-described DNA region was designed to be divided into 22 fragments shown in Figure **10****.** Since the 3rd fragment comprises a region of the border between an AAV fragment and a Helper fragment and the 16th fragment comprises a region of the border between a Helper fragment and an RC2 fragment, the 1st, 3rd, and 16th fragments were prepared by MAP method (Japanese Patent Application No. 2018-93024) after chemically synthesizing material DNAs as shown below. Specifically, a doublestranded DNA obtained by assembling single-stranded DNAs of SEQ ID NOs: 5 to 10, SEQ ID NOs: 11 to 16, and SEQ ID NOs: 17 to 22 by MAP method was used for the 1st fragment, the 3rd fragment, and the 16th fragment, respectively. For the rest of the regions, pAAV-CMV Vector, pHelper Vector, and pRC2-mi342 Vector were used as a template for the 2nd fragment, the 4th to 15th fragments, and the 17th to 22nd fragments, respectively, and amplified by PCR under the following condition by using a combination of F and R primers with the numbering for each fragment set forth in SEQ ID NOs: 23 to 66. For each reaction, 10 µl of 2 × Prime Star mix (Takara Bio), 1 µl of template DNA, 1 µl of a solution comprising 3.2 pmol of each primer, and 8 µl of sterile water were added, and after 2 minutes at 96°C, a cycle of 10 seconds at 98°C, 15 seconds at 55°C, and 5 seconds of 72°C was performed 30 times.

### Cloning of assembling fragments for OGAB into a vector

After DNA fragments were obtained by MAP method or PCR method, these DNAs were purified using MinElute PCR Purification Kit (Qiagen) and finally eluted from a column by 15 µl of TE buffer (Nacalai Tesque). 0.2 µl of 10 × Ex-Taq buffer (Takara Bio), 0.2 µl of 2 mM dNTP (Takara Bio), and 0.2 µl of 10xA-attachment mix (TOYOBO) were added to 1.4 µl of the obtained DNA solution, and the mixture thereof was reacted at 60°C for 1 hour. Subsequently, 1 µl of pMD19 simple (Takara Bio) was diluted 20-fold with TE buffer, 3 µl of DNA Ligation Kit <Mighty Mix> was added thereto, a ligation reaction was performed at 16°C for 3 hours, and transformation was performed using *Escherichia coli* JM109 competent cells (Takara Bio). After culture all night, the base sequence of colonies that appeared on a carbenicillin-containing LB plate was confirmed, thereby obtaining a clone with a correct sequence for each of the fragments.

### Preparation of an equimolar mixture of fragments for OGAB

After *Escherichia coli* having these clones was proliferated in 2 ml of LB medium, 900 µl thereof was used to purify the plasmids by automated system QIAcube by QIAprep Spin Miniprep Kit (Qiagen), which were finally eluted into 30 µl of TE buffer. A solution comprising the plasmid DNA corresponding to 2.5 µ thereof was added with TE buffer so that the solution would be 50 µl. Furthermore, 6 µl of 10XPlasmid safe buffer (epicentre), 2.4 µl of 25 mM ATP, and 2 µl of Plasmid-Safe ATP-Dependent DNase (epicentre) were added thereto, and the mixture thereof was reacted at 37°C for 1 hour, followed by deactivation at 70°C for 30 minutes to thereby cleave DNAs other than those with a cyclic structure. Subsequently, the reaction solution was purified using MinElute PCR Purification Kit (Qiagen) and finally eluted from the column using 15 µl of TE buffer (Nacalai Tesque) . The obtained DNA solution was measured using a trace spectrophotometer (Nano drop One, Thermofisher), and diluted by addition of TE buffer so that the solution would be 100 ng/µl. Subsequently, the concentration was measured again, the DNA volume required for accurately fractionating 500 ng of DNA was calculated to µl of two decimal places, and DNA was fractionated by a micropipette, thereby equimolarly fractionating each plasmid. The fractionated DNA solution was pooled separately in two 1.5 ml centrifugation tubes depending on the type of restriction enzyme that was subsequently used. The plasmids for cloning the 1st, 3rd, 5th, 7th, 9th, 11th, 13th, 14th, 17th, 19th, and 21st fragments were pooled in a tube for cleavage by restriction enzyme AarI while the plasmids for cloning the 2nd, 4th, 6th, 8th, 10th, 12th, 15th, 16th, 18th, 20th, and 22nd fragments were pooled in a group for cleavage by restriction enzyme BsaI. Subsequently, 1.94 volume of sterile water, 0.33 volume of 10 × Buffer AarI (Thermofisher), 0.06 volume of 50 × oligonucleotide (0.025 mM), and 0.17 volume of AarI (Thermofisher), when considering the sum of the volume of each plasmid as 1 volume, were added to the tube for cleavage by AarI, and the mixture thereof was reacted at 37°C for 2 hours. Further, 2 volume of sterile water, 0.33 volume of 10XCutSmart buffer (NEB), and 0.17 volume of BsaI-HF v2, when considering the sum of the volume of each plasmid as 1 volume, were added to the tube for cleavage by BsaI, and the mixture thereof was reacted at 37°C for 2 hours. To each of the tubes, TE saturated phenol was added at an equal amount to the restriction enzyme reaction solution and mixed well to deactivate the restriction enzymes, followed by integrating the mixture of emulsified phenol and DNA solution into one 2 ml centrifugation tube and subjected to centrifugation at 20,000 × g for 10 minutes, thereby separating the mixture into a phenol phase and an aqueous phase. The upper layer was transferred to a new tube, 1-butanol at an equal amount was added thereto and stirred well, and the mixture thereof was centrifuged at 20,000 × g for 1 minute. Subsequently, the upper layer was removed by aspiration with a pipette, and the operation of adding 1-butanol at an equal amount again, performing centrifugation, and disposing of the upper layer was repeated until the volume of the bottom layer was 450 µl or less. Subsequently, 50 µl of P3 buffer was added, 900 µl of ethanol was added, and the mixture thereof was centrifuged at 20,000 × g for 10 minutes. After the supernatant was disposed of in a manner that the precipitate would not be lost, the resulting product was rinsed with 900 µl of 70% ethanol, followed by aspirating the supernatant with a pipette and disposing of the supernatant. Subsequently, centrifugation was performed again to collect the remaining supernatant at the bottom, and the liquid was completely removed by a pipette. 50 µl of TE was immediately added and the precipitate was tapped for 5 minutes to completely dissolve the precipitate. Subsequently, an equimolar mixture of 22 types of fragments of about 750 bp cut out from pMD19 was size-fractionated by electrophoresis with low melting point agarose gel, and the mixture of 22 types of fragments was purified in accordance with the method of cutting out DNA fragments from electrophoresis gel and purification described in Example 1.

### pGETS103-AAV-Helper-RC2 reconstitution by OGAB method

Subsequently, TE buffer was added to 1 fmol of the obtained DNA solution and 1 fmol of pGETS103ΔAarI-Linker that was cleaved by AarI and purified so that the total amount would be 4 µl. 5 µl of 2 × ligation buffer (15% polyethylene glycol 6000, 500 mM NaCl, 132 mM Tris·HCl (pH 7.6), 13.2 mM MgCl₂, 20 mM DTT, and 0.2 mM ATP) was added thereto and mixed well, followed by adding 1 µl of T4 DNA Ligase (Takara Bio) and incubation at 37°C for 5 hours. 100 µl of hay bacillus competent cells were added thereto and the mixture thereof was rotary-cultured by a duck rotor at 37°C for 30 minutes. Subsequently, 300 µl of LB medium was added and the mixture thereof was rotary-cultured by a duck rotor at 37°C for 1 hour. Subsequently, the culture broth was spread on an LB plate containing 10 µg/ml of tetracycline (Sigma-Aldrich) and cultured overnight at 37°C. 94 colonies were obtained.

Confirmation of the plasmid structure of a transformant

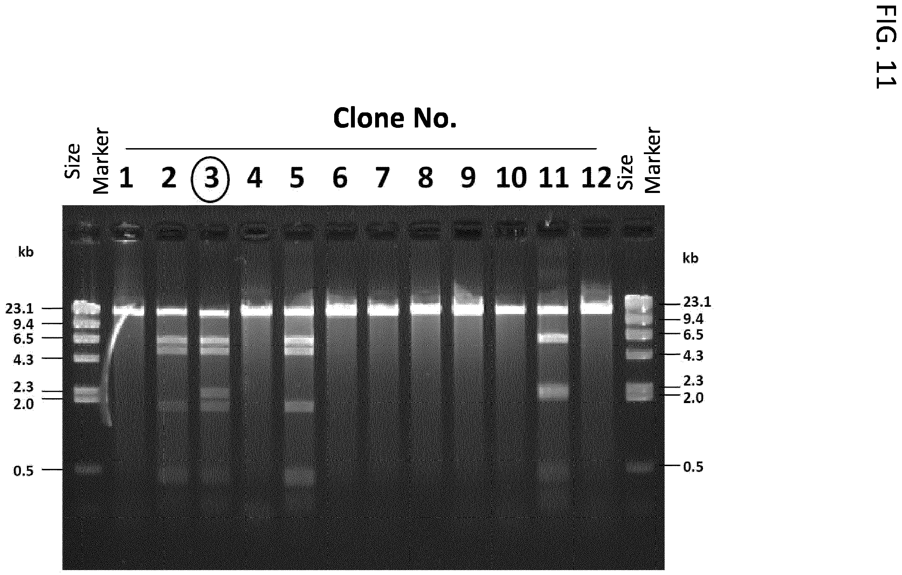

Colonies of 12 strains were randomly selected and cultured in an LB medium containing 2 ml of 10 µg/ml tetracycline overnight. IPTG was added to amplify the copy number of the inside plasmid so that the final concentration would be 1 mM, and further cultured at 37°C for 3 hours. A small amount of plasmid was extracted from the obtained microbial body in the following manner. 900 µl of microbial solution was placed in a 1.5 ml centrifugation tube and centrifuged at 6800 × g for 3 minutes, and the supernatant was removed with a micropipette. After the obtained microbial body pellet was suspended well, 100 µl of P1 buffer comprising 10 mg/ml of egg white lysozyme (wako) was added, followed by incubation at 37°C for 5 minutes. 200 µl of P2 buffer was added thereto and inverted and mixed four times. Subsequently, 150 µl of P3 was added and inverted and mixed four times. The resulting product was separated into a white precipitate and a supernatant by being centrifuged at 20,000 × g for 10 minutes. The supernatant was transferred to a new 1.5 ml centrifugation tube, and 450 µl of TE saturated phenol (Nacalai Tesque) was added thereto and mixed well, followed by centrifuging the mixture thereof at 20,000 × g for 10 minutes to separate a phenol phase and an aqueous phase. 320 µl of the aqueous phase was transferred to a new tube, 900 µl of ethanol was added thereto, and the mixture thereof was centrifuged at 20,000 × g for 10 minutes. The supernatant was removed with a micropipette, 900 µl of 70% ethanol was added, and the whole tube was rinsed. Subsequently, 70% ethanol was removed with a micropipette, and the obtained precipitate was dissolved with 27 µl of TE buffer. 8 µl of the obtained plasmid solution was collected, 1 µl of 10 × 3.1 NEB buffer (NEB) and 1 µl of SmaI were added thereto, and the mixture thereof was reacted at 37°C for 1 hour, followed by confirming the cleavage pattern by agarose gel electrophoresis. As shown in Figure **11****,** one (Clone No. 3) out of 12 clones exhibited an expected cleavage pattern. The whole base sequence of this clone was determined to confirm that pGETS103-AAV-Helper-RC2 was reconstituted.

### (Example 8: Construction of various AAV all-in-one vector plasmids)

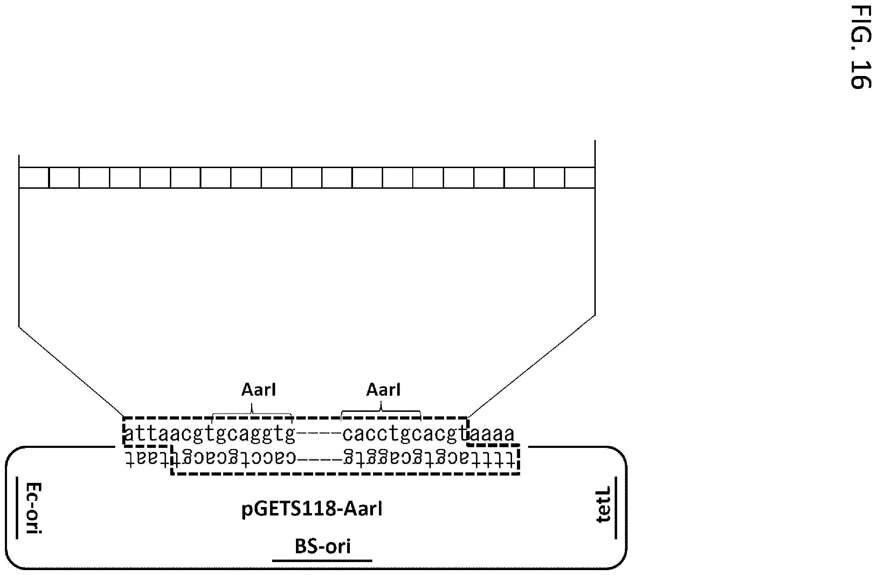

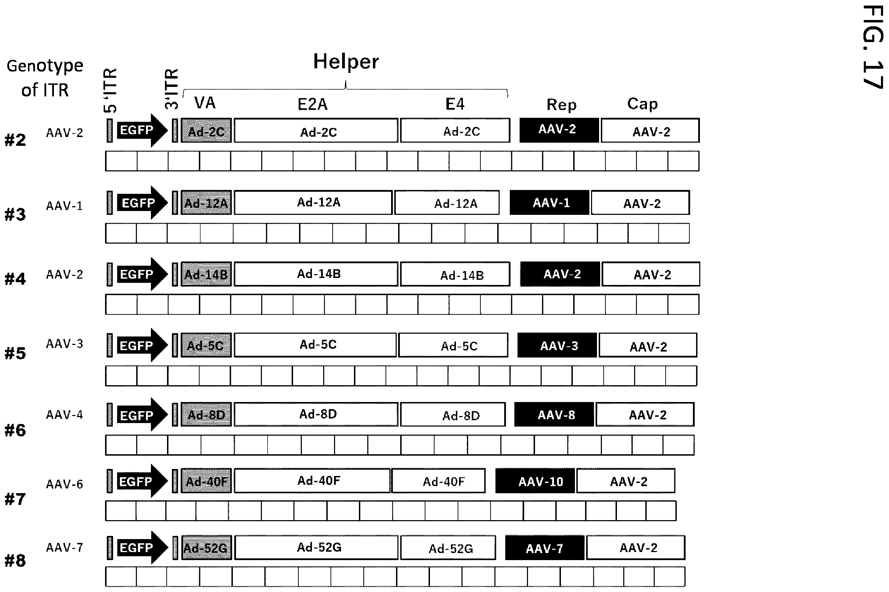

Similarly, 7 types of vector plasmids with an all-in-one structure for producing an AAV virus vector were constructed using an AAV genome of other serotypes and a constituent element obtained from an adenovirus genome. Each plasmid was constructed based on pGETS118-AarI (Tsuge, K., Sato, Y., Kobayashi, Y., Gondo, M., Hasebe, M., Togashi, T., Tomita, M., and Itaya, M. Method of preparing an equimolar DNA mixture for one-step DNA assembly of over 50 fragments. Sci Rep 5, 10655 (2015).) (Figure **16** shows the schematic diagram) . The table below shows the serotypes of the virus genome from which ITR, Rep, Cap, and Helper (VA, E2A, and E4) that were used in each of the vector plasmids are derived (see Figure **17** as well).

**[Table 6]**

| Vector plasmid No. | 5' ITR | 3' ITR | Helper | Rep | Cap |
|---|---|---|---|---|---|
| 1∗ | AAV-2 | AAV-2 | Ad-2C | AAV-2 | AAV-2 |
| 2 | AAV-2 | AAV-2 | Ad-2C | AAV-2 | AAV-2 |
| 3 | AAV-1 | AAV-1 | Ad-12A | AAV-1 | AAV-2 |
| 4 | AAV-2 | AAV-2 | Ad-14B | AAV-2 | AAV-2 |
| 5 | AAV-3 | AAV-3 | Ad-5C | AAV-3 | AAV-2 |
| 6 | AAV-4 | AAV-4 | Ad-8D | AAV-8 | AAV-2 |
| 7 | AAV-6 | AAV-6 | Ad-40F | AAV-10 | AAV-2 |
| 8 | AAV-7 | AAV-7 | Ad-52G | AAV-7 | AAV-2 |

| | | | | | |
|---|---|---|---|---|---|
| The vector plasmid of *1 is the one used in the above Examples (pGETS103-AAV-Helper-RC2) . AAV: Adeno-associated virus serotype Ad: Adenovirus serotype and group | | | | | |

Each of the above all-in-one nucleic acids (15 to 16 kb in full length) was designed, and DNA fragments comprising 18 to 19 unit DNA fragments (700 to 900 bp) depending on the length of said nucleic acids were chemically synthesized. These DNA fragments were cut out by any of the restriction enzymes AarI, BbsI, BsaI, and BsmBI to prepare unit DNA fragments. These unit DNA fragments were linked to pGETS118 cleaved by AarI in a tandem repeat manner and then caused to form a cyclic plasmid in hay bacillus by OGAB method in a similar manner to the above Examples. As a result, construction of additional 7 types of AAV vector plasmids with an all-in-one structure described above was observed.

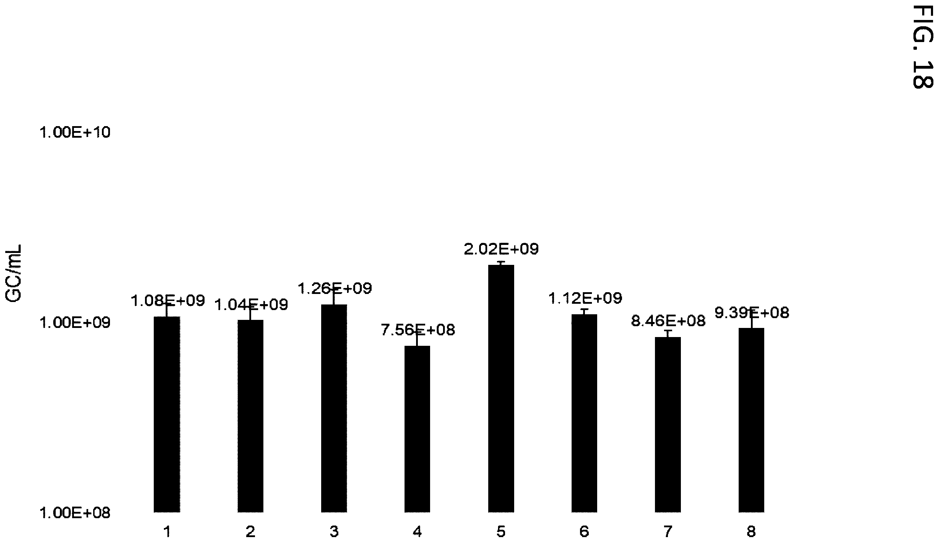

These plasmids were introduced into a cultured cell (producer cell) to confirm production of each virus vector (Figure **18**)**.**

### (Example 9: Construction of an adenovirus all-in-one vector plasmid)

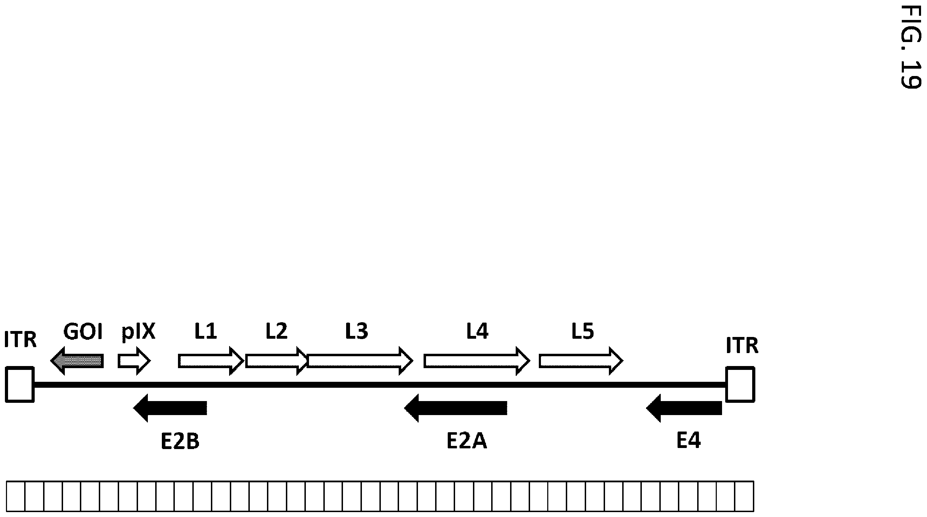

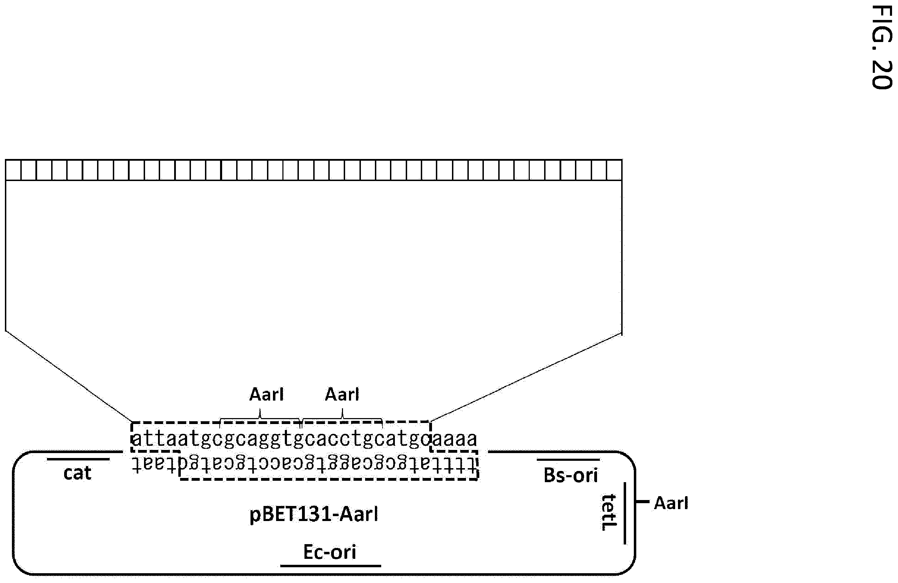

For adenovirus vector Ad5, an all-in-one structure for producing an adenovirus vector with a full length of about 36 kb resulting from introducing GIO gene into pAxCAwtit2 of Takara Bio was designed (Figure **19**). Said structure was divided into 40 fragments each having about 800 bp to prepare DNA fragments comprising a unit DNA fragment by PCR or chemical synthesis. These DNA fragments were cut out by any of the restriction enzymes AarI, BbsI, BsaI, and BsmBI to prepare unit DNA fragments, which were then linked, in a tandem repeat manner, to pGETS118-AarI cleaved by AarI or to two large fragments obtained by cleaving by AarI pBET131-AarI (Figure **20**), which was produced by introducing a linker DNA into BamHI site of pBET131 (Tanaka, T., and M. Ogura.1998. A novel Bacillus natto plasmid pLS32 capable of replication in Bacillus subtilis. FEBS Lett.422:243-246) and introducing two new AarI sites. Subsequently, a cyclic plasmid was formed in hay bacillus by OGAB method in a similar manner to the above Examples. As a result, construction of a clone with an all-in-one structure for producing an adenovirus vector was observed.

### (Example 10: Examples of the structure of further AAV virus all-in-one vector plasmids)

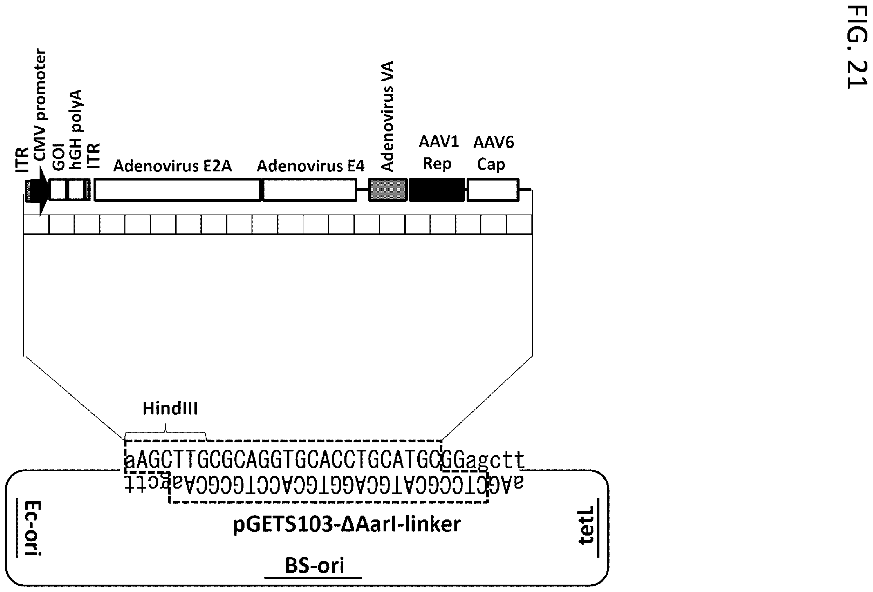

The AAV virus all-in-one vector plasmids shown in Figures **21** to **26** are also intended.
* Figure **21** is an example of construction based on pGETS103-ΔAarI using Rep of AAV1 and Cap of AAV6.

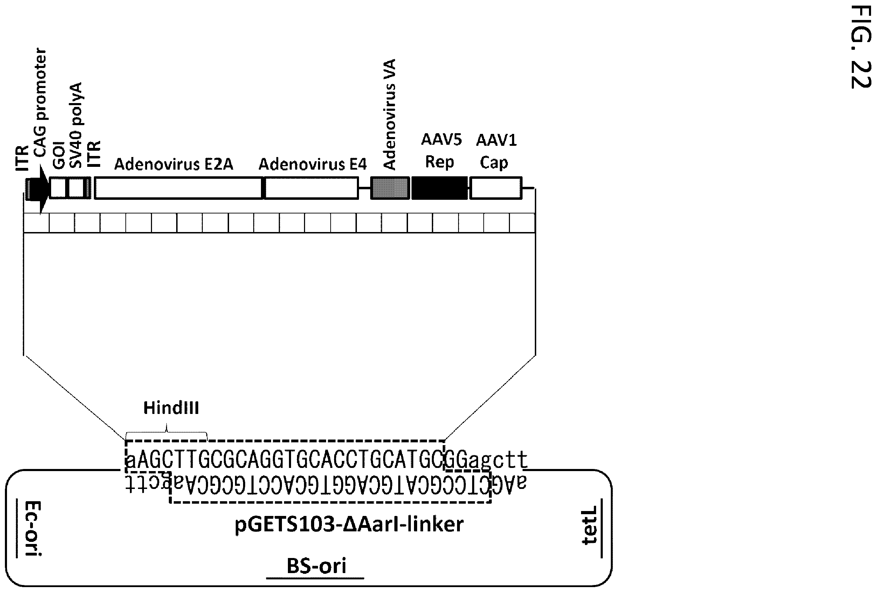

* Figure **22** is an example of construction based on pGETS103-ΔAarI using CAG promoter, Rep of AAV5, and Cap of AAV1.

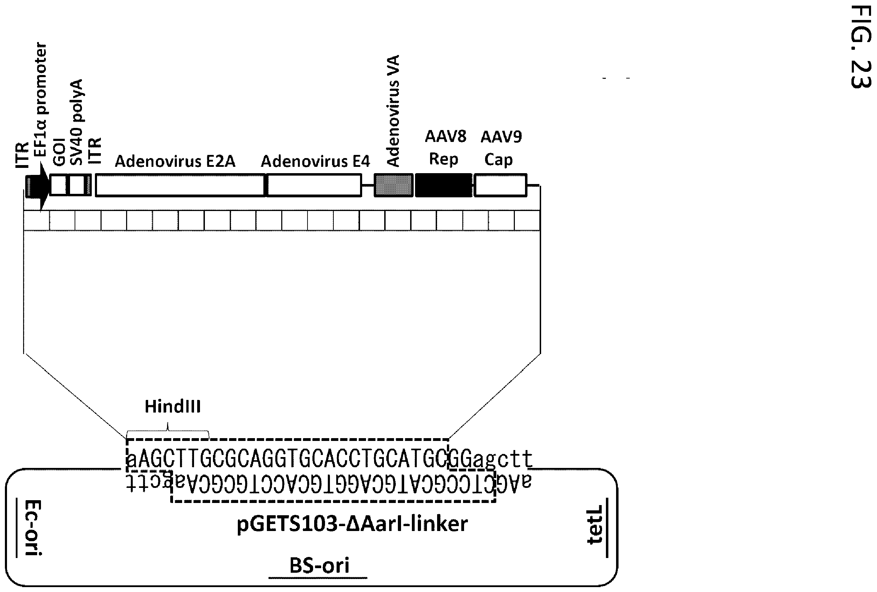

* Figure **23** is an example of construction based on pGETS103-ΔAarI using EF1α promoter, Rep of AAV8, and Cap of AAV9.

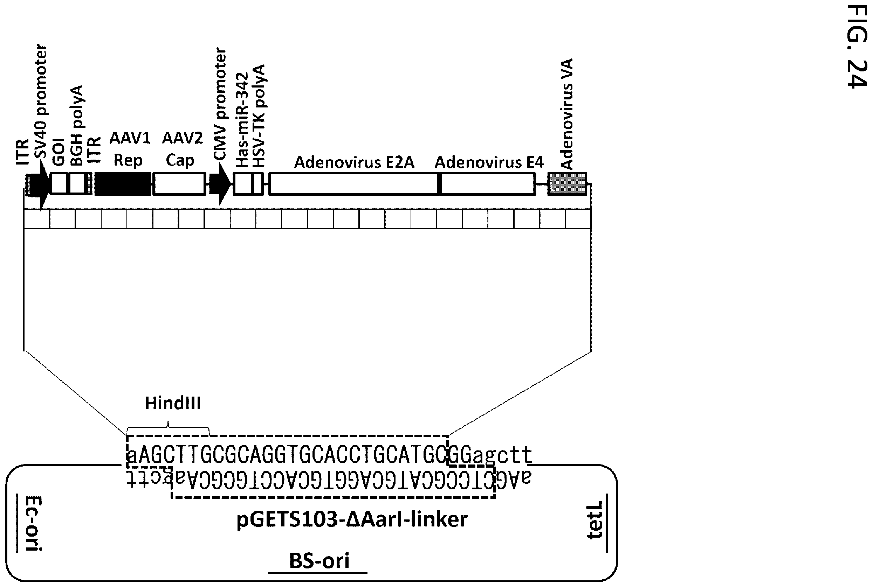

* Figure **24** is an example of construction based on pGETS103-ΔAarI using SV40 promoter, wherein Rep, Cap, and Helper are placed in a different order.

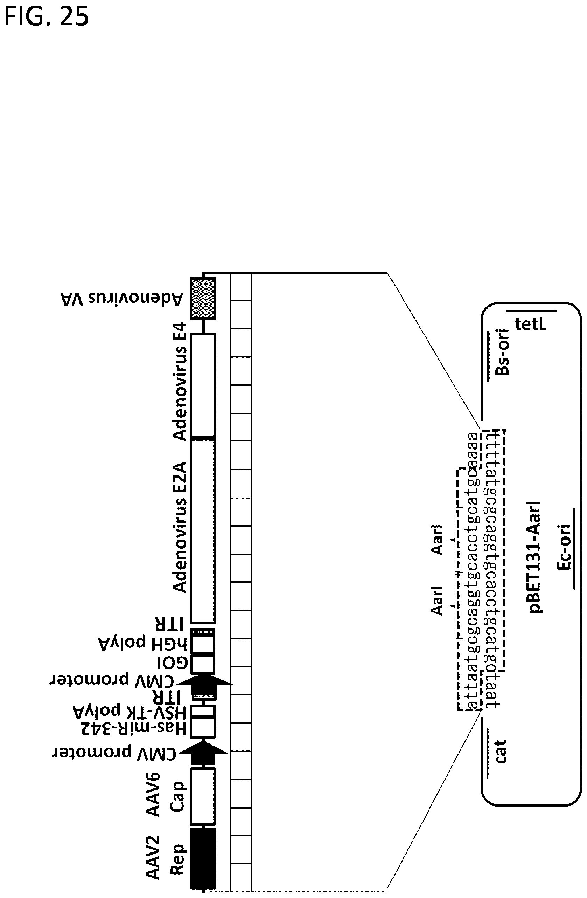

* Figure **25** is an example of construction based on pGETS131-AarI wherein Rep, Cap, and Helper are placed in a different order.

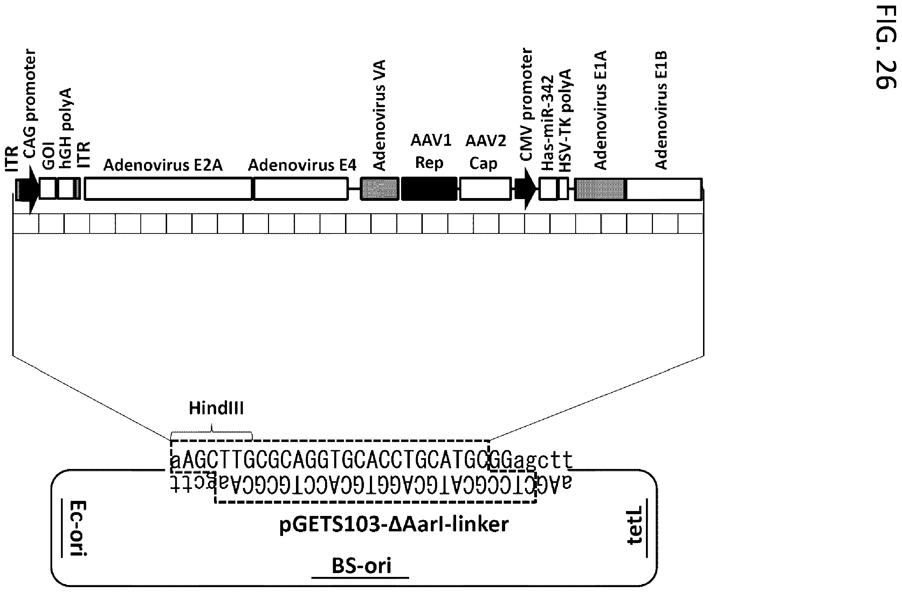

* Figure **26** is an example of construction based on pBETS103-ΔAarI, in which the element of Helper gene has been changed (adenoviruses E1A and E1B are added separately from other elements).

### (Example 11: Construction of a virus vector plasmid based on various viruses)

An AAV virus vector plasmid can be constructed to further comprise E1A and E1B genes in addition to the above-described structures of the Examples when a producer cell other than HEK293 (such as NIH3T3, HT1080, A549, HeLa, or HEK 293T) is used.

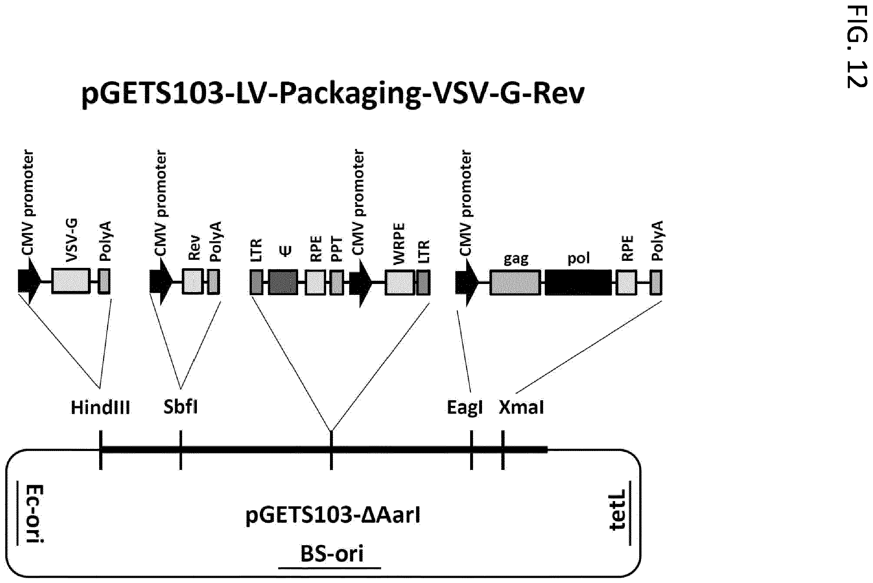

For example, Figures **12** to **15** based on pGETS103ΔAarI of the above-described Examples are contemplated as a specific configuration of a retrovirus vector plasmid, a lentivirus vector plasmid, a Sendai virus vector plasmid, or an adenovirus vector plasmid.

### Lentivirus vector plasmid (Figure 12)

A vector plasmid is constructed by adding the following nucleic acid sequences based on pGETS103ΔAarI.
* CMV promoter, VSV-G (glycoprotein G of a vesicular stomatitis virus), poly A·CMV promoter, rev, and poly A
* 5' LTR, Ψ (packaging signal sequence), RPE, PPT, CMV promoter, WPRE, and 3' LTR
* CMV promoter, gag, pol, RPE, and poly A
(A gene of interest can be positioned downstream of the promoter between LTRs.)

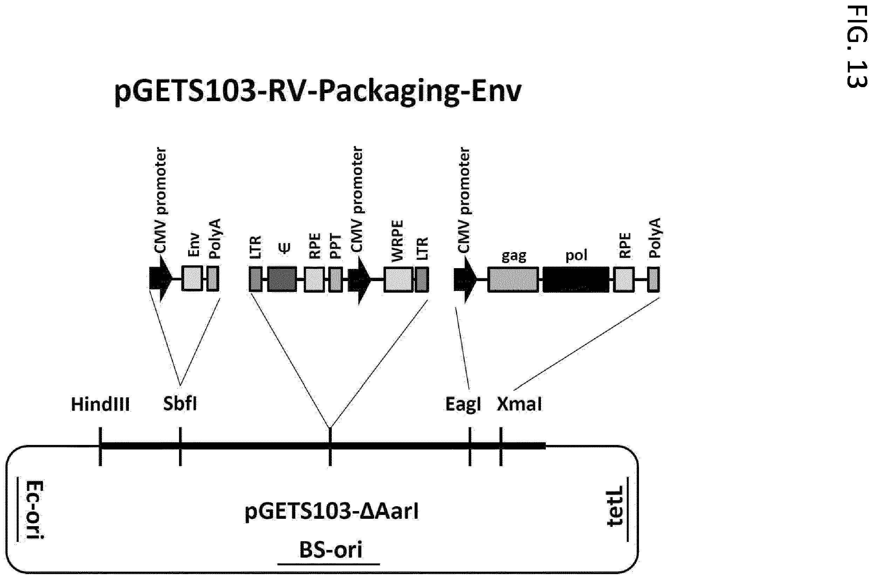

### Retrovirus vector plasmid (Figure 13)

A vector plasmid is constructed by adding the following nucleic acid sequences based on pGETS103ΔAarI.
* CMV promoter, env, and poly A
* 5' LTR, Ψ (packaging signal sequence), RPE, PPT, CMV promoter, WPRE, and 3' LTR
* CMV promoter, gag, pol, RPE, and poly A
(A gene of interest can be positioned downstream of the promoter between LTRs.)

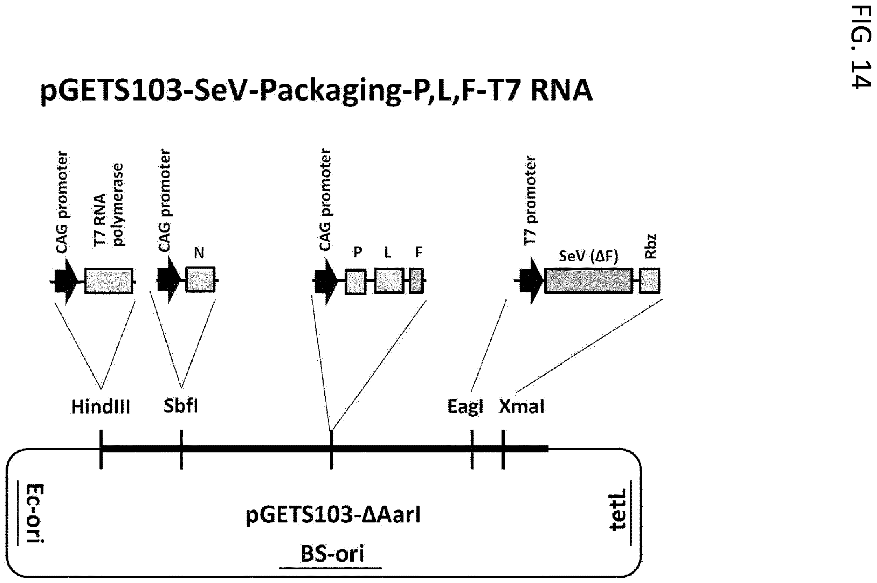

### Sendai virus vector plasmid (Figure 14)

A vector plasmid is constructed by adding the following nucleic acid sequences based on pGETS103ΔAarI
* CAG promoter and T7 RNA polymerase
* CAG promoter and N
* CAG promoter, P, L, and F
* T7 promoter, a Sendai virus genome with F deleted, and Rbz (A gene of interest can be positioned between any of the genes of the Sendai virus genome.)

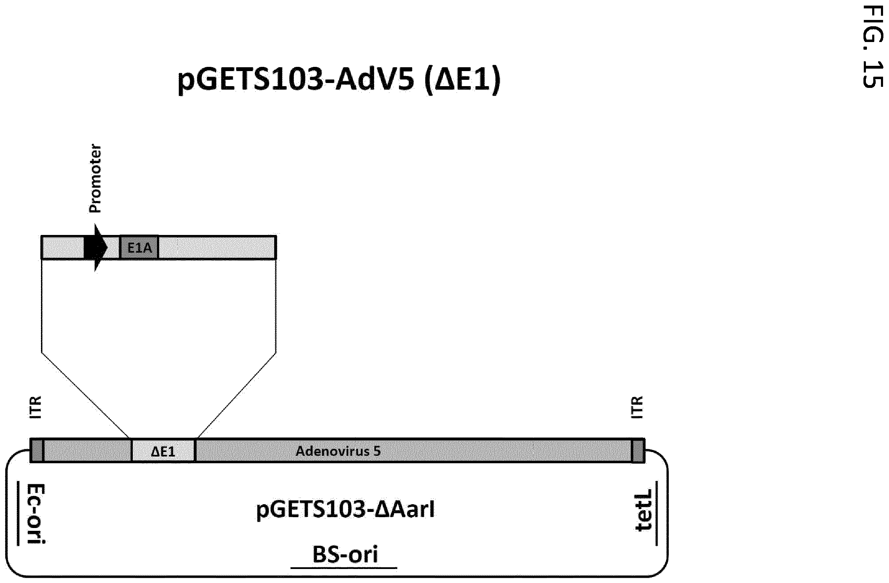

### Adenovirus vector plasmid (Figure 15)

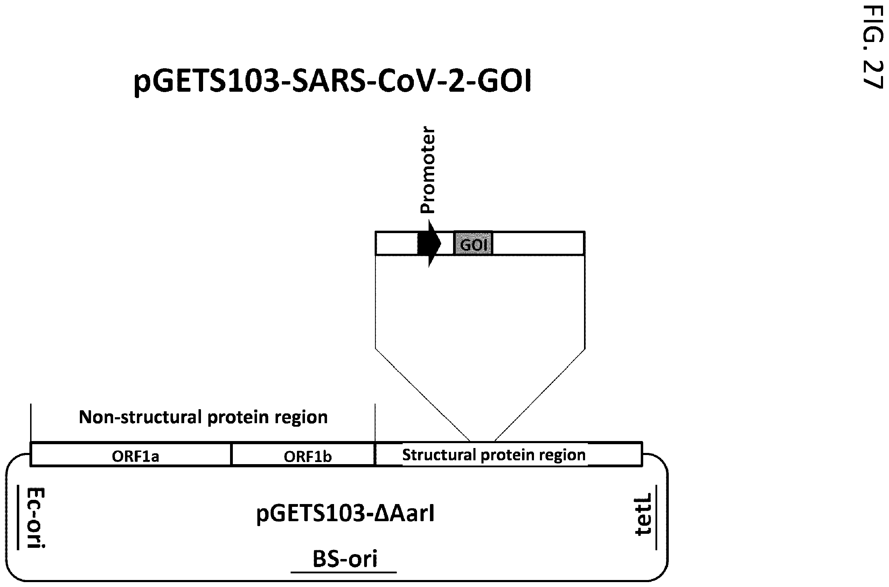

A vector plasmid is constructed by adding the following nucleic acid sequences based on pGETS103ΔAarI.
* 5' ITR, a genome of adenovirus serotype 5 (the E1 gene site is substituted with a promoter and E1A gene), and 3' ITR
   (In addition to the E1A gene, a gene of interest can also be positioned downstream of the above promoter or other promoters.)
* Corona virus vector plasmid (Figure **27**)
   A coronavirus vector plasmid is constructed by adding the following nucleic acid sequence based on pGETS103ΔAarI.
* The nucleic acid sequence comprises ORFla that is a non-structural protein region, ORF1b that is a non-structural protein region, and a structural protein region, wherein a gene of interest is comprised in the structural protein region. The nucleic acid sequence may be deficient in spike gene S, envelope gene E, matrix gene M, nucleocapsid gene N or the like in the structural protein region as needed.

### (Note)

As described above, the present disclosure is exemplified by the use of its preferred embodiments. However, it is understood that the scope of the present disclosure should be interpreted solely based on the Claims. It is also understood that any patent, any patent application, and any references cited herein should be incorporated herein by reference in the same manner as the contents are specifically described herein.

The present application claims priority to Japanese Patent Application No. 2020-184491 filed to the Japan Patent Office on November 4, 2020. The entire content thereof is incorporated herein by reference.

### [Industrial Applicability]

The present disclosure provides a vector plasmid with a new structure and can enable supply of a virus vector based on the vector plasmid.

### [Sequence Listing Free Text]

SEQ ID NO: 1: PCR product 
tagGGTCTCaAGCTtgCCTGCAGGcaGATCatgcgcaggtgcacctgcatgcGATCCGG
GGTTCGAACCCCGGTCGTCCGCCATGATACCCTTGCGAATTTATCCACCAGACCACGGA
AGAGTGCCCGCTTACAGGCTCTCCTTTTGCACGGTCTAGAGCGTCAACGACTGCGCACG
CCTCACCGGCCAGAGCGTCCCGACCATGGAGCACTTTTTGCCGCTGCGCAACATCTGGA
ACCGCGTCCGCGACTTTCCGCGCGCCTCCACCACCGCCGCCGGCATCACCTGGATGTCC
AGGTACATCTACGGATTACGTCGACGTTTAAACCATATGAGCGGCCGCatatatCCCGG
GAGCTaGAGACCtca
SEQ ID NO: 2: pGETS103-AAV-Helper-RC2
   (as described in the sequence listing)
SEQ ID NO: 3: linker F
   AGCTTGCGCAGGTGCACCTGCATGCGG
SEQ ID NO: 4: linker R
   AGCTCCGCATGCAGGTGCACCTGCGCA
SEQ ID NO: 5: 1-1
SEQ ID NO: 6: 1-2
SEQ ID NO: 7: 1-3
SEQ ID NO: 8: 1-4
SEQ ID NO: 9: 1-5 AATGGGAGTTTGTTTTGGCACCAAAATCAACGGGACT
SEQ ID NO: 10: 1-6
SEQ ID NO: 11: 3-1
SEQ ID NO: 12: 3-2
SEQ ID NO: 13: 3-3
SEQ ID NO: 14: 3-4
SEQ ID NO: 15: 3-5
SEQ ID NO: 16: 3-6
SEQ ID NO: 17: 16-1
SEQ ID NO: 18: 16-2 
CAGAATCTGGCGGCAACTCCCATTCCTTCTCGGCCACCCAGTTCACAAAGCTGTCAGAA
ATGCCGGGCAGATGCTCGTCAAGGTCGCTGGGGACCTTAATCACAATCTCGTAAAACCC
CGGCATGGCGGCTGCGCAGATCAGAAGTTCCTATAC
SEQ ID NO: 19: 16-3
SEQ ID NO: 20: 16-4
SEQ ID NO: 21: 16-5
SEQ ID NO: 22: 16-6
SEQ ID NO: 23: 1-F TAGCACCTGCATGCAAGCTTGCCTGCAGGCA
SEQ ID NO: 24: 1-R TAGCACCTGCGCATCTGGGCCCTTAAGGATATC
SEQ ID NO: 25: 2-F TAGGGTCTCGCCAGCCGGCC
SEQ ID NO: 26: 2-R TAGGGTCTCCTTCCCAATAGACCCCGCA
SEQ ID NO: 27: 3-F TAGCACCTGCATGCGGAACCAAGCTGGAGTG
SEQ ID NO: 28: 3-R TAGCACCTGCGCATTGTCCCTGCCAGTGG
SEQ ID NO: 29: 4-F TAGGGTCTCGGACACGTTGCGATACTGGT
SEQ ID NO: 30: 4-R TAGGGTCTCCCACGAGCCCACGG
SEQ ID NO: 31: 5-F
   TAGCACCTGCATGCCGTGGTGCTTGTAGGTTAC
SEQ ID NO: 32: 5-R
   TAGCACCTGCGCATGAGCGCGGACGC
SEQ ID NO: 33: 6-F
   TAGGGTCTCGGCTCGGGGGTGGT
SEQ ID NO: 34: 6-R
   TAGGGTCTCCTTGCCGCGCGTTTCTC
SEQ ID NO: 35: 7-F
   TAGCACCTGCATGCGCAAACGCTCTGCAACAAGA
SEQ ID NO: 36: 7-R
   TAGCACCTGCGCATGTCCGCCAGGTGC
SEQ ID NO: 37: 8-F
   TAGGGTCTCGGGACATTATCTTCCCCGAAC
SEQ ID NO: 38: 8-R
   TAGGGTCTCCGTGGCGGCGGC
SEQ ID NO: 39: 9-F
   TAGCACCTGCATGCCCACCCACGGACGA
SEQ ID NO: 40: 9-R
   TAGCACCTGCGCATGAGGGAGCGCAGAGA
SEQ ID NO: 41: 10-F
   TAGGGTCTCGCCTCACCCGCAGCTG
SEQ ID NO: 42: 10-R
   TAGGGTCTCCGACTAAAAAATGACGTAACGGTTAAAGTC
SEQ ID NO: 43: 11-F
   TAGCACCTGCATGCAGTCCTATATATACTCGCTCTGTACT
SEQ ID NO: 44: 11-R
   TAGCACCTGCGCATGGAGCTATGCTAACCAGC
SEQ ID NO: 45: 12-F
   TAGGGTCTCGCTCCGAGTATGCGTGTCA
SEQ ID NO: 46: 12-R
   TAGGGTCTCCGTAGTTGTAGTATATCCACTCTCTCA
SEQ ID NO: 47: 13-F
   TAGCACCTGCATGCCTACTACACAGAGCGAGCT
SEQ ID NO: 48: 13-R
   TAGCACCTGCGCATGCACAGCACCACAATATTGTTCA
SEQ ID NO: 49: 14-F
   TAGCACCTGCATGCGTGCTGCAGTTACTGTGCT
SEQ ID NO: 50: 14-R
   TAGCACCTGCGCATTAGCGAGGTAAGCACTTACTCT
SEQ ID NO: 51: 15-F
   TAGGGTCTCGGCTAGTTTCTGTGGATTCACTAGA
SEQ ID NO: 52: 15-R
   TAGGGTCTCCCCTGGACATCCAGGTGA
SEQ ID NO: 53: 16-F
   TAGGGTCTCGCAGGTACATCTACGGATTACGT
SEQ ID NO: 54: 16-R
   TAGGGTCTCCCTGCTCCTGCGTCTG
SEQ ID NO: 55: 17-F
   TAGCACCTGCATGCGCAGAACAAAGAGAATCAGAATCC
SEQ ID NO: 56: 17-R
   TAGCACCTGCGCATGGTGAGTTCAAATTTGAACATCCG
SEQ ID NO: 57: 18-F
   TAGGGTCTCGCACCCGCCGTCTG
SEQ ID NO: 58: 18-R
   TAGGGTCTCCCGAGGGCCGCG
SEQ ID NO: 59: 19-F
   TAGCACCTGCATGCCTCGAGCACGACAAAGC
SEQ ID NO: 60: 19-R
   TAGCACCTGCGCATTGACTTGAATGTTAAAGAGCTTGAAGTTGA
SEQ ID NO: 61: 20-F
   TAGGGTCTCGGTCAAAGAGGTCACGCAGA
SEQ ID NO: 62: 20-R
   TAGGGTCTCCTTGGTTGTCCTGATTTCCTCTTC
SEQ ID NO: 63: 21-F
   TAGCACCTGCATGCCCAATCCCGTGGCTAC
SEQ ID NO: 64: 21-R
   TAGCACCTGCGCATGCATATGATACACTTGATGTACTGC
SEQ ID NO: 65: 22-F
   TAGGGTCTCGATGCCAAGTACGCCCCCT
SEQ ID NO: 66: 22-R
   TAGGGTCTCCCTCCCGGGCTGTAGT
SEQ ID NO: 67: ITR targeting primer (forward)
   GGAACCCCTAGTGATGGAGTT
SEQ ID NO: 68: ITR targeting primer (reverse)
   CGGCCTCAGTGAGCGA
SEQ ID NO: 69: CMV targeting primer (forward)
   CATCAATGGGCGTGGATAGC
SEQ ID NO: 70: CMV targeting primer (reverse)
   GGAGTTGTTACGACATTTTGGAAA
SEQ ID NO: 71: Ampr gene region targeting primer (forward)
   TTGATCGTTGGGAACCGGAG
SEQ ID NO: 72: Ampr gene region targeting primer (reverse)
   TTGTTGCCGGGAAGCTAGAG

## Claims

1. A plasmid comprising:
a nucleic acid sequence which promotes plasmid replication in hay bacillus; and
at least one of nucleic acid sequences required for constituting a virus vector.

2. The plasmid of claim 1, wherein the nucleic acid sequences required for constituting a virus vector comprise:
a nucleic acid sequence encoding a capsid protein of the virus;
a nucleic acid sequence encoding a protein which packages, transcribes, and replicates a genome of the virus;
two terminal repeat sequences of the virus; and
a nucleic acid sequence encoding a helper gene.

3. The plasmid of claim 1, comprising at least two of:
a nucleic acid sequence encoding a capsid protein of the virus;
a nucleic acid sequence encoding a protein which packages, transcribes, and replicates a genome of the virus;
two terminal repeat sequences of the virus; and
a nucleic acid sequence encoding a helper gene.

4. The plasmid of claim 1, comprising at least three of:
a nucleic acid sequence encoding a capsid protein of the virus;
a nucleic acid sequence encoding a protein which packages, transcribes, and replicates a genome of the virus;
two terminal repeat sequences of the virus; and
a nucleic acid sequence encoding a helper gene.

5. The plasmid of claim 1, comprising:
a nucleic acid sequence encoding a capsid protein of the virus;
a nucleic acid sequence encoding a protein which packages, transcribes, and replicates a genome of the virus;
two terminal repeat sequences of the virus; and
a nucleic acid sequence encoding a helper gene.

6. The plasmid of any one of claims 1 to 5, wherein the nucleic acid sequences required for constituting a virus vector are about 10 kb or greater.

7. The plasmid of any one of claims 2 to 6, wherein at least one of the nucleic acid sequence encoding a capsid protein, the nucleic acid sequence encoding a protein which packages, transcribes, and replicates a genome, and the helper gene is outside a region sandwiched by the two terminal repeat sequences.

8. The plasmid of any one of claims 1 to 7, wherein the nucleic acid sequence which promotes plasmid replication in hay bacillus comprises a replication origin point which operates in hay bacillus.

9. The plasmid of any one of claims 1 to 8, further comprising a nucleic acid sequence which amplifies a plasmid in *Escherichia coli.*

10. The plasmid of any one of claims 2 to 9, wherein the nucleic acid sequence encoding a capsid protein of the virus comprises at least one of L1, L2, L3, L4, L5, cap, and gag.

11. The plasmid of any one of claims 2 to 10, wherein the nucleic acid sequence encoding a protein which packages, transcribes, and replicates a genome of the virus comprises at least one of E1A, E1B, E2A, E2B, E4, rep, ψ, APP, MAAP, pol, and rev.

12. The plasmid of any one of claims 2 to 11, wherein the terminal repeat sequences are inverted terminal repeats (ITRs) or long terminal repeats (LTRs).

13. The plasmid of any one of claims 2 to 12, wherein the helper gene comprises at least one of E1A, E1B, E2A, E4, VA, env, tat, RPE, PPT, PRE, and pro.

14. The plasmid of any one of claims 2 to 13, wherein at least one of the nucleic acid sequence encoding a capsid protein of the virus and the nucleic acid sequence encoding a protein which transcribes and replicates a genome of the virus is derived from any of serotypes 1 to 52 of an adenovirus or serotypes 1 to 12 of an adeno-associated virus and a variant thereof.

15. The plasmid of any one of claims 1 to 14, wherein the plasmid does not comprise a sequence of a gene of at least a part of a whole genome of the virus.

16. The plasmid of any one of claims 1 to 15, wherein the virus is an adenovirus, an adeno-associated virus, a lentivirus, a retrovirus, or a Sendai virus.

17. The plasmid of any one of claims 1 to 15, wherein the virus is an adeno-associated virus or a lentivirus.

18. The plasmid of any one of claims 1 to 15, wherein the virus is an adeno-associated virus.

19. The plasmid of any one of claims 2 to 18, wherein the terminal repeat sequences are inverted terminal repeats (ITRs) derived from any of serotypes 1 to 12 of an adeno-associated virus and a variant thereof.

20. The plasmid of claim 19, comprising a promotor, a gene of interest, and a terminator from upstream between 5'ITR and 3'ITR.

21. The plasmid of any one of claims 2 to 20, wherein the helper gene comprises at least one of E1A, E1B, E2A, E4, and VA.

22. The plasmid of any one of claims 2 to 20, wherein the helper gene comprises E2A, E4, and VA.

23. The plasmid of any one of claims 2 to 22, wherein the helper gene is each derived from any of serotypes 1 to 52 of an adenovirus and a variant thereof.

24. The plasmid of any one of claims 2 to 23, wherein the nucleic acid sequence encoding a protein which packages, transcribes, and replicates a genome of the virus comprises a rep.

25. The plasmid of claim 24, wherein the rep is derived from any of serotypes 1 to 12 of an adeno-associated virus and a variant thereof.

26. The plasmid of any one of claims 2 to 25, wherein the nucleic acid sequence encoding a capsid protein of the virus comprises a cap.

27. The plasmid of claim 26, wherein the cap is derived from any of serotypes 1 to 12 of an adeno-associated virus and a variant thereof.

28. The plasmid of any one of claims 2 to 27, comprising a gene of interest between the two terminal repeat sequences of the virus.

29. The plasmid of claim 28, wherein the gene of interest is 2 to 100 genes.

30. The plasmid of claim 28, wherein the gene of interest is 10 to 100 genes.

31. The plasmid of any one of claims 28 to 30, wherein the gene of interest comprises a promoter sequence.

32. The plasmid of any one of claims 1 to 31, wherein the plasmid and a producer cell comprise a nucleic acid sequence required for constituting the virus vector together.

33. The plasmid of any one of claims 1 to 32, wherein virus vector particles which do not comprise a nucleic acid in all virus vector particles which are produced from a producer cell introduced with the plasmid are 65% or less.

34. The plasmid of any one of claims 1 to 33, wherein, when the plasmid is introduced into a producer cell, virus vector particles comprising a nucleic acid comprising all genes of interest in all virus vector particles which are produced are 90% or greater.

35. The plasmid of any one of claims 1 to 34, wherein, when the plasmid is introduced into a producer cell, virus vector particles comprising a nucleic acid derived from the plasmid other than a desired nucleic acid in all virus vector particles which are produced are 2% or less.

36. The plasmid of any one of claims 1 to 35, wherein a CCC (covalently closed circular) purity is 80% or greater.

37. A composition for use in expressing a gene of interest, the composition comprising the plasmid of any one of claims 1 to 36, wherein the gene of interest is placed between the two terminal repeat sequences of the virus on the plasmid.

38. A method for producing the plasmid of any one of claims 1 to 36, the method comprising operably linking the nucleic acid sequence of any one of claims 1 to 32.

39. A composition comprising a plasmid produced by the method of claim 38.

40. The composition comprising a plasmid of claim 39, wherein a CCC (covalently closed circular) purity of a plasmid is 80% or greater.

41. A kit for use in creating the plasmid of any one of claims 1 to 36, the kit comprising at least one nucleic acid comprising:
a nucleic acid sequence which promotes plasmid replication in hay bacillus; and
a nucleic acid sequence required for constituting a virus.

42. A composition for use in preparing a virus vector, the composition comprising the plasmid of any one of claims 1 to 36.

43. A method for preparing a virus vector, the method comprising producing a virus vector by using the plasmid of any one of claims 1 to 36.

44. The method of claim 43, wherein at least a part of a nucleic acid contained in the plasmid is incorporated into a chromosome of a producer cell introduced with the plasmid.

45. A virus vector produced by using the plasmid of any one of claims 1 to 36 or produced by using the method of claim 43 or 44.

46. A composition comprising a virus vector produced by using the plasmid of any one of claims 1 to 36 or produced by using the method of claim 43 or 44.

47. The composition of claim 46, wherein virus vector particles which do not comprise a nucleic acid in all virus vector particles are 65% or less.

48. The composition of claim 46 or 47, wherein virus vector particles comprising a nucleic acid comprising all genes of interest in all virus vector particles are 90% or greater.

49. The composition of any one of claims 46 to 48, wherein virus vector particles comprising a nucleic acid derived from the plasmid other than a desired nucleic acid in all virus vector particles are 2% or less.

50. Hay bacillus or *Escherichia coli* comprising the plasmid of any one of claims 1 to 36.

51. A producer cell introduced with the plasmid of any one of claims 1 to 36, wherein at least a part of a nucleic acid contained in the plasmid is incorporated into a chromosome of the producer cell.
